# EUROPEAN PATENT APPLICATION

(11) **EP 4 741 502 A1**
(43) Date of publication of application: **13.05.2026**
(21) Application number: 24835353.4
(22) Date of filing: 03.07.2024
(51) Int. Cl.: C12N 15/113, A61K 31/713, A61K 48/00, A61P 3/06

(54) **DSRNA MOLECULE FOR INHIBITING LP(A) GENE EXPRESSION AND USE THEREOF**

(30) Priority: 04.07.2023 CN 202310810311
(71) Applicant: CSPC Zhongqi Pharmaceutical Technology (Shijiazhuang) Co., Ltd., Shijiazhuang, Hebei 050035 (CN)
(72) Inventor: CHEN, Huiyu, Shijiazhuang, Hebei 050035 (CN); WANG, Lingyu, Shijiazhuang, Hebei 050035 (CN); SU, Xiaoye, Shijiazhuang, Hebei 050035 (CN); REN, Jiafeng, Shijiazhuang, Hebei 050035 (CN); SHAO, Yu, Shijiazhuang, Hebei 050035 (CN); ZHONG, Qiang, Shijiazhuang, Hebei 050035 (CN)
(74) Representative: Barrow, Nicholas Martin
(86) International application number: PCT/CN2024/103238
(87) International publication number: WO 2025/007872

(57) **Abstract**

Disclosed are a modified dsRNA molecule and a use thereof. Specifically, provided is a dsRNA molecule for inhibiting LP(a) gene expression, comprising a sense strand and an antisense strand complementary to form a double-stranded region, wherein the sense strand and/or the antisense strand comprises 15-25 nucleotides or consists of 15-25 nucleotides. The dsRNA molecule can be used for treating and/or preventing LP(a) gene-mediated diseases.

## Description

### Field

The present application belongs to the field of molecular biology and relates to a modified dsRNA molecule and a use thereof, and specifically to a dsRNA molecule for inhibiting the expression of LP(a) gene and a pharmaceutical composition thereof, as well as a method for reducing the expression level of LP(a) gene using the dsRNA molecule or the pharmaceutical composition thereof.

### Background

RNA interference (RNAi) refers to the phenomenon of highly conserved, highly efficient and specific degradation of homologous mRNA induced by double-stranded RNA (dsRNA) during evolution. RNAi is a monitoring mechanism commonly found in eukaryotes to resist viral invasion, inhibit transposon activity, and regulate gene expression. Small interfering RNA (siRNA) is a type of short double-stranded RNA molecule with a length of 19 to 30 and is one of the important tools in RNAi technology. In natural organisms, after dsRNA enters the cell, it will be specifically recognized by the Dicer enzyme and cut into small RNA fragments of 21 to 23 nucleotides in length (i.e., siRNA). The dsRNA fragments produced by the cutting are unwound into single strands and form complexes with certain proteins (referred to as RISC). RISC can bind to the mRNA complementary to dsRNAs in the cell and cut the mRNA to degrade it, resulting in the inability to synthesize proteins and produce a "silencing" phenomenon of genes. In industrial production, people prefer to chemically synthesize dsRNA and modify it to further improve the stability and effectiveness of dsRNA drugs. In recent years, breakthroughs have been made in the research of dsRNA drugs. Many dsRNA drugs for rare diseases have been approved by the FDA, such as patisiran (for the treatment of hereditary transthyretin amyloidosis), eteplirsen (Duchenne muscular dystrophy), givosiran (acute intermittent porphyria), spinraza (spinal muscular atrophy), etc. The treatment field of dsRNA drugs has gradually expanded from rare diseases to common diseases. For example, inclisiran, which has just been approved for the treatment of hyperlipidemia, DCRHBVS, which is used to treat hepatitis B, tivanisiran (dry eye syndrome), QPI-1007 (optic atrophy), SYL040012 (glaucoma), QPI-1002, which is used to treat severe kidney disease, etc. The continuous deepening of clinical research on dsRNA drugs and their successful marketing have made the development path of dsRNA drugs gradually clear. With their unique gene silencing ability, they have cured more and more diseases and brought good news to all mankind.

Lipoprotein A (Lp(a)) particles are essentially low-density lipoprotein-like particles, consisting of apolipoprotein A linked to LDL-like particles by the ApoB polypeptide. They are synthesized in the liver from independent triglycerides and are not affected by age or diet. High level of Lp(a) can lead to atherosclerosis and it has been found in the walls of arteries. Because its structure is similar to that of plasminogen, it can also inhibit fibrinolysis, thus forming blood clots; high serum levels of Lp(a) are associated with premature atherosclerosis and stroke. Serum concentrations of Lp(a) are mainly related to genetics and are basically unaffected by sex, age, weight, and most cholesterol-lowering drugs. Studies have shown that normal Lp(a) levels should be less than 300 mg/L (30 mg/dL). When Lp(a) concentrations exceed 34 mg/dL, the risk of coronary artery disease increases by about two times. When evaluated together with low-density lipoprotein cholesterol concentrations, the risk increases by about six times. Without considering other plasma lipoproteins, Lp(a) assessment values are considered to be the most sensitive feature of the development of coronary artery disease. Therefore, Lp(a) inhibitors are expected to become potential therapeutic targets for the treatment and prevention of cardiovascular and cerebrovascular diseases and their complications by reducing the concentration of Lp(a) in the blood.

Traditional statin lipid-regulating and plaque-stabilizing drugs do not cause significant changes in Lp(a) levels, nor do they lead to clinically important differences in Lp(a) in patients at risk for CVD. In some studies, statins can even lead to elevated Lp(a). Fibrates and ezetimibe are also ineffective in lowering Lp(a). Currently, drugs found to be effective in lowering Lp(a) comprise: niacin, PCSK9 inhibitors, estrogen, mipomersen, and lomitapide. Considering the effect of lowering Lp(a), economy, clinical adverse events, clinical operability and scalability, and cardiovascular benefits, the above treatment options are not the best choice. New Lp(a)-lowering drugs - small nucleic acid drugs are expected to become effective intervention measures to lower Lp(a). Therefore, the development of an efficient inhibitor for silencing Lp(a) will provide effective cardiovascular and cerebrovascular diseases for long-term treatment, making it have better efficacy, specificity, stability, targeting or tolerability.

### Summary of the invention

The present application provides a dsRNA molecule, agent, kit and pharmaceutical composition for inhibiting Lp(a) gene expression, as well as methods and uses of the above dsRNA molecule, agent, kit or pharmaceutical composition in inhibiting or reducing Lp(a) gene expression, preventing or treating diseases or symptoms related to an elevated level of Lp(a) protein. The dsRNA molecule promotes sequence-specific degradation of Lp(a) mRNA through RNAi action, thereby inhibiting Lp(a) gene expression or reducing the level of Lp(a) gene expression.

In one aspect, the present application provides a double-stranded ribonucleic acid (dsRNA) agent for inhibiting Lp(a) expression, wherein the dsRNA comprises a sense strand and an antisense strand, wherein the sense strand and/or antisense strand comprises at least 15 consecutive nucleotides that differ by no more than 3 nucleotides from the nucleotide sequence of any sense sequence and antisense sequence listed in Table 2 or Table 8.

In some embodiments, the sense strand and/or the antisense strand comprises or consists of 15-25 nucleotides, the antisense strand is complementary to 15, 16, 17, 18, 19, 20 or 21 consecutive nucleotides of a sense strand sequence in Table 2, and the double-stranded region is 15-25, preferably 19-21 in length, and at least one nucleotide in the dsRNA molecule is modified. In some embodiments, the modification is selected from any one or more of the following: locked nucleic acid (LNA) modification, open ring or unlocked (UNA) modification, 2'-methoxyethyl modification, 2'-O-methyl modification, 2'-O-allyl modification, 2'-C-allyl modification, 2'-fluoro modification, 2'-deoxy modification, 2'-hydroxyl modification, phosphorothioate backbone modification, DNA modification, fluorescent probe modification, and ligand modification.

The present application provides a dsRNA molecule, which has a sense strand having a nucleotide sequence set forth in any sense strand sequence in Table 2, and an antisense strand having a nucleotide sequence set forth in any antisense strand sequence in Table 2.

The dsRNA molecule can be selected from the dsRNAs listed in Table 2 of the Examples.

In some embodiments, the sense strand structure of the dsRNA molecule is shown in any of the following sequences:
AGAGUUAUCGAGGCACGUACU (SEQ ID NO:485)
GAGGCACGUACUCCACCACUG (SEQ ID NO:491)
AGUUAUCGAGGCACAUACUCC (SEQ ID NO:531)
CUGCCAAGCUUGGUCAUCUAU (SEQ ID NO:119)
CAGAGUUAUCGAGGCACAUUC (SEQ ID NO:709);

The antisense strand structure of the dsRNA molecule is shown in any of the following sequences:
AGUACGUGCCUCGAUAACUCUGU (SEQ ID NO:486)
CAGUGGUGGAGUACGUGCCUCGA (SEQ ID NO:492)
GGAGUAUGUGCCUCGAUAACUCU (SEQ ID NO:532)
AUAGAUGACCAAGCUUGGCAGGU (SEQ ID NO: 120)
GAAUGUGCCUCGAUAACUCUGGC (SEQ ID NO:710).

In some embodiments, the modification patterns of the dsRNA molecules of the present application include: (1) positive strand: 17-21nt, such as 21nt in length; composed of alternating 2'-O-methyl modified regions and 2'-fluoro modified regions, each modified region being 1 to 3 nucleotides in length; the modification patterns of the first modified region from the 5' end and that from the 3' end being the same; (2) antisense strand: 19-23nt, such as 23nt in length; composed of alternating 2'-O-methyl modified regions, 2'-fluoro modified regions, unmodified regions or DNA regions, each modified region being 1 to 5 nucleotides in length; and the consecutive nucleotide regions from the 2nd to the 5th position from the 5' end, and the consecutive nucleotide regions from the 1st to the 3rd position from the 3' end all being ligated by phosphorothioate backbones. In some alternative embodiments, the modification patterns of the dsRNA molecule of the present application may also include: (1) sense strand: 17-21nt, preferably 21nt in length; composed of alternating 2'-O-methyl modified regions and 2'-fluoro modified regions, each modified region being 1 to 10 nucleotides in length; the modification patterns of the first modified region from the 5' end and that from the 3' end being the same; and the consecutive nucleotide regions from the 1st to the 3rd position from the 5' end being ligated by phosphorothioate backbones; (2)antisense strand: 19-23nt, preferably 23nt in length; composed of alternating 2'-O-methyl modified regions, 2'-fluoro modified regions, unmodified regions or DNA regions, each modified region being 1-11 nucleotides in length; and the consecutive nucleotide regions from the 1st to the 3rd position from the 5' end and the consecutive nucleotide regions from the 1st to the 3rd position from the 3 ' end all being ligated by phosphorothioate backbones.

In one embodiment, the 2'-O-methyl modified region in the sense strand is 1-10 nucleotides in length, and the 2'-fluoro modified region is 1-3 nucleotides in length; the 2'-O-methyl modified region in the antisense strand is 1-11 nucleotides in length, and the 2'-fluoro modified region is 1-3 nucleotides in length.

In some embodiments, the structure of the sense strand of the dsRNA molecule is shown in any of the following sequences:
AmsGmsAmGmUmUmAfUmCfGfAfGmGmCmAmCmGmUmAmCmUm (SEQ ID NO: 1)
GmsAmsGmGmCmAmCfGmUfAfCfUmCmCmAmCmCmAmCmUmGm (SEQ ID NO:3)
AmsGmsUmUmAmUmCfGmAfGfGfCmAmCmAmUmAmCmUmCmCm (SEQ ID NO:5)
CmsUmsGmCmCmAmAfGmCfUfUfGmGmUmCmAmUmCmUmAmUm (SEQ ID NO:7)
CmsAmsGmAmGmUmUfAmUfCfGfAmGmGmCmAmCmAmUmUmCm (SEQ ID NO:9).
the structure of the antisense strand of the dsRNA molecule is shown in any of the following sequences:
   AmsGfsUmAmCmGmUmGmCmCmUmCmGmAfUmAfAmCmUmCmUmsGmsUm (SEQ ID NO:2)
   CmsAfsGmUmGmGmUmGmGmAmGmUmAmCfGmUfGmCmCmUmCmsGmsAm (SEQ ID NO:4)
   GmsGfsAmGmUmAmUmGmUmGmCmCmUmCfGmAfUmAmAmCmUmsCmsUm (SEQ ID NO:6)
   AmsUfsAmGmAmUmGmAmCmCmAmAmGmCfUmUfGmGmCmAmGmsGmsUm (SEQ ID NO:8)
   GmsAfsAmUmGmUmGmCmCmUmCmGmAmUfAmAfCmUmCmUmGmsGmsCm (SEQ ID NO:10),
   wherein Am, Um, Cm and Gm represent 2'-O-methyl modified ribonucleotides A, U, C and G respectively; Af, Uf, Cf and Gf represent 2'-fluoro modified ribonucleotides A, U, C and G respectively; s indicates that the preceding and the posterior nucleotides are ligated by a phosphorothioate backbone.

In some preferred embodiments, the dsRNA molecule is selected from dsRNA molecules represented by LP164, LP173, LP508, LP2941, and LP4927, wherein:
LP164:
   sense strand:
      AmsGmsAmGmUmUmAfUmCfGfAfGmGmCmAmCmGmUmAmCmUm (SEQ ID NO:1)
   antisense strand:
      AmsGfsUmAmCmGmUmGmCmCmUmCmGmAfUmAfAmCmUmCmUmsGmsUm (SEQ ID NO:2);
LP173:
   sense strand:
      GmsAmsGmGmCmAmCfGmUfAfCfUmCmCmAmCmCmAmCmUmGm (SEQ ID NO:3)
   antisense strand:
      CmsAfsGmUmGmGmUmGmGmAmGmUmAmCfGmUfGmCmCmUmCmsGmsAm (SEQ ID NO:4):
LP508:
   sense strand:
      AmsGmsUmUmAmUmCfGmAfGfGfCmAmCmAmUmAmCmUmCmCm (SEQ ID NO:5)
   antisense strand:
      GmsGfsAmGmUmAmUmGmUmGmCmCmUmCfGmAfUmAmAmCmUmsCmsUm (SEQ ID NO:6);
LP2941:
   sense strand:
      CmsUmsGmCmCmAmAfGmCfUfUfGmGmUmCmAmUmCmUmAmUm (SEQ ID NO:7)
   antisense strand:
      AmsUfsAmGmAmUmGmAmCmCmAmAmGmCfUmUfGmGmCmAmGmsGmsUm (SEQ ID NO:8); and
LP4927:
   sense strand:
      CmsAmsGmAmGmUmUfAmUfCfGfAmGmGmCmAmCmAmUmUmCm (SEQ ID NO:9)
   antisense strand:
      GmsAfsAmUmGmUmGmCmCmUmCmGmAmUfAmAfCmUmCmUmGmsGmsCm (SEQ ID NO:10),
wherein, Am, Um, Cm, and Gm represent 2'-O-methyl-modified ribonucleotides A, U, C, and G respectively; Af, Uf, Cf, and Gf represent 2'-fluoro-modified ribonucleotides A, U, C, and G, respectively; and s indicates that the preceding and the posterior nucleotides are ligated by a phosphorothioate backbone.

The dsRNA of the present application may further comprise ligand modification. The ligand may be a portion that is taken up by a host cell. Ligand modification can improve the cellular uptake, intracellular targeting, half-life, or drug metabolism or kinetics of the dsRNA molecule. In some embodiments, compared with a dsRNA without ligand modification, the dsRNA with ligand modification has enhanced affinity or cellular uptake for selected targets (such as specific tissue types, cell types, organelles, etc.), such as hepatocytes. Ligand modification does not interfere with the activity of the dsRNA.

In some embodiments, the ligand modification is to make one or more ligand modifications on the 3' end, 5' end and/or the middle of the sequence of the dsRNA molecule of the present application.

In some preferred embodiments, the ligand is selected from the group consisting of cholesterol, biotin, vitamins, galactose derivatives or analogs, lactose derivatives or analogs, N-acetylgalactosamine derivatives or analogs, and N-acetylglucosamine derivatives or analogs. The ligand targets a cell surface receptor, including galactose, galactosamine, lactose, or N-acetylgalactosamine/glucosamine moiety. The ligand is preferably targeted to the liver, particularly to parenchymal cells of the liver.

In some preferred embodiments, the ligand targets the ASGPR receptor.

In some preferred embodiments, the ligand may also be human serum albumin (HSA), hyaluronic acid, a polypeptide, etc.

In some preferred embodiments, the ligand-modified dsRNA is selected from any one of the following dsRNA molecules (5'->3' direction):
1) LP164-E05
   sense strand:
      AmsGmsAmGmUmUmAfUmCfGfAfGmGmCmAmCmGmUmAmCmUm (SEQ ID NO:1)-L96,
   antisense strand:
      AmsGfsUmAmCmGmUmGmCmCmUmCmGmAfUmAfAmCmUmCmUmsGmsUm (SEQ ID NO:2);
2) LP173-E05
   sense strand:
      GmsAmsGmGmCmAmCfGmUfAfCfUmCmCmAmCmCmAmCmUmGm (SEQ ID NO:3)-L96,
   antisense strand:
      CmsAfsGmUmGmGmUmGmGmAmGmUmAmCfGmUfGmCmCmUmCmsGmsAm (SEQ ID NO:4):
3) LP508-E05
   sense strand:
      AmsGmsUmUmAmUmCfGmAfGfGfCmAmCmAmUmAmCmUmCmCm (SEQ ID NO:5)-L96,
   antisense strand:
      GmsGfsAmGmUmAmUmGmUmGmCmCmUmCfGmAfUmAmAmCmUmsCmsUm (SEQ ID NO:6);
4) LP2941-E05
   sense strand:
      CmsUmsGmCmCmAmAfGmCfUfUfGmGmUmCmAmUmCmUmAmUm (SEQ ID NO:7)-L96,
   antisense strand:
      AmsUfsAmGmAmUmGmAmCmCmAmAmGmCfUmUfGmGmCmAmGmsGmsUm (SEQ ID NO:8); and
5) LP4927-E05
   sense strand:
      CmsAmsGmAmGmUmUfAmUfCfGfAmGmGmCmAmCmAmUmUmCm (SEQ ID NO:9)-L96,
   antisense strand:
      GmsAfsAmUmGmUmGmCmCmUmCmGmAmUfAmAfCmUmCmUmGmsGmsCm (SEQ ID NO: 10);
wherein, Am, Um, Cm, and Gm represent 2'-O-methyl-modified ribonucleotides A, U, C, and G respectively; Af, Uf, Cf, and Gf represent 2'-fluoro-modified ribonucleotides A, U, C, and G respectively; and s indicates that the preceding and the posterior nucleotides are ligated by a phosphorothioate backbone. The structure of L96 and the its linkage mode to the sense strand nucleotide (i.e., "-L96") are shown below:

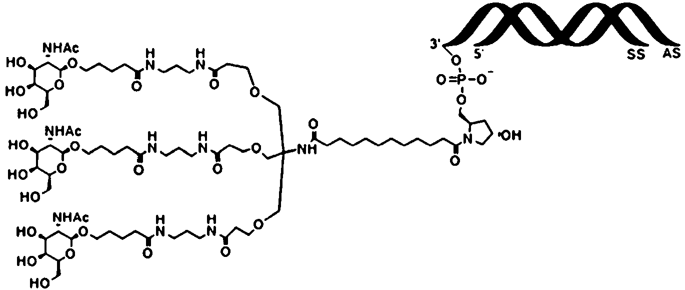

In some embodiments, the 3' end, 5' end and/or the middle of the sequence of the dsRNA of the present application may also be modified with 1-5, 2-4 or 3 N-acetylgalactosamine derivatives or analogs, wherein the N-acetylgalactosamine derivative is preferably L96.

In some preferred embodiments, each strand of the dsRNA molecule of the present invention may comprise 0%-100% modified nucleotides, such as 5%, 10%, 15%, 20%, 25%, 30%, 35%, 40%, 45%, 50%, 55%, 60%, 65%, 70%, 75%, 80%, 85%, 90%, 95%, 99%, or 100 % modified nucleotides. The modifications may be in the overhang region or the double-stranded region. The modifications may be used to improve the in vitro or in vivo properties of the dsRNA molecule, such as stability, biodistribution, inhibitory activity, etc. The above modifications may be used in combination.

In some preferred embodiments, the ends of each strand of the dsRNA molecule of the present application have an overhang or are blunt ends; including 1-8 overhangs, such as 1, 2, 3, 4, 5, 6, 7, or 8 overhangs at the 5' and/or 3' ends of any one or both strands, and the overhangs are arbitrarily selected from U, A, G, C, T, and dT.

In some preferred embodiments, the dsRNA molecule of the present application is capable of inhibiting human Lp(a) gene expression. In some embodiments, when the antisense strand of the dsRNA of the present application hybridizes with the pre-mRNA or mature mRNA of the Lp(a) gene at maximum complementarity, the number of base mismatches between the antisense strand and the pre-mRNA or mature mRNA does not exceed two.

In some embodiments, the dsRNA molecule of the present application comprises a sense strand and an antisense strand, wherein the sense strand is 15, 16, 17, 18, 19, 20, 21, 22, 23, 24 or 25 nucleotides, preferably 21 nucleotides in length, and the antisense strand is 15, 16, 17, 18, 19, 20, 21, 22, 23, 24 or 25 nucleotides, preferably 23 nucleotides in length.

In one embodiment, the dsRNA molecule of the present application comprises a sense strand and an antisense strand, wherein the base sequences thereof respectively comprise at least 15 consecutive nucleotides that differ by no more than 3 nucleotides from the sense strand set forth in CAGAGUUAUCGAGGCACAUUC (SEQ ID NO: 709) and the antisense strand set forth in GAAUGUGCCUCGAUAACUCUGGC (SEQ ID NO: 710).

In one embodiment, the dsRNA molecule of the present application comprises a sense strand and an antisense strand, wherein the base sequences thereof comprise at least 15 consecutive nucleotides that differ by no more than 3 nucleotides from the sense strand set forth in CAGAGUUAUCGAGGCACAUUC (SEQ ID NO:709) and the antisense strand set forth in GAAUGUGCCUCGAUAACUCUGGC (SEQ ID NO:710); and the sense strand is 15, 16, 17, 18, 19, 20, 21, 22, 23, 24, or 25 nucleotides in length, and the antisense strand is 15, 16, 17, 18, 19, 20, 21, 22, 23, 24, or 25 nucleotides in length.

In some embodiments, the sense strand and/or the antisense strand of the dsRNA molecule of the present application comprises or consists of 15-25 nucleotides, and the antisense strand is complementary to 15, 16, 17, 18, 19, 20 or 21 consecutive nucleotides of the sense strand sequence of GAAUGUGCCUCGAUAACUCUGGC (SEQ ID NO: 710), wherein the double-stranded region of the dsRNA molecule is 15-25, preferably 19-21bp, for example, 16, 17, 18, 20, 22, 23, or 24bp in length. In one embodiment, the sense strand of the dsRNA molecule of the present application is 18, 19, 20, 21 or 22nt in length, and the base sequence of the sense strand has at least 19 consecutive identical bases with CAGAGUUAUCGAGGCACAUUC (SEQ ID NO: 709); and the antisense strand is about 21, 22, 23, 24 or 25nt in length, and the base sequence of the antisense strand has 21 consecutive identical bases with GAAUGUGCCUCGAUAACUCUGGC (SEQ ID NO: 710).

In one embodiment, the base sequence of the sense strand of the dsRNA molecule of the present application comprises CAGAGUUAUCGAGGCACAUUC (SEQ ID NO: 709) and the base sequence of the antisense strand comprises GAAUGUGCCUCGAUAACUCUGGC (SEQ ID NO: 710).

In one embodiment, the base sequence of the sense strand of the dsRNA molecule of the present application consists of CAGAGUUAUCGAGGCACAUUC (SEQ ID NO: 709) and 0, 1, 2, 3 or 4 additional nucleotides, and the base sequence of the antisense strand consists of GAAUGUGCCUCGAUAACUCUGGC (SEQ ID NO: 710) and 0, 1, 2, 3 or 4 additional nucleotides.

In one embodiment, the base sequence of the sense strand of the dsRNA molecule of the present application is CAGAGUUAUCGAGGCACAUUC (SEQ ID NO: 709), and the base sequence of the antisense strand is GAAUGUGCCUCGAUAACUCUGGC (SEQ ID NO: 710).

In one embodiment, the dsRNA molecule of the present application comprises a sense strand and an antisense strand, wherein the base sequences thereof comprise at least 15 consecutive nucleotides that differ by no more than 3 nucleotides from the sense strand set forth in AGAGUUAUCGAGGCACGUACU (SEQ ID NO: 485) and the antisense strand set forth in AGUACGUGCCUCGAUAACUCUGU (SEQ ID NO: 486).

In one embodiment, the dsRNA molecule of the present application comprises a sense strand and an antisense strand, wherein the base sequences thereof comprises at least 15 consecutive nucleotides that differ by no more than 3 nucleotides from the sense strand set forth in CAGAGUUAUCGAGGCACAUUCAGAGUUAUCGAGGCACGUACU (SEQ ID NO:485) and the antisense strand set forth in AGUACGUGCCUCGAUAACUCUGU (SEQ ID NO:486); and the sense strand is 15, 16, 17, 18, 19, 20, 21, 22, 23, 24, or 25 nucleotides in length, and the antisense strand is 15, 16, 17, 18, 19, 20, 21, 22, 23, 24, or 25 nucleotides in length.

In some embodiments, the sense strand and/or the antisense strand of the dsRNA molecule of the present application comprises or consists of 15-25 nucleotides, and the antisense strand is complementary to 15, 16, 17, 18, 19, 20 or 21 consecutive nucleotides of the sense strand sequence of AGUACGUGCCUCGAUAACUCUGU (SEQ ID NO:486), wherein the double-stranded region of the dsRNA molecule is 15-25, preferably 19-21bp, for example, 16, 17, 18, 20, 22, 23, or 24bp in length. In one embodiment, the sense strand of the dsRNA molecule of the present application is 18, 19, 20, 21 or 22nt in length, and the base sequence of the sense strand has at least 19 consecutive identical bases with AGAGUUAUCGAGGCACGUACU (SEQ ID NO:485); and the antisense strand is about 21, 22, 23, 24 or 25nt in length, and the base sequence of the antisense strand has 21 consecutive identical bases with AGUACGUGCCUCGAUAACUCUGU (SEQ ID NO:486).

In one embodiment, the base sequence of the sense strand of the dsRNA molecule of the present application comprises AGAGUUAUCGAGGCACGUACU (SEQ ID NO:485) and base sequence of the antisense strand comprises AGUACGUGCCUCGAUAACUCUGU (SEQ ID NO:486).

In one embodiment, the base sequence of the sense strand of the dsRNA molecule of the present application consists of AGAGUUAUCGAGGCACGUACU (SEQ ID NO:485) and 0, 1, 2, 3 or 4 additional nucleotides, and the base sequence of the antisense strand consists of AGUACGUGCCUCGAUAACUCUGU (SEQ ID NO:486) and 0, 1, 2, 3 or 4 additional nucleotides.

In one embodiment, the base sequence of the sense strand of the dsRNA molecule of the present application is AGAGUUAUCGAGGCACGUACU (SEQ ID NO:485), and the base sequence of the antisense strand is AGUACGUGCCUCGAUAACUCUGU (SEQ ID NO:486).

In one embodiment, the dsRNA molecule of the present application comprises a sense strand and an antisense strand, wherein the base sequences thereof comprise at least 15 consecutive nucleotides that differ by no more than 3 nucleotides from the sense strand set forth in GAGGCACGUACUCCACCACUG (SEQ ID NO:491) and the antisense strand set forth in CAGUGGUGGAGUACGUGCCUCGA (SEQ ID NO:492).

In one embodiment, the dsRNA molecule of the present application comprises a sense strand and an antisense strand, wherein the base sequences thereof comprises at least 15 consecutive nucleotides that differ by no more than 3 nucleotides from the sense strand set forth in GAGGCACGUACUCCACCACUG (SEQ ID NO:491) and the antisense strand set forth in CAGUGGUGGAGUACGUGCCUCGA (SEQ ID NO:492); and the sense strand is 15, 16, 17, 18, 19, 20, 21, 22, 23, 24, or 25 nucleotides in length, and the antisense strand is 15, 16, 17, 18, 19, 20, 21, 22, 23, 24, or 25 nucleotides in length.

In some embodiments, the sense strand and/or the antisense strand of the dsRNA molecule of the present application comprises or consists of 15-25 nucleotides, and the antisense strand is complementary to 15, 16, 17, 18, 19, 20 or 21 consecutive nucleotides of the sense strand sequence of CAGUGGUGGAGUACGUGCCUCGA (SEQ ID NO:492), wherein the double-stranded region of the dsRNA molecule is 15-25, preferably 19-21bp, for example, 16, 17, 18, 20, 22, 23, or 24bp in length. In one embodiment, the sense strand of the dsRNA molecule of the present application is 18, 19, 20, 21 or 22nt in length, and the base sequence of the sense strand has at least 19 consecutive identical bases with GAGGCACGUACUCCACCACUG (SEQ ID NO:491); and the antisense strand is about 21, 22, 23, 24 or 25nt in length, and the base sequence of the antisense strand has 21 consecutive identical bases with CAGUGGUGGAGUACGUGCCUCGA (SEQ ID NO:492).

In one embodiment, the base sequence of the sense strand of the dsRNA molecule of the present application comprises GAGGCACGUACUCCACCACUG (SEQ ID NO:491) and base sequence of the antisense strand comprises CAGUGGUGGAGUACGUGCCUCGA (SEQ ID NO:492).

In one embodiment, the base sequence of the sense strand of the dsRNA molecule of the present application consists of GAGGCACGUACUCCACCACUG (SEQ ID NO:491) and 0, 1, 2, 3 or 4 additional nucleotides, and the base sequence of the antisense strand consists of CAGUGGUGGAGUACGUGCCUCGA (SEQ ID NO:492) and 0, 1, 2, 3 or 4 additional nucleotides.

In one embodiment, the base sequence of the sense strand of the dsRNA molecule of the present application is AGAGUUAUCGAGGCACGUACU (SEQ ID NO:491), and the base sequence of the antisense strand is CAGUGGUGGAGUACGUGCCUCGA (SEQ ID NO:492).

In one embodiment, the dsRNA molecule of the present application comprises a sense strand and an antisense strand, wherein the base sequences thereof comprise at least 15 consecutive nucleotides that differ by no more than 3 nucleotides from the sense strand set forth in AGUUAUCGAGGCACAUACUCC (SEQ ID NO:531) and the antisense strand set forth in GGAGUAUGUGCCUCGAUAACUCU (SEQ ID NO:532).

In one embodiment, the dsRNA molecule of the present application comprises a sense strand and an antisense strand, which respectively comprises at least 15 consecutive nucleotides that differ by no more than 3 nucleotides from the sense strand set forth in AGUUAUCGAGGCACAUACUCC (SEQ ID NO:531) and the antisense strand set forth in GGAGUAUGUGCCUCGAUAACUCU (SEQ ID NO:532); and the sense strand is 15, 16, 17, 18, 19, 20, 21, 22, 23, 24, or 25 nucleotides in length, and the antisense strand is 15, 16, 17, 18, 19, 20, 21, 22, 23, 24, or 25 nucleotides in length.

In some embodiments, the sense strand and/or the antisense strand of the dsRNA molecule of the present application comprises or consists of 15-25 nucleotides, and the antisense strand is complementary to 15, 16, 17, 18, 19, 20 or 21 consecutive nucleotides of the sense strand sequence of GGAGUAUGUGCCUCGAUAACUCU (SEQ ID NO:532), wherein the double-stranded region of the dsRNA molecule is 15-25, preferably 19-21bp, for example, 16, 17, 18, 20, 22, 23, or 24bp in length. In one embodiment, the sense strand of the dsRNA molecule of the present application is 18, 19, 20, 21 or 22nt in length, and the base sequence of the sense strand has at least 19 consecutive identical bases with AGUUAUCGAGGCACAUACUCC (SEQ ID NO:531); and the antisense strand is about 21, 22, 23, 24 or 25nt in length, and the base sequence of the antisense strand has 21 consecutive identical bases with GGAGUAUGUGCCUCGAUAACUCU (SEQ ID NO:532).

In one embodiment, the base sequence of the sense strand of the dsRNA molecule of the present application comprises AGUUAUCGAGGCACAUACUCC (SEQ ID NO:531) and base sequence of the antisense strand comprises GGAGUAUGUGCCUCGAUAACUCU (SEQ ID NO:532).

In one embodiment, the base sequence of the sense strand of the dsRNA molecule of the present application consists of AGUUAUCGAGGCACAUACUCC (SEQ ID NO:531) and 0, 1, 2, 3 or 4 additional nucleotides, and the base sequence of the antisense strand consists of GGAGUAUGUGCCUCGAUAACUCU (SEQ ID NO:532) and 0, 1, 2, 3 or 4 additional nucleotides.

In one embodiment, the base sequence of the sense strand of the dsRNA molecule of the present application is AGUUAUCGAGGCACAUACUCC (SEQ ID NO:531), and the base sequence of the antisense strand is GGAGUAUGUGCCUCGAUAACUCU (SEQ ID NO:532).

In one embodiment, the dsRNA molecule of the present application comprises a sense strand and an antisense strand, wherein the base sequences thereof respectively comprise at least 15 consecutive nucleotides that differ by no more than 3 nucleotides from the sense strand set forth in ACUGCCAAGCUUGGUCAUCUAU (SEQ ID NO:119) and the antisense strand set forth in AUAGAUGACCAAGCUUGGCAGGU (SEQ ID NO:120).

In one embodiment, the dsRNA molecule of the present application comprises a sense strand and an antisense strand, wherein the base sequences thereof respectively comprises at least 15 consecutive nucleotides that differ by no more than 3 nucleotides from the sense strand set forth in ACUGCCAAGCUUGGUCAUCUAU (SEQ ID NO:119) and the antisense strand set forth in AUAGAUGACCAAGCUUGGCAGGU (SEQ ID NO:120); and the sense strand is 15, 16, 17, 18, 19, 20, 21, 22, 23, 24, or 25 nucleotides in length, and the antisense strand is 15, 16, 17, 18, 19, 20, 21, 22, 23, 24, or 25 nucleotides in length.

In some embodiments, the sense strand and/or the antisense strand of the dsRNA molecule of the present application comprises or consists of 15-25 nucleotides, and the antisense strand is complementary to 15, 16, 17, 18, 19, 20 or 21 consecutive nucleotides of the sense strand sequence of AUAGAUGACCAAGCUUGGCAGGU (SEQ ID NO:120), wherein the double-stranded region of the dsRNA molecule is 15-25, preferably 19-21bp, for example, 16, 17, 18, 20, 22, 23, or 24bp in length. In one embodiment, the sense strand of the dsRNA molecule of the present application is 18, 19, 20, 21 or 22nt in length, and the base sequence of the sense strand has at least 19 consecutive identical bases with ACUGCCAAGCUUGGUCAUCUAU (SEQ ID NO:119); and the antisense strand is about 21, 22, 23, 24 or 25nt in length, and the base sequence of the antisense strand has 21 consecutive identical bases with AUAGAUGACCAAGCUUGGCAGGU (SEQ ID NO:120).

In one embodiment, the base sequence of the sense strand of the dsRNA molecule of the present application comprises ACUGCCAAGCUUGGUCAUCUAU (SEQ ID NO:119) and base sequence of the antisense strand comprises AUAGAUGACCAAGCUUGGCAGGU (SEQ ID NO:120).

In one embodiment, the base sequence of the sense strand of the dsRNA molecule of the present application consists of ACUGCCAAGCUUGGUCAUCUAU (SEQ ID NO:119) and 0, 1, 2, 3 or 4 additional nucleotides, and the base sequence of the antisense strand consists of AUAGAUGACCAAGCUUGGCAGGU (SEQ ID NO:120) and 0, 1, 2, 3 or 4 additional nucleotides.

In one embodiment, the base sequence of the sense strand of the dsRNA molecule of the present application is ACUGCCAAGCUUGGUCAUCUAU (SEQ ID NO:119), and the base sequence of the antisense strand is AUAGAUGACCAAGCUUGGCAGGU (SEQ ID NO:120).

In one embodiment, the sequence of SEQ ID NO: 709, SEQ ID NO: 710, SEQ ID NO: 485, SEQ ID NO: 486, SEQ ID NO: 491, SEQ ID NO: 492, SEQ ID NO: 531, SEQ ID NO: 532, SEQ ID NO: 119 or SEQ ID NO: 120 may comprise one or more nucleotide modifications selected from 2'-O-methyl modification, 2'-fluoro modification, phosphorothioate backbone modification and ligand modification. The specific ligands are as described above.

In one embodiment, in the above-mentioned dsRNAs, the characteristics of the sense strands of the dsRNAs comprising SEQ ID NO: 709, SEQ ID NO : 485, SEQ ID NO: 491, SEQ ID NO: 531, and SEQ ID NO: 119 include: 17-21nt, such as 21nt in length; composed of alternating 2'-O-methyl modified regions and 2'-fluoro modified regions, each modified region being 1 to 10 nucleotides in length; the modification patterns of the first modified region from the 5' end and that from the 3' end being the same; and the consecutive nucleotide regions from the 1st to 3rd position from the 5' end being ligated by phosphorothioate backbones. In the above-mentioned dsRNAs, the characteristics of the antisense strand of the dsRNAs comprising SEQ ID NO: 710, SEQ ID NO: 486, SEQ ID NO: 492, SEQ ID NO: 532, and SEQ ID NO: 120 include: 19-23nt, preferably 23nt in length; composed of alternating 2'-O-methyl modified regions and 2'-fluoro modified regions, each modified region being 1-11 nucleotides in length; and the consecutive nucleotide regions from the 1st to the 3rd position from the 5' end and the consecutive nucleotide regions from the 1st to the 3rd position from the 3 ' end all being ligated by phosphorothioate backbones.

In one embodiment, the 2'-O-methyl modified region in the sense strand is 1-10 nucleotides in length, and the 2'-fluoro modified region is 1-3 nucleotides in length; the 2'-O-methyl modified region in the antisense strand is 1-11 nucleotides in length, and the 2'-fluoro modified region is 1-3 nucleotides in length.

In one embodiment, each of the nucleotide sequences of the above-mentioned SEQ ID NO:709, SEQ ID NO:710, SEQ ID NO :485, SEQ ID NO: 486, SEQ ID NO:491, SEQ ID NO:492, SEQ ID NO:531, SEQ ID NO:532, SEQ ID NO:119 and SEQ ID NO:120 is modified, and the modification patterns are as described above.

In one embodiment, the above-mentioned modified SEQ ID NO: 709, SEQ ID NO: 710, SEQ ID NO: 485, SEQ ID NO: 486, SEQ ID NO: 491, SEQ ID NO: 492, SEQ ID NO: 531, SEQ ID NO: 532, SEQ ID NO: 119 and SEQ ID NO: 120 may further comprise a ligand modification at the 3' end, 5' end and/or in the middle of the sequence. The specific ligands are as described above.

In some embodiments, the sense strand of the dsRNA of the present application is 18nt, 19nt, 20nt, 21nt, or 22nt in length, and comprises or is:
CmsAmsGmAmGmUmUfAmUfCfGfAmGmGmCmAmCmAmUmUmCm (SEQ ID NO:9); the antisense strand is 21, 22, 23, 24 or 25nt in length, and comprises or is:
GmsAfsAmUmGmUmGmCmCmUmCmGmAmUfAmAfCmUmCmUmGmsGmsCm (SEQ ID NO:10).

In some embodiments, the sense strand of the dsRNA of the present application is 18, 19, 20, 21 or 22nt in length, and comprises or is:
AmsGmsAmGmUmUmAfUmCfGfAfGmGmCmAmCmGmUmAmCmUm (SEQ ID NO: 1);
the antisense strand is 21, 22, 23, 24 or 25nt in length, and comprises or is:
AmsGfsUmAmCmGmUmGmCmCmUmCmGmAfUmAfAmCmUmCmUmsGmsUm (SEQ ID NO:2).

In some embodiments, the sense strand of the dsRNA of the present application is 18, 19, 20, 21 or 22nt in length, and comprises or is:
GmsAmsGmGmCmAmCfGmUfAfCfUmCmCmAmCmCmAmCmUmGm (SEQ ID NO:3);
the antisense strand is 21, 22, 23, 24 or 25nt in length, and comprises or is:

CmsAfsGmUmGmGmUmGmGmAmGmUmAmCfGmUfGmCmCmUmCmsGmsAm (SEQ ID NO:4).

In some embodiments, the sense strand of the dsRNA of the present application is 18, 19, 20, 21 or 22nt in length, and comprises or is:
AmsGmsUmUmAmUmCfGmAfGfGfCmAmCmAmUmAmCmUmCmCm (SEQ ID NO:5);
the antisense strand is 21, 22, 23, 24 or 25nt in length, and comprises or is:
GmsGfsAmGmUmAmUmGmUmGmCmCmUmCfGmAfUmAmAmCmUmsCmsUm (SEQ ID NO:6); and
In some embodiments, the sense strand of the dsRNA of the present application is 18, 19, 20, 21 or 22nt in length, and comprises or is:
CmsUmsGmCmCmAmAfGmCfUfUfGmGmUmCmAmUmCmUmAmUm (SEQ ID NO:7);
the antisense strand is 21, 22, 23, 24 or 25nt in length, and comprises or is:
AmsUfsAmGmAmUmGmAmCmCmAmAmGmCfUmUfGmGmCmAmGmsGmsUm (SEQ ID NO:8).

In the present application, Am, Um, Cm and Gm represent 2'-O-methyl modified ribonucleotides A, U, C and G respectively; Af, Uf, Cf and Gf represent 2'-fluoro modified ribonucleotides A, U, C and G respectively; s indicates that the preceding and the posterior nucleotides are ligated by a phosphorothioate backbone.

In another aspect, the present application also relates to a biological material related to the dsRNA of the present application. The biological material related to the dsRNA of the present application can be selected from any one of the following:
(A) a DNA molecule capable of producing the dsRNA of the present application;
(B) a vector capable of expressing the dsRNA of the present application;
(C) an agent or a kit comprising the dsRNA or DNA molecule of the present application or the vector;
(D) A pharmaceutical composition comprising the dsRNA molecule of the present application and other pharmaceutically acceptable components.

In some embodiments, the pharmaceutical composition comprises a pharmacologically effective amount of the dsRNA molecule of the present application and other pharmaceutically acceptable components. The "effective amount" refers to the amount of the dsRNA molecule that can effectively produce an expected pharmacologically therapeutic effect.

In some embodiments, "other components" include pharmaceutically acceptable carriers, such as water, saline, glucose, buffers (such as PBS), excipients, diluents, disintegrants, binders, lubricants, sweeteners, flavorings, preservatives, or a combination thereof.

In another aspect, the present application relates to a dsRNA or related biological material, for use in preventing and/or treating a disease mediated by Lp(a) gene, or alleviating a symptom of a disease mediated by Lp(a) gene.

In some embodiments, the present application further provides any of the following uses:
Use of the dsRNA or biological material of the present application in inhibiting Lp(a) gene expression or preparing a product for inhibiting Lp(a) gene expression.

Wherein, the inhibition of Lp(a) gene expression is to inhibit or reduce the expression level of Lp(a) gene of human or primates in a cell in vivo or in vitro; the inhibition of Lp(a) gene expression is that the expression level of Lp(a) is inhibited or reduced by at least 95%, 90%, 85%, 80%, 75%, 70%, 60%, 50%, 40%, 30%, 20%, 10% or 5%.

In some embodiments, the cell is a mammalian cell expressing Lp(a) gene, such as a primate cell or human cell, more preferably, a target cell that expresses a high level of Lp(a) gene, more preferably, a target cell derived from the brain, lung, liver, kidney, or tumor.

In some embodiments, the cell is selected from HepG2, HEP3B, Huh7, MHCC97H, Hela, cynomolgus monkey primary cells, and human primary cells.

In some embodiments, the final cellular concentration of the dsRNA molecule of the present application is 0.001-1000 nM, such as 0.001-10 nM, 10-500 nM, 25-300 nM or 50-100 nM.

In some embodiments, the dsRNA or related biological material of the present application can be administered by any suitable means, such as parenteral administration, including intramuscular, intravenous, intraarterial, peritoneal, or subcutaneous injection. The administration modes include but are not limited to single administration or multiple administration.

In some preferred embodiments, the dosage range is 0.1 mg/kg to 100 mg/kg, 0.5 mg/kg to 50 mg/kg, 3 mg/kg to 36 mg/kg, 2.5 mg/kg to 20 mg/kg, 5 mg/kg to 15 mg/kg, such as, 3 mg/kg, 4 mg/kg, 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg, 20 mg/kg, 21 mg/kg, 22 mg/kg, 23 mg/kg, 24 mg/kg, 25 mg/kg, 26 mg/kg, 27 mg/kg, 28 mg /kg, 29 mg/kg, 30 mg/kg, 31 mg/kg, 32 mg/kg, 33 mg/kg, 34 mg/kg, 35 mg/kg, 36 mg/kg, 37 mg/kg, 38 mg/kg, 39 mg/kg, 40 mg/kg, 41 mg/kg, 42 mg/kg, 43 mg/kg, 44 mg/kg, 45 mg/kg, 46 mg/kg, 47 mg/kg, 48 mg/kg, 49 mg/kg, 50 mg/ kg, 5mg/kg, 26mg/kg, 27mg/kg, 28mg/kg, 29mg/kg, 30mg/kg, 31mg/kg, 32mg/kg, 33mg/kg, 34mg/kg, 35mg/kg, 36mg/kg.

In some embodiments, a single dose of the pharmaceutical composition can be long-lasting, for example, the reduction in Lp(a) expression lasts for at least 3, 5, 7, 10, 14, 18, 22, 25, 27, 29, 32, 35, 40, 45, 50, 55, 60 days or longer.

In some embodiments, the present application relates to use of the dsRNA of the present application or the biological material of the present application for reducing Lp(a) in serum or in preparing a product for reducing Lp(a) in serum.

Wherein, reducing Lp(a) concentration in serum is to lower the Lp(a) concentration in the serum of human or primates; for example, the concentration or content of Lp(a) in serum is reduced by at least 5%, 10%, 15%, 20%, 25%, 30%, 35%, 40%, 50%, 60%, 70%, 80%, 90%, 95%, or 98%.

In some embodiments, the present application relates to use of the dsRNA or biological material of the present application in preventing and/or treating a disease mediated by Lp(a) gene or in preparing a product for preventing and/or treating a disease mediated by Lp(a) gene.

In some embodiments, the present application relates to use of the dsRNA or biological material of the present application in alleviating a symptom of a disease mediated by Lp(a) gene or in preparing a product for alleviating a symptom of a disease mediated by Lp(a) gene.

In some embodiments, the disease or symptom mediated by Lp(a) gene may be caused by overexpression of Lp(a) gene or overproduction of Lp(a) protein and may be regulated by downregulating Lp(a) gene expression. The treatment refers to alleviation, reduction, or cure of the disease or symptom mediated by Lp(a) gene, such as a reduction in serum Lp(a) level. For example, the content or concentration of serum Lp(a) is reduced by 5%, 10%, 15%, 20%, 25%, 30%, 35%, 40%, 50%, 60%, 70%, 80%, 90%, or 95%.

In another aspect, the present application also provides a method and/or a combination therapy for treating a subject suffering from a condition that would benefit from inhibiting or reducing Lp(a) gene expression, such as an Lp(a)-associated disease, wherein the method or combination therapy comprises administering the dsRNA or biological material of the present application alone or in combination with another therapeutic agent.

In certain embodiments, the present application also includes use of the LPA-targeting dsRNA of the present application in the preparation of a medicament for treating or preventing a cardiovascular disease, including coronary artery disease, peripheral artery disease, myocardial infarction or stroke, in a patient in need thereof.

In certain embodiments, the present application provides use of the LPA-targeting dsRNA of the present application in the preparation of a medicament for reducing LP(a) level in a patient in need thereof.

The combination therapy of the present application comprises administering an RNAi agent of the present application and a further therapeutic agent to a patient suffering from an Lp(a)-associated disease. The combination therapy of the present application reduces Lp(a) level in the subject (e.g., by about 30%, 35%, 40%, 45%, 50%, 55%, 60%, 65%, 70%, 75%, 80%, 85%, 90%, 95%, or about 99%).

In some embodiments, the further therapeutic agent can be an anti-Lp(a) antibody or an antigen-binding fragment or derivative thereof.

In some embodiments, the cardiovascular disease includes, but is not limited to, myocardial infarction, heart failure, stroke (ischemic and hemorrhagic), atherosclerosis, coronary artery disease, peripheral vascular disease (e.g., peripheral arterial disease), cerebrovascular disease, vulnerable plaque, and aortic stenosis.

In some embodiments, the patient in need of reducing LPA expression is a patient at risk for myocardial infarction. A patient at risk for myocardial infarction can be a patient with a history of myocardial infarction (e.g., having suffered a previous myocardial infarction). A patient at risk for myocardial infarction can also be a patient with a family history of myocardial infarction or with one or more risk factors for myocardial infarction. These risk factors include, but are not limited to, hypertension, elevated non-HDL cholesterol level, elevated triglyceride level, diabetes, obesity, or a history of an autoimmune disease (e.g., rheumatoid arthritis, lupus).

In one embodiment, the patient at risk for myocardial infarction is a patient suffering from or diagnosed with coronary artery disease.

In some embodiments, the present application also includes an LPA-targeting dsRNA for use in a method of reducing LP(a) level in a patient in need thereof.

In some embodiments, the present application provides LPA-targeting dsRNA for use in a method of reducing the risk of myocardial infarction in a patient in need thereof.

As examples, the present application also provides the following alternative technical solutions:
1. A dsRNA molecule for inhibiting Lp(a) gene expression, comprising a sense strand and an antisense strand that are complementary to each other to form a double-stranded region, wherein the sense strand and the antisense strand each comprise or consist of 15-25 nucleotides, and the antisense strand is complementary to at least 15, 16, 17, 18, 19, 20 or 21 consecutive nucleotides of a sense strand sequence shown in Table 2, and the double-stranded region is 15-25bp in length. Optionally, at least one nucleotide in the dsRNA molecule is modified.
2. The dsRNA molecule according to item 1, characterized in that the nucleotide sequence of the sense strand and the nucleotide sequence of the antisense strand are shown in Table 2.
3. dsRNA molecule according to item 1 or 2, characterized in that the modification is selected from any one or more of the following: locked nucleic acid modification, open ring or unlocked nucleic acid modification, 2'-methoxyethyl modification, 2'-O-methyl modification, 2'-O-allyl modification, 2'-C-allyl modification, 2'-fluoro modification, 2'-deoxy modification, 2'-hydroxyl modification, phosphorothioate backbone modification, DNA modification, fluorescent probe modification, and ligand modification.
4. The dsRNA molecule according to item 3, characterized in that the modification patterns of the dsRNA molecule include: (1) sense strand: 17-21nt, preferably 21nt in length; composed of alternating 2'-O-methyl modified regions and 2'-fluoro modified regions, each modified region being 1 to 3 nucleotides in length; the modification patterns of the first modified region from the 5' end and that from the 3' end being the same; (2) antisense strand: 19-23nt, preferably 23nt in length; composed of alternating 2'-O-methyl modified regions, 2'-fluoro modified regions, unmodified regions or DNA regions, each modified region being 1 to 5 nucleotides in length; and the consecutive nucleotide regions from the 2nd to the 5th position from the 5' end, and the consecutive nucleotide regions from the 1st to the 3rd position from the 3' end all being ligated by phosphorothioate backbones.
5. The dsRNA molecule according to item 4, characterized in that the structure of the sense strand of the dsRNA molecule is shown in (A1), (A2), (A3), (A4) or (A5); the structure of the antisense strand of the dsRNA molecule is shown in (A6), (A7), (A8), ( A9 ) or ( A10 ):
   (A1) AGAGUUAUCGAGGCACGUACU (SEQ ID NO:485)
   (A2) GAGGCACGUACUCCACCACUG (SEQ ID NO:491)
   (A3) AGUUAUCGAGGCACAUACUCC (SEQ ID NO:531)
   (A4) CUGCCAAGCUUGGUCAUCUAU (SEQ ID NO:119)
   (A5) CAGAGUUAUCGAGGCACAUUC (SEQ ID NO:709)
   (A6) AGUACGUGCCUCGAUAACUCUGU (SEQ ID NO:486)
   (A7) CAGUGGUGGAGUACGUGCCUCGA (SEQ ID NO:492)
   (A8) GGAGUAUGUGCCUCGAUAACUCU (SEQ ID NO:532)
   (A9) AUAGAUGACCAAGCUUGGCAGGU (SEQ ID NO:120)
   (A10) GAAUGUGCCUCGAUAACUCUGGC (SEQ ID NO:710).
6. The dsRNA molecule according to item 5, characterized in that the structure of the sense strand of the dsRNA molecule is shown in (B1), (B2), (B3), (B4), or (B5) ; and the structure of the antisense strand of the dsRNA molecule is set forth in (B6), (B7), (B8), (B9), or (B10).
   (B1) AmsGmsAmGmUmUmAfUmCfGfAfGmGmCmAmCmGmUmAmCmUm (SEQ ID NO: 1)
   (B2) GmsAmsGmGmCmAmCfGmUfAfCfUmCmCmAmCmCmAmCmUmGm (SEQ ID NO:3)
   (B3) AmsGmsUmUmAmUmCfGmAfGfGfCmAmCmAmUmAmCmUmCmCm (SEQ ID NO:5)
   (B4) CmsUmsGmCmCmAmAfGmCfUfUfGmGmUmCmAmUmCmUmAmUm (SEQ ID NO:7)
   (B5) CmsAmsGmAmGmUmUfAmUfCfGfAmGmGmCmAmCmAmUmUmCm (SEQ ID NO:9)
   (B6) AmsGmsUmAmCmGmUmGmCmCmUmCmGmAfUmAfAmCmUmCmUmsGmsUm (SEQ ID NO:2)
   (B7) CmsAfsGmUmGmGmUmGmGmAmGmUmAmCfGmUfGmCmCmUmCmsGmsAm(SEQ ID NO:4)
   (B8) GmsGfsAmGmUmAmUmGmUmGmCmCmUmCfGmAfUmAmAmCmUmsCmsUm(SEQ ID NO:6)
   (B9) AmsUfsAmGmAmUmGmAmCmCmAmAmGmCfUmUfGmGmCmAmGmsGmsUm(SEQ ID NO:8)
   (B10) GmsAfsAmUmGmUmGmCmCmUmCmGmAmUfAmAfCmUmCmUmGmsGmsCm(SEQ ID NO: 10)
   wherein, Am, Um, Cm and Gm represent 2'-O-methyl modified ribonucleotides A, U, C and G respectively; Af, Uf, Cf and Gf represent 2'- fluoro-modified ribonucleotides A, U, C and G respectively; (s) indicates that the preceding and the posterior nucleotides are ligated by a phosphorothioate backbone.
7. The dsRNA molecule according to item 6, characterized in that the dsRNA molecule is further modified with a ligand, and
   the structure of the sense strand of the modified dsRNA is shown in (C1), (C2), (C3), (C4) or (C5); the structure of the antisense strand of the dsRNA molecule is shown in (C6), (C7), (C8), (C9) or (C10):
   (C1) AmsGmsAmGmUmUmAfUmCfGfAfGmGmCmAmCmGmUmAmCmUm (SEQ ID NO:1)-L96
   (C2) GmsAmsGmGmCmAmCfGmUfAfCfUmCmCmAmCmCmAmCmUmGm (SEQ ID NO:3)-L96
   (C3) AmsGmsUmUmAmUmCfGmAfGfGfCmAmCmAmUmAmCmUmCmCm (SEQ ID NO:5)-L96
   (C4) CmsUmsGmCmCmAmAfGmCfUfUfGmGmUmCmAmUmCmUmAmUm (SEQ ID NO:7)-L96
   (C5) CmsAmsGmAmGmUmUfAmUfCfGfAmGmGmCmAmCmAmUmUmCm (SEQ ID NO:9)-L96
   (C6) AmsGmsUmAmCmGmUmGmCmCmUmCmGmAfUmAfAmCmUmCmUmsGmsUm (SEQ ID NO:2)
   (C7) CmsAfsGmUmGmGmUmGmGmAmGmUmAmCfGmUfGmCmCmUmCmsGmsAm (SEQ ID NO:4)
   (C8) GmsGfsAmGmUmAmUmGmUmGmCmCmUmCfGmAfUmAmAmCmUmsCmsUm (SEQ ID NO:6)
   (C9) AmsUfsAmGmAmUmGmAmCmCmAmAmGmCfUmUfGmGmCmAmGmsGmsUm (SEQ ID NO:8)
   (C10) GmsAfsAmUmGmUmGmCmCmUmCmGmAmUfAmAfCmUmCmUmGmsGmsCm (SEQ ID NO:10),
   wherein Am, Um, Cm, and Gm represent 2'-O-methyl-modified ribonucleotides A, U, C, and G respectively; Af, Uf, Cf, and Gf represent 2'-fluoro-modified ribonucleotides A, U, C, and G respectively; (s) indicates that the preceding and the posterior nucleotides are ligated by a phosphorothioate backbone; L96 is linked to a 3' terminal nucleotide of the nucleotide sequence of the sense strand of the dsRNA through a phosphodiester bond or a phosphorothioate bond; and the structure of the L96 and its linkage mode to the nucleotide sequence of the sense strand are as follows: 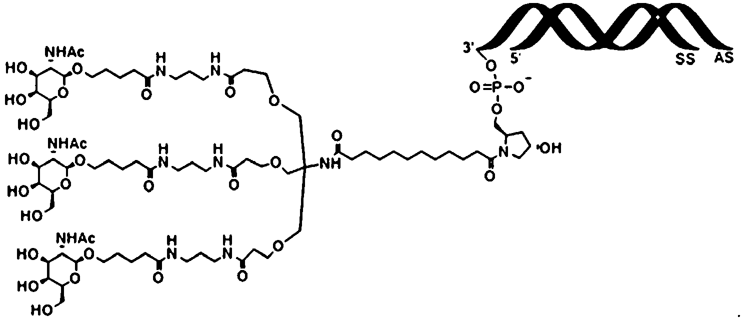
8. The dsRNA molecule according to item 7, wherein the direction is 5'->3', characterized in that the dsRNA molecule is selected from the following double-stranded RNA group:
   1) sense strand:
      AmsGmsAmGmUmUmAfUmCfGfAfGmGmCmAmCmGmUmAmCmUm (SEQ ID NO:1)-L96 antisense strand:
      AmsGmsUmAmCmGmUmGmCmCmUmCmGmAfUmAfAmCmUmCmUmsGmsUm (SEQ ID NO:2)
   2) sense strand:
      GmsAmsGmGmCmAmCfGmUfAfCfUmCmCmAmCmCmAmCmUmGm (SEQ ID NO:3)-L96 antisense strand:
      CmsAfsGmUmGmGmUmGmGmAmGmUmAmCfGmUfGmCmCmUmCmsGmsAm (SEQ ID NO:4)
   3) sense strand:
      AmsGmsUmUmAmUmCfGmAfGfGfCmAmCmAmUmAmCmUmCmCm (SEQ ID NO:5)-L96 antisense strand:
      GmsGfsAmGmUmAmUmGmUmGmCmCmUmCfGmAfUmAmAmCmUmsCmsUm (SEQ ID NO:6)
   4) sense strand:
      CmsUmsGmCmCmAmAfGmCfUfUfGmGmUmCmAmUmCmUmAmUm (SEQ ID NO:7)-L96 antisense strand:
      AmsUfsAmGmAmUmGmAmCmCmAmAmGmCfUmUfGmGmCmAmGmsGmsUm (SEQ ID NO:8)
   5) sense strand:
      CmsAmsGmAmGmUmUfAmUfCfGfAmGmGmCmAmCmAmUmUmCm (SEQ ID NO:9)-L96
      antisense strand:
         GmsAfsAmUmGmUmGmCmCmUmCmGmAmUfAmAfCmUmCmUmGmsGmsCm (SEQ ID NO:10)
      wherein Am, Um, Cm and Gm represent 2'-O-methyl modified ribonucleotides A, U, C and G respectively; Af, Uf, Cf, and Gf represent 2'- fluoro-modified ribonucleotides A, U, C, and G respectively; (s) indicates that the preceding and the posterior nucleotides are ligated by a phosphorothioate backbone; L96 is linked to a 3' terminal nucleotide of the nucleotide sequence of the sense strand of the dsRNA through a phosphodiester bond or a phosphorothioate bond.
9. A biomaterial selected from any one of the following:
   (A) a DNA molecule capable of producing the dsRNA according to any one of items 1 to 8;
   (B) a vector capable of expressing the dsRNA according to any one of items 1 to 8;
   (C) an agent or a kit comprising the dsRNA according to any one of items 1 to 8, or a DNA molecule of the dsRNA, or a vector comprising the dsRNA;
   (D) A pharmaceutical composition consisting of the dsRNA molecule according to any one of items 1 to 8 and other pharmaceutically acceptable components.
10. Use of a dsRNA, selected from any one of the following:
   (I) Use of the dsRNA according to any one of Items 1 to 8 or the biomaterial according to Item 9 for inhibiting Lp(a) gene expression or for preparing a product for inhibiting Lp (a) gene expression;
   (II) Use of the dsRNA according to any one of items 1 to 8 or the biomaterial according to item 9 in a product for reducing the level of Lp(a) particles;
   (III) Use of the dsRNA according to any one of items 1 to 8 or the biomaterial according to item 9 for preventing and/or treating a condition, pathology or syndrome associated with an elevated level of Lp(a) particles, or for the preparation of a product for preventing and/or treating a condition, pathology or syndrome associated with an elevated level of Lp(a) particles;
   wherein the disease associated with an elevated level of Lp(a) particles include: stroke, atherosclerosis, thrombosis or cardiovascular diseases such as coronary heart disease or aortic valve stenosis, and any other diseases or pathologies associated with an elevated level of Lp(a) particles, as well as reducing the risk of developing stroke, atherosclerosis, thrombosis or cardiovascular diseases such as coronary heart disease or aortic valve stenosis, and any other diseases or pathologies associated with an elevated level of Lp(a) particles.

The innovation of the present application includes but is not limited to the following points: 1. The modified dsRNA molecules have high stability and high inhibitory activity; 2. While maintaining high inhibitory activity and stability, the ligand-modified dsRNA molecules also have good liver targeting and the ability to promote cellular endocytosis, which can reduce the impact on other tissues or organs and reduce the amount of dsRNA molecules used, thereby achieving the purpose of reducing toxicity and reducing costs. In addition, the ligand-modified dsRNA molecules can enter target cells and target tissues without the need for transfection reagents. This reduces the negative effects of transfection reagents, such as cell or tissue toxicity, thereby provides possibilities for targeted therapy. Although many modifications can be attempted to improve the performance of dsRNAs, these attempts are generally difficult to both mediate RNA interference and have improved stability in serum (for example, increased resistance to nucleases and/or extended duration). However, the modified dsRNAs of the present application have high stability while maintaining high inhibitory activity, and achieve unexpected technical effects.

### Definition:

As used herein, "LPA " and "Lp(a)" are used interchangeably and, depending on the context, may be used to refer to lipoprotein A or a nucleic acid molecule encoding lipoprotein A. For example, a dsRNA targeting LPA is a dsRNA targeting an LPA encoding gene, such as a dsRNA targeting an mRNA or pre-mRNA transcribed from an LPA encoding gene.

"G," "C," "A," "T," and "U" generally represent nucleotides which have guanine, cytosine, adenine, thymine, and uracil as bases, respectively.

GalNAc: refers to 2-(acetylamino)-2-deoxy-D-galactopyranose, commonly referred to as N-acetylgalactosamine in the literature. Reference to "GalNAc" or "N-acetylgalactosamine" includes both the β form: 2-(acetylamino)-2-deoxy-β-D-galactopyranose and the α form: 2-(acetylamino)-2-deoxy-α-D-galactopyranose. The β form: 2-(acetylamino)-2-deoxy-β-D-galactopyranose and the α form: 2-(acetylamino)-2-deoxy-α-D-galactopyranose are used interchangeably. Preferably, the compound of the present application comprises the β form, i.e., 2-(acetylamino)-2-deoxy-β-D-galactopyranose.

L96: A GalNac conjugate with the following structure:

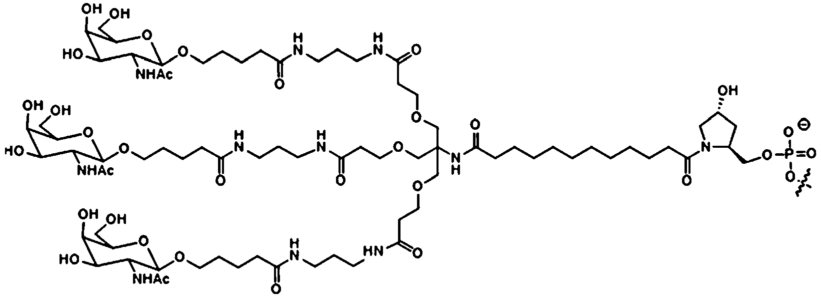

Typically, the majority of the nucleotides in each strand of a dsRNA molecule are ribonucleotides, but as described in detail herein, each strand or both strands may also comprise one or more non-ribonucleotides, such as deoxyribonucleotides and/or modified nucleotides. Additionally, as used herein, an "RNAi agent" may include ribonucleotides with chemical modifications, and an RNAi agent may include a variety of modifications on multiple nucleotides. Such modifications may include all types of modifications disclosed herein or known in the art. Any such modifications, such as those used in dsRNA, siRNA-type molecules, are encompassed within the "RNAi agent" for the purposes of this specification and items.

As used herein, "dsRNA" refers to a double-stranded RNA. Since siRNA is a double-stranded RNA, the term "dsRNA " encompasses an siRNA. dsRNA also includes a double-stranded RNA that is longer than siRNA. "longer than siRNA " can mean that the sense strand is longer than the siRNA, the antisense strand is longer than the siRNA, or both the sense and antisense strands are longer than the siRNA. Typically, after entering a cell, a double-stranded RNA that is longer than siRNA sequences comprised therein is broken down into siRNAs by a type III endonuclease called Dicer. In some embodiments, the two strands of the dsRNA are each independently 15 to 30 nucleotides in length (in this application, "nt" means nucleotides). Once the "siRNA" is incorporated into a RNA-induced silencing complex (RISC), one or more helicases within the RISC unwind the double helix of the siRNA. Upon binding to a target mRNA complementary to the antisense strand of the siRNA, one or more endonucleases within the RISC cleave the target, inducing gene silencing. Typically, the majority of nucleotides in each strand of the dsRNA molecule are ribonucleotides, but this does not exclude the inclusion of one or more non-ribonucleotides, such as deoxyribonucleotides and/or non-natural nucleotides, in either or both strands. In some embodiments, the dsRNA molecule does not comprise non-natural nucleotides. In some embodiments, each nucleotide in the dsRNA is a ribonucleotide. As used herein, a dsRNA may comprise one or more chemically modified nucleotides or may not comprise chemically modified nucleotides.

### BRIEF DESCRIPTION OF THE DRAWINGS

Figure 1: LPA gene expression in RT4 cells after administration of candidate modified sequences.
Figure 2: LPA gene expression in RT4 cells after administration of candidate modified sequences.
Figure 3: IC₅₀ values of chemically modified candidate molecules in Hep3B cells.
Figure 4: Percentage of serum Lp(a) levels in rhesus monkeys relative to baseline level before administration.
Figure 5: Map of Psicheck-2 plasmid.

### DETAILED DESCRIPTION

### Example 1. LPA-siRNA activity screening

### 1. siRNA Design

Based on the human LPA mRNA sequence (NM_005577.4), various LPA siRNAs were designed by selecting different sites. All designed single siRNAs can target all transcripts of the target gene (as shown in Table 1). The above sequences (as shown in Table 2) have the lowest homology with all other non-target gene sequences after alignment by sequence similarity software. The positive control sequence was designed as: OLP2706, an siRNA drug developed by Amgen, and SLN2545, an siRNA drug developed by Silence Therapeutics. The sense and antisense strands of OLP2706 are listed in WO 2021/119034A1 as SEQ ID Numbers 281 and 470 respectively. The sense and antisense strands of SLN2545 are listed in WO 2019/092283A1 as SEQ ID Numbers 9 and 10, respectively.

**Table 1 Target genes**

| target gene | species | Gene ID | NM_ID |
|---|---|---|---|
| LPA | Homo sapiens (human) | 4018 | NM_005577.4 |

**Table 2 High-throughput screening of unmodified sequences**

| name | sequence | | length | Molecular weight(g/mol) | GC content |
|---|---|---|---|---|---|
| | Sense strand (5'->3') | SEQ ID Number | | | |
| | Antisense strand (5'->3') | | | | |
| LP4927 | CAGAGUUAUCGAGGCACAUUC | SEQ ID NO:709 | 21 | 6696.1 | 47.6 |
| | GAAUGUGCCUCGAUAACUCUGGC | SEQ ID NO:710 | 23 | 7323.45 | 52.2 |
| LP1787 | CCGGUUCCAAGCCUAGAGGCU | SEQ ID NO:11 | 21 | 6687.08 | 61.9 |
| | AGCCUCUAGGCUUGGAACCGGGG | SEQ ID NO:12 | 23 | 7417.53 | 65.2 |
| LP1790 | GUUCCAAGCCUAGAGGCUCCU | SEQ ID NO:13 | 21 | 6648.04 | 57.1 |
| | AGGAGCCUCUAGGCUUGGAACCG | SEQ ID NO:14 | 23 | 7401.53 | 60.9 |
| LP1795 | AAGCCUAGAGGCUCCUUCCGA | SEQ ID NO:15 | 21 | 6671.08 | 57.1 |
| | UCGGAAGGAGCCUCUAGGCUUGG | SEQ ID NO:16 | 23 | 7418.52 | 60.9 |
| LP1798 | CCUAGAGGCUCCUUCCGAACA | SEQ ID NO:17 | 21 | 6631.05 | 57.1 |
| | UGUUCGGAAGGAGCCUCUAGGCU | SEQ ID NO:18 | 23 | 7379.48 | 56.5 |
| LP1799 | CUAGAGGCUCCUUCCGAACAA | SEQ ID NO:19 | 21 | 6655.08 | 52.4 |
| | UUGUUCGGAAGGAGCCUCUAGGC | SEQ ID NO:20 | 23 | 7379.48 | 56.5 |
| LP1802 | GAGGCUCCUUCCGAACAAGCA | SEQ ID NO:21 | 21 | 6694.12 | 57.1 |
| | UGCUUGUUCGGAAGGAGCCUCUA | SEQ ID NO:22 | 23 | 7340.44 | 52.2 |
| LP1806 | CUCCUUCCGAACAAGCACCGA | SEQ ID NO:23 | 21 | 6614.06 | 57.1 |
| | UCGGUGCUUGUUCGGAAGGAGCC | SEQ ID NO:24 | 23 | 7395.48 | 60.9 |
| LP1810 | UUCCGAACAAGCACCGACUGA | SEQ ID NO:25 | 21 | 6678.12 | 52.4 |
| | UCAGUCGGUGCUUGUUCGGAAGG | SEQ ID NO:26 | 23 | 7396.47 | 56.5 |
| LP1813 | CGAACAAGCACCGACUGAGCA | SEQ ID NO:27 | 21 | 6740.2 | 57.1 |
| | UGCUCAGUCGGUGCUUGUUCGGA | SEQ ID NO:28 | 23 | 7333.4 | 56.5 |
| LP1816 | ACAAGCACCGACUGAGCAAAG | SEQ ID NO:29 | 21 | 6764.23 | 52.4 |
| | CUUUGCUCAGUCGGUGCUUGUUC | SEQ ID NO:30 | 23 | 7231.29 | 52.2 |
| LP1817 | CAAGCACCGACUGAGCAAAGG | SEQ ID NO:31 | 21 | 6780.23 | 57.1 |
| | CCUUUGCUCAGUCGGUGCUUGUU | SEQ ID NO:32 | 23 | 7231.29 | 52.2 |
| LP1820 | GCACCGACUGAGCAAAGGCCU | SEQ ID NO:33 | 21 | 6733.16 | 61.9 |
| | AGGCCUUUGCUCAGUCGGUGCUU | SEQ ID NO:34 | 23 | 7293.37 | 56.5 |
| LP1825 | GACUGAGCAAAGGCCUGGGGU | SEQ ID NO:35 | 21 | 6830.21 | 61.9 |
| | ACCCCAGGCCUUUGCUCAGUCGG | SEQ ID NO:36 | 23 | 7274.4 | 65.2 |
| LP1833 | AAAGGCCUGGGGUGCAGGAGU | SEQ ID NO:37 | 21 | 6870.24 | 61.9 |
| | ACUCCUGCACCCCAGGCCUUUGC | SEQ ID NO:38 | 23 | 7194.34 | 65.2 |
| LP1836 | GGCCUGGGGUGCAGGAGUGCU | SEQ ID NO:39 | 21 | 6839.17 | 71.4 |
| | AGCACUCCUGCACCCCAGGCCUU | SEQ ID NO:40 | 23 | 7217.38 | 65.2 |
| LP1839 | CUGGGGUGCAGGAGUGCUACC | SEQ ID NO:41 | 21 | 6783.14 | 66.7 |
| | GGUAGCACUCCUGCACCCCAGGC | SEQ ID NO:42 | 23 | 7296.45 | 69.6 |
| LP 1840 | UGGGGUGCAGGAGUGCUACCA | SEQ ID NO:43 | 21 | 6807.17 | 61.9 |
| | UGGUAGCACUCCUGCACCCCAGG | SEQ ID NO:44 | 23 | 7297.44 | 65.2 |
| LP2249 | GGAAGAACCUGCCAAGCUUGG | SEQ ID NO:45 | 21 | 6774.18 | 57.1 |
| | CCAAGCUUGGCAGGUUCUUCCUG | SEQ ID NO:46 | 23 | 7276.38 | 56.5 |
| LP2250 | GAAGAACCUGCCAAGCUUGGU | SEQ ID NO:47 | 21 | 6735.14 | 52.4 |
| | ACCAAGCUUGGCAGGUUCUUCCU | SEQ ID NO:48 | 23 | 7260.38 | 52.2 |
| LP2253 | GAACCUGCCAAGCUUGGUCAU | SEQ ID NO:49 | 21 | 6672.07 | 52.4 |
| | AUGACCAAGCUUGGCAGGUUCUU | SEQ ID NO:50 | 23 | 7324.44 | 47.8 |
| LP2257 | CUGCCAAGCUUGGUCAUCUAA | SEQ ID NO:51 | 21 | 6609.99 | 47.6 |
| | AUAGAUGACCAAGCUUGGCAGGA | SEQ ID NO:52 | 23 | 7410.55 | 47.8 |
| LP2261 | CAAGCUUGGUCAUCUAUGACA | SEQ ID NO:53 | 21 | 6657.06 | 42.9 |
| | UGUCAUAGAUGACCAAGCUUGGC | SEQ ID NO:54 | 23 | 7347.48 | 47.8 |
| LP2264 | GCUUGGUCAUCUAUGACACCA | SEQ ID NO:55 | 21 | 6633.03 | 47.6 |
| | UGGUGUCAUAGAUGACCAAGCUU | SEQ ID NO:56 | 23 | 7348.47 | 43.5 |
| LP2482 | CCUAGAGGCUCCUUCCGAACA | SEQ ID NO:57 | 21 | 6631.05 | 57.1 |
| | UGUUCGGAAGGAGCCUCUAGGCU | SEQ ID NO:58 | 23 | 7379.48 | 56.5 |
| LP2523 | CUGGGGUGCAGGAGUGCUACC | SEQ ID NO:59 | 21 | 6783.14 | 66.7 |
| | GGUAGCACUCCUGCACCCCAGGC | SEQ ID NO:60 | 23 | 7296.45 | 69.6 |
| LP2526 | GGGUGCAGGAGUGCUACCACG | SEQ ID NO:61 | 21 | 6806.18 | 66.7 |
| | CGUGGUAGCACUCCUGCACCCCA | SEQ ID NO:62 | 23 | 7257.41 | 65.2 |
| LP2528 | GUGCAGGAGUGCUACCACGGU | SEQ ID NO:63 | 21 | 6767.14 | 61.9 |
| | ACCGUGGUAGCACUCCUGCACCC | SEQ ID NO:64 | 23 | 7257.41 | 65.2 |
| LP2531 | CAGGAGUGCUACCACGGUAAU | SEQ ID NO:65 | 21 | 6735.14 | 52.4 |
| | AUUACCGUGGUAGCACUCCUGCA | SEQ ID NO:66 | 23 | 7283.42 | 52.2 |
| LP2540 | UACCACGGUAAUGGACAGAGU | SEQ ID NO:67 | 21 | 6759.17 | 47.6 |
| | ACUCUGUCCAUUACCGUGGUAGC | SEQ ID NO:68 | 23 | 7260.38 | 52.2 |
| LP2542 | CCACGGUAAUGGACAGAGUUA | SEQ ID NO:69 | 21 | 6759.17 | 47.6 |
| | UAACUCUGUCCAUUACCGUGGUA | SEQ ID NO:70 | 23 | 7245.37 | 43.5 |
| LP2545 | CGGUAAUGGACAGAGUUAUCG | SEQ ID NO:71 | 21 | 6776.16 | 47.6 |
| | CGAUAACUCUGUCCAUUACCGUG | SEQ ID NO:72 | 23 | 7244.38 | 47.8 |
| LP2546 | GGUAAUGGACAGAGUUAUCGA | SEQ ID NO:73 | 21 | 6800.19 | 42.9 |
| | UCGAUAACUCUGUCCAUUACCGU | SEQ ID NO:74 | 23 | 7205.34 | 43.5 |
| LP2552 | GGACAGAGUUAUCGAGGCACA | SEQ ID NO:75 | 21 | 6798.21 | 52.4 |
| | UGUGCCUCGAUAACUCUGUCCAU | SEQ ID NO:76 | 23 | 7221.34 | 47.8 |
| LP2553 | GACAGAGUUAUCGAGGCACAU | SEQ ID NO:77 | 21 | 6759.17 | 47.6 |
| | AUGUGCCUCGAUAACUCUGUCCA | SEQ ID NO:78 | 23 | 7244.38 | 47.8 |
| LP2556 | AGAGUUAUCGAGGCACAUACU | SEQ ID NO:79 | 21 | 6720.13 | 42.9 |
| | AGUAUGUGCCUCGAUAACUCUGU | SEQ ID NO:80 | 23 | 7285.4 | 43.5 |
| LP2681 | UACUGCAGGAAUCCAGAUCCU | SEQ ID NO:81 | 21 | 6656.07 | 47.6 |
| | AGGAUCUGGAUUCCUGCAGUAGU | SEQ ID NO:82 | 23 | 7364.47 | 47.8 |
| LP2683 | CUGCAGGAAUCCAGAUCCUGU | SEQ ID NO:83 | 21 | 6672.07 | 52.4 |
| | ACAGGAUCUGGAUUCCUGCAGUA | SEQ ID NO:84 | 23 | 7347.48 | 47.8 |
| LP2687 | AGGAAUCCAGAUCCUGUGGCA | SEQ ID NO:85 | 21 | 6735.14 | 52.4 |
| | UGCCACAGGAUCUGGAUUCCUGC | SEQ ID NO:86 | 23 | 7299.42 | 56.5 |
| LP2689 | GAAUCCAGAUCCUGUGGCAGC | SEQ ID NO:87 | 21 | 6711.11 | 57.1 |
| | GCUGCCACAGGAUCUGGAUUCCU | SEQ ID NO:88 | 23 | 7299.42 | 56.5 |
| LP2693 | CCAGAUCCUGUGGCAGCCCCU | SEQ ID NO:89 | 21 | 6623.02 | 66.7 |
| | AGGGGCUGCCACAGGAUCUGGAU | SEQ ID NO:90 | 23 | 7441.56 | 60.9 |
| LP2696 | GAUCCUGUGGCAGCCCCUUAU | SEQ ID NO:91 | 21 | 6625 | 57.1 |
| | AUAAGGGGCUGCCACAGGAUCUG | SEQ ID NO:92 | 23 | 7425.56 | 56.5 |
| LP2702 | GUGGCAGCCCCUUAUUGUUAU | SEQ ID NO:93 | 21 | 6626.98 | 47.6 |
| | AUAACAAUAAGGGGCUGCCACAG | SEQ ID NO:94 | 23 | 7416.6 | 47.8 |
| LP2704 | GGCAGCCCCUUAUUGUUAUAC | SEQ ID NO:95 | 21 | 6609.99 | 47.6 |
| | GUAUAACAAUAAGGGGCUGCCAC | SEQ ID NO:96 | 23 | 7393.56 | 47.8 |
| LP2737 | CAGGUGGGAGUACUGCAACCU | SEQ ID NO:97 | 21 | 6751.14 | 57.1 |
| | AGGUUGCAGUACUCCCACCUGAC | SEQ ID NO:98 | 23 | 7282.43 | 56.5 |
| LP2739 | GGUGGGAGUACUGCAACCUGA | SEQ ID NO:99 | 21 | 6791.17 | 57.1 |
| | UCAGGUUGCAGUACUCCCACCUG | SEQ ID NO:100 | 23 | 7259.39 | 56.5 |
| LP2745 | AGUACUGCAACCUGACACAAU | SEQ ID NO:101 | 21 | 6663.11 | 42.9 |
| | AUUGUGUCAGGUUGCAGUACUCC | SEQ ID NO:102 | 23 | 7301.4 | 47.8 |
| LP2748 | ACUGCAACCUGACACAAUGCU | SEQ ID NO:103 | 21 | 6639.08 | 47.6 |
| | AGCAUUGUGUCAGGUUGCAGUAC | SEQ ID NO:104 | 23 | 7364.47 | 47.8 |
| LP2752 | CAACCUGACACAAUGCUCAGA | SEQ ID NO:105 | 21 | 6662.12 | 47.6 |
| | UCUGAGCAUUGUGUCAGGUUGCA | SEQ ID NO:106 | 23 | 7341.43 | 47.8 |
| LP2756 | CUGACACAAUGCUCAGACGCA | SEQ ID NO:107 | 21 | 6678.12 | 52.4 |
| | UGCGUCUGAGCAUUGUGUCAGGU | SEQ ID NO:108 | 23 | 7357.43 | 52.2 |
| LP2759 | ACACAAUGCUCAGACGCAGAA | SEQ ID NO:109 | 21 | 6725.19 | 47.6 |
| | UUCUGCGUCUGAGCAUUGUGUCA | SEQ ID NO:110 | 23 | 7278.36 | 47.8 |
| LP2765 | UGCUCAGACGCAGAAGGGACU | SEQ ID NO:111 | 21 | 6774.18 | 57.1 |
| | AGUCCCUUCUGCGUCUGAGCAUU | SEQ ID NO:112 | 23 | 7237.34 | 52.2 |
| LP2767 | CUCAGACGCAGAAGGGACUGC | SEQ ID NO:113 | 21 | 6773.19 | 61.9 |
| | GCAGUCCCUUCUGCGUCUGAGCA | SEQ ID NO:114 | 23 | 7275.39 | *60.9* |
| LP2770 | AGACGCAGAAGGGACUGCCGU | SEQ ID NO:115 | 21 | 6813.22 | 61.9 |
| | ACGGCAGUCCCUUCUGCGUCUGA | SEQ ID NO:116 | 23 | 7275.39 | 60.9 |
| LP2933 | GGAAGAACCUGCCAAGCUUGG | SEQ ID NO:117 | 21 | 6774.18 | 57.1 |
| | CCAAGCUUGGCAGGUUCUUCCUG | SEQ ID NO:118 | 23 | 7276.38 | 56.5 |
| LP2941 | CUGCCAAGCUUGGUCAUCUAU | SEQ ID NO:119 | 21 | 6609.99 | 47.6 |
| | AUAGAUGACCAAGCUUGGCAGGU | SEQ ID NO:120 | 23 | 7410.55 | 47.8 |
| LP2945 | CAAGCUUGGUCAUCUAUGACA | SEQ ID NO:121 | 21 | 6657.06 | 42.9 |
| | UGUCAUAGAUGACCAAGCUUGGC | SEQ ID NO:122 | 23 | 7347.48 | 47.8 |
| LP2952 | GGUCAUCUAUGACACCACACU | SEQ ID NO:123 | 21 | 6616.04 | 47.6 |
| | AGUGUGGUGUCAUAGAUGACCAA | SEQ ID NO:124 | 23 | 7411.54 | 43.5 |
| LP3020 | AACUACUGCCGAAAUCCAGAU | SEQ ID NO:125 | 21 | 6663.11 | 42.9 |
| | AUCUGGAUUUCGGCAGUAGUUCU | SEQ ID NO:126 | 23 | 7302.39 | 43.5 |
| *LP3022* | CUACUGCCGAAAUCCAGAUCC | SEQ ID NO:127 | 21 | 6615.05 | 52.4 |
| | GGAUCUGGAUUUCGGCAGUAGUU | SEQ ID NO:128 | 23 | 7381.46 | 47.8 |
| LP3023 | UACUGCCGAAAUCCAGAUCCU | SEQ ID NO:129 | 21 | 6616.04 | 47.6 |
| | AGGAUCUGGAUUUCGGCAGUAGU | SEQ ID NO:130 | 23 | 7404.5 | 47.8 |
| LP3025 | CUGCCGAAAUCCAGAUCCUGU | SEQ ID NO:131 | 21 | 6632.04 | 52.4 |
| | ACAGGAUCUGGAUUUCGGCAGUA | SEQ ID NO:132 | 23 | 7387.51 | 47.8 |
| LP3029 | CGAAAUCCAGAUCCUGUGGCA | SEQ ID NO:133 | 21 | 6695.11 | 52.4 |
| | UGCCACAGGAUCUGGAUUUCGGC | SEQ ID NO:134 | 23 | 7339.45 | 56.5 |
| LP3039 | AUCCUGUGGCAGCCCCUUGGU | SEQ ID NO:135 | 21 | 6641 | 61.9 |
| | ACCAAGGGGCUGCCACAGGAUCU | SEQ ID NO:136 | 23 | 7384.54 | 60.9 |
| LP3042 | CUGUGGCAGCCCCUUGGUGUU | SEQ ID NO:137 | 21 | 6657.99 | 61.9 |
| | AACACCAAGGGGCUGCCACAGGA | SEQ ID NO:138 | 23 | 7430.62 | 60.9 |
| LP3045 | UGGCAGCCCCUUGGUGUUAUA | SEQ ID NO:139 | 21 | 6666.02 | 52.4 |
| | UAUAACACCAAGGGGCUGCCACA | SEQ ID NO:140 | 23 | 7352.54 | 52.2 |
| LP3046 | GGCAGCCCCUUGGUGUUAUAC | SEQ ID NO:141 | 21 | 6665.03 | 57.1 |
| | GUAUAACACCAAGGGGCUGCCAC | SEQ ID NO:142 | 23 | 7368.54 | 56.5 |
| LP3047 | GCAGCCCCUUGGUGUUAUACA | SEQ ID NO:143 | 21 | 6649.03 | 52.4 |
| | ^{.}UGUAUAACACCAAGGGGCUGCCA | SEQ ID NO:144 | 23 | 7369.53 | 52.2 |
| LP3073 | UCCCAGUGUCAGGUGGGAGUA | SEQ ID NO:145 | 21 | 6768.13 | 57.1 |
| | UACUCCCACCUGACACUGGGAUC | SEQ ID NO:146 | 23 | 7242.4 | 56.5 |
| LP3074 | CCCAGUGUCAGGUGGGAGUAC | SEQ ID NO:147 | 21 | 6767.14 | 61.9 |
| | GUACUCCCACCUGACACUGGGAU | SEQ ID NO:148 | 23 | 7282.43 | 56.5 |
| LP3075 | CCAGUGUCAGGUGGGAGUACU | SEQ ID NO:149 | 21 | 6768.13 | 57.1 |
| | AGUACUCCCACCUGACACUGGGA | SEQ ID NO:150 | 23 | 7305.47 | 56.5 |
| LP3079 | UGUCAGGUGGGAGUACUGCAA | SEQ ID NO:151 | 21 | 6792.16 | 52.4 |
| | UUGCAGUACUCCCACCUGACACU | SEQ ID NO:152 | 23 | 7203.36 | 52.2 |
| LP3084 | GGUGGGAGUACUGCAACCUGA | SEQ ID NO:153 | 21 | 6791.17 | 57.1 |
| | UCAGGUUGCAGUACUCCCACCUG | SEQ ID NO:154 | 23 | 7259.39 | 56.5 |
| LP3090 | AGUACUGCAACCUGACACGAU | SEQ ID NO:155 | 21 | 6679.11 | 47.6 |
| | AUCGUGUCAGGUUGCAGUACUCC | SEQ ID NO:156 | 23 | 7300.41 | 52.2 |
| LP3097 | CAACCUGACACGAUGCUCAGA | SEQ ID NO:157 | 21 | 6678.12 | 52.4 |
| | UCUGAGCAUCGUGUCAGGUUGCA | SEQ ID NO:158 | 23 | 7340.44 | 52.2 |
| LP3098 | AACCUGACACGAUGCUCAGAU | SEQ ID NO:159 | 21 | 6679.11 | 47.6 |
| | AUCUGAGCAUCGUGUCAGGUUGC | SEQ ID NO:160 | 23 | 7340.44 | 52.2 |
| LP3100 | CCUGACACGAUGCUCAGAUGC | SEQ ID NO:161 | 21 | 6671.08 | 57.1 |
| | GCAUCUGAGCAUCGUGUCAGGUU | SEQ ID NO:162 | 23 | 7340.44 | 52.2 |
| LP3104 | ACACGAUGCUCAGAUGCAGAA | SEQ ID NO:163 | 21 | 6742.18 | 47.6 |
| | UUCUGCAUCUGAGCAUCGUGUCA | SEQ ID NO:164 | 23 | 7261.37 | 47.8 |
| LP3275 | GGAAGAACUUGCCAAGCUUGG | SEQ ID NO:165 | 21 | 6775.17 | 52.4 |
| | CCAAGCUUGGCAAGUUCUUCCUG | SEQ ID NO:166 | 23 | 7260.38 | 52.2 |
| LP3276 | GAAGAACUUGCCAAGCUUGGU | SEQ ID NO:167 | 21 | 6736.13 | 47.6 |
| | ACCAAGCUUGGCAAGUUCUUCCU | SEQ ID NO:168 | 23 | 7244.38 | 47.8 |
| LP3279 | GAACUUGCCAAGCUUGGUCAU | SEQ ID NO:169 | 21 | 6673.06 | 47.6 |
| | AUGACCAAGCUUGGCAAGUUCUU | SEQ ID NO:170 | 23 | 7308.44 | 43.5 |
| LP3283 | UUGCCAAGCUUGGUCAUCUAU | SEQ ID NO:171 | 21 | 6610.98 | 42.9 |
| | AUAGAUGACCAAGCUUGGCAAGU | SEQ ID NO:172 | 23 | 7394.55 | 43.5 |
| LP3287 | CAAGCUUGGUCAUCUAUGACA | SEQ ID NO:173 | 21 | 6657.06 | 42.9 |
| | UGUCAUAGAUGACCAAGCUUGGC | SEQ ID NO:174 | 23 | 7347.48 | 47.8 |
| LP3294 | GGUCAUCUAUGACACCACACC | SEQ ID NO:175 | 21 | 6615.05 | 52.4 |
| | GGUGUGGUGUCAUAGAUGACCAA | SEQ ID NO:176 | 23 | 7427.54 | 47.8 |
| LP3299 | UCUAUGACACCACACCAGCAU | SEQ ID NO:177 | 21 | 6599.05 | 47.6 |
| | AUGCUGGUGUGGUGUCAUAGAUG | SEQ ID NO:178 | 23 | 7421.49 | 47.8 |
| LP3302 | AUGACACCACACCAGCAUAGU | SEQ ID NO:179 | 21 | 6662.12 | 47.6 |
| | ACUAUGCUGGUGUGGUGUCAUAG | SEQ ID NO:180 | 23 | 7381.46 | 47.8 |
| LP3306 | CACCACACCAGCAUAGUCGGA | SEQ ID NO:181 | 21 | 6677.13 | 57.1 |
| | UCCGACUAUGCUGGUGUGGUGUC | SEQ ID NO:182 | 23 | 7333.4 | 56.5 |
| LP3311 | CACCAGCAUAGUCGGACCCCA | SEQ ID NO:183 | 21 | 6653.1 | 61.9 |
| | UGGGGUCCGACUAUGCUGGUGUG | SEQ ID NO:184 | 23 | 7412.47 | 60.9 |
| LP3343 | AAAUGCUGGCCUGACCAGGAA | SEQ ID NO:185 | 21 | 6758.18 | 52.4 |
| | UUCCUGGUCAGGCCAGCAUUUGG | SEQ ID NO:186 | 23 | 7316.41 | 56.5 |
| LP3347 | GCUGGCCUGACCAGGAACUAC | SEQ ID NO:187 | 21 | 6710.12 | 61.9 |
| | GUAGUUCCUGGUCAGGCCAGCAU | SEQ ID NO:188 | 23 | 7339.45 | 56.5 |
| LP3348 | CUGGCCUGACCAGGAACUACU | SEQ ID NO:189 | 21 | 6671.08 | 57.1 |
| | AGUAGUUCCUGGUCAGGCCAGCA | SEQ ID NO:190 | 23 | 7362.49 | 56.5 |
| LP3350 | GGCCUGACCAGGAACUACUGC | SEQ ID NO:191 | 21 | 6710.12 | 61.9 |
| | GCAGUAGUUCCUGGUCAGGCCAG | SEQ ID NO:192 | 23 | 7378.49 | 60.9 |
| LP3353 | CUGACCAGGAACUACUGCAGG | SEQ ID NO:193 | 21 | 6734.15 | 57.1 |
| | CCUGCAGUAGUUCCUGGUCAGGC | SEQ ID NO:194 | 23 | 7315.42 | 60.9 |
| LP3355 | GACCAGGAACUACUGCAGGAA | SEQ ID NO:195 | 21 | 6781.22 | 52.4 |
| | UUCCUGCAGUAGUUCCUGGUCAG | SEQ ID NO:196 | 23 | 7277.37 | 52.2 |
| LP3357 | CCAGGAACUACUGCAGGAAUC | SEQ ID NO:197 | 21 | 6718.15 | 52.4 |
| | GAUUCCUGCAGUAGUUCCUGGUC | SEQ ID NO:198 | 23 | 7277.37 | 52.2 |
| LP3362 | AACUACUGCAGGAAUCCAGAU | SEQ ID NO:199 | 21 | 6703.14 | 42.9 |
| | AUCUGGAUUCCUGCAGUAGUUCC | SEQ ID NO:200 | 23 | 7261.37 | 47.8 |
| LP3365 | UACUGCAGGAAUCCAGAUGCU | SEQ ID NO:201 | 21 | 6696.1 | 47.6 |
| | AGCAUCUGGAUUCCUGCAGUAGU | SEQ ID NO:202 | 23 | 7324.44 | 47.8 |
| LP3367 | CUGCAGGAAUCCAGAUGCUGA | SEQ ID NO:203 | 21 | 6735.14 | 52.4 |
| | UCAGCAUCUGGAUUCCUGCAGUA | SEQ ID NO:204 | 23 | 7284.41 | 47.8 |
| LP3370 | CAGGAAUCCAGAUGCUGAGAU | SEQ ID NO:205 | 21 | 6759.17 | 47.6 |
| | AUCUCAGCAUCUGGAUUCCUGCA | SEQ ID NO:206 | 23 | 7244.38 | 47.8 |
| LP3372 | GGAAUCCAGAUGCUGAGAUUC | SEQ ID NO:207 | 21 | 6736.13 | 47.6 |
| | GAAUCUCAGCAUCUGGAUUCCUG | SEQ ID NO:208 | 23 | 7284.41 | 47.8 |
| LP3373 | GAAUCCAGAUGCUGAGAUUCG | SEQ ID NO:209 | 21 | 6736.13 | 47.6 |
| | CGAAUCUCAGCAUCUGGAUUCCU | SEQ ID NO:210 | 23 | 7244.38 | 47.8 |
| LP3378 | CAGAUGCUGAGAUUCGCCCUU | SEQ ID NO:211 | 21 | 6649.03 | 52.4 |
| | AAGGGCGAAUCUCAGCAUCUGGA | SEQ ID NO:212 | 23 | 7409.56 | 52.2 |
| LP3380 | GAUGCUGAGAUUCGCCCUUGG | SEQ ID NO:213 | 21 | 6705.06 | 57.1 |
| | CCAAGGGCGAAUCUCAGCAUCUG | SEQ ID NO:214 | 23 | 7345.5 | 56.5 |
| LP3385 | UGAGAUUCGCCCUUGGUGUUA | SEQ ID NO:215 | 21 | 6667.01 | 47.6 |
| | UAACACCAAGGGCGAAUCUCAGC | SEQ ID NO:216 | 23 | 7352.54 | 52.2 |
| LP3414 | AUCCCAGUGUCAGGUGGGAGU | SEQ ID NO:217 | 21 | 6768.13 | 57.1 |
| | ACUCCCACCUGACACUGGGAUCC | SEQ ID NO:218 | 23 | 7241.41 | 60.9 |
| LP3416 | CCCAGUGUCAGGUGGGAGUAC | SEQ ID NO:219 | 21 | 6767.14 | 61.9 |
| | GUACUCCCACCUGACACUGGGAU | SEQ ID NO:220 | 23 | 7282.43 | 56.5 |
| LP3426 | GGUGGGAGUACUGCAACCUGA | SEQ ID NO:221 | 21 | 6791.17 | 57.1 |
| | UCAGGUUGCAGUACUCCCACCUG | SEQ ID NO:222 | 23 | 7259.39 | 56.5 |
| LP3568 | CCAUGGUGAUGGACAGAGUUA | SEQ ID NO:223 | 21 | 6776.16 | 47.6 |
| | UAACUCUGUCCAUCACCAUGGUA | SEQ ID NO:224 | 23 | 7228.38 | 43.5 |
| LP3572 | GGUGAUGGACAGAGUUAUCGA | SEQ ID NO:225 | 21 | 6816.19 | 47.6 |
| | UCGAUAACUCUGUCCAUCACCAU | SEQ ID NO:226 | 23 | 7188.35 | 43.5 |
| LP3576 | AUGGACAGAGUUAUCGAGGCU | SEQ ID NO:227 | 21 | 6776.16 | 47.6 |
| | AGCCUCGAUAACUCUGUCCAUCA | SEQ ID NO:228 | 23 | 7227.39 | 47.8 |
| LP3578 | GGACAGAGUUAUCGAGGCUCA | SEQ ID NO:229 | 21 | 6775.17 | 52.4 |
| | UGAGCCUCGAUAACUCUGUCCAU | SEQ ID NO:230 | 23 | 7244.38 | 47.8 |
| LP3579 | GACAGAGUUAUCGAGGCUCAU | SEQ ID NO:231 | 21 | 6736.13 | 47.6 |
| | AUGAGCCUCGAUAACUCUGUCCA | SEQ ID NO:232 | 23 | 7267.42 | 47.8 |
| LP3582 | AGAGUUAUCGAGGCUCAUUCU | SEQ ID NO:233 | 21 | 6674.05 | 42.9 |
| | AGAAUGAGCCUCGAUAACUCUGU | SEQ ID NO:234 | 23 | 7331.48 | 43.5 |
| LP3607 | CACUGUCACAGGAAGGACAUG | SEQ ID NO:235 | 21 | 6758.18 | 52.4 |
| | CAUGUCCUUCCUGUGACAGUGGU | SEQ ID NO:236 | 23 | 7277.37 | 52.2 |
| LP3608 | ACUGUCACAGGAAGGACAUGU | SEQ ID NO:237 | 21 | 6759.17 | 47.6 |
| | ACAUGUCCUUCCUGUGACAGUGG | SEQ ID NO:238 | 23 | 7300.41 | 52.2 |
| LP3612 | UCACAGGAAGGACAUGUCAGU | SEQ ID NO:239 | 21 | 6759.17 | 47.6 |
| | ACUGACAUGUCCUUCCUGUGACA | SEQ ID NO:240 | 23 | 7244.38 | 47.8 |
| LP3617 | GGAAGGACAUGUCAGUCUUGG | SEQ ID NO:241 | 21 | 6792.16 | 52.4 |
| | CCAAGACUGACAUGUCCUUCCUG | SEQ ID NO:242 | 23 | 7243.39 | 52.2 |
| LP3618 | GAAGGACAUGUCAGUCUUGGU | SEQ ID NO:243 | 21 | 6753.12 | 47.6 |
| | ACCAAGACUGACAUGUCCUUCCU | SEQ ID NO:244 | 23 | 7227.39 | 47.8 |
| LP3623 | ACAUGUCAGUCUUGGUCCUCU | SEQ ID NO:245 | 21 | 6586.95 | 47.6 |
| | AGAGGACCAAGACUGACAUGUCC | SEQ ID NO:246 | 23 | 7392.57 | 52.2 |
| LP3627 | GUCAGUCUUGGUCCUCUAUGA | SEQ ID NO:247 | 21 | 6626.98 | 47.6 |
| | UCAUAGAGGACCAAGACUGACAU | SEQ ID NO:248 | 23 | 7377.56 | 43.5 |
| LP3629 | CAGUCUUGGUCCUCUAUGACA | SEQ ID NO:249 | 21 | 6609.99 | 47.6 |
| | UGUCAUAGAGGACCAAGACUGAC | SEQ ID NO:250 | 23 | 7393.56 | 47.8 |
| LP3632 | UCUUGGUCCUCUAUGACACCA | SEQ ID NO:251 | 21 | 6569.96 | 47.6 |
| | UGGUGUCAUAGAGGACCAAGACU | SEQ ID NO:252 | 23 | 7410.55 | 47.8 |
| LP3636 | GGUCCUCUAUGACACCACACU | SEQ ID NO:253 | 21 | 6592.01 | 52.4 |
| | AGUGUGGUGUCAUAGAGGACCAA | SEQ ID NO:254 | 23 | 7450.58 | 47.8 |
| LP3641 | UCUAUGACACCACACUGGCAU | SEQ ID NO:255 | 21 | 6616.04 | 47.6 |
| | AUGCCAGUGUGGUGUCAUAGAGG | SEQ ID NO:256 | 23 | 7443.54 | 52.2 |
| LP3645 | UGACACCACACUGGCAUCAGA | SEQ ID NO:257 | 21 | 6678.12 | 52.4 |
| | UCUGAUGCCAGUGUGGUGUCAUA | SEQ ID NO:258 | 23 | 7341.43 | 47.8 |
| LP3651 | CACACUGGCAUCAGAGGACAA | SEQ ID NO:259 | 21 | 6741.19 | 52.4 |
| | UUGUCCUCUGAUGCCAGUGUGGU | SEQ ID NO:260 | 23 | 7294.36 | 52.2 |
| LP3657 | GGCAUCAGAGGACAACAGAAU | SEQ ID NO:261 | 21 | 6805.25 | 47.6 |
| | AUUCUGUUGUCCUCUGAUGCCAG | SEQ ID NO:262 | 23 | 7238.33 | 47.8 |
| LP3659 | CAUCAGAGGACAACAGAAUAU | SEQ ID NO:263 | 21 | 6750.21 | 38.1 |
| | AUAUUCUGUUGUCCUCUGAUGCC | SEQ ID NO:264 | 23 | 7199.29 | 43.5 |
| LP3662 | CAGAGGACAACAGAAUAUUAU | SEQ ID NO:265 | 21 | 6751.2 | 33.3 |
| | AUAAUAUUCUGUUGUCCUCUGAU | SEQ ID NO:266 | 23 | 7208.31 | 30.4 |
| LP3662 | CAGAGGACAACAGAAUAUUAU | SEQ ID NO:267 | 21 | 6751.2 | 33.3 |
| | AUAAUAUUCUGUUGUCCUCUGAU | SEQ ID NO:268 | 23 | 7208.31 | 30.4 |
| LP3664 | GAGGACAACAGAAUAUUAUCC | SEQ ID NO:269 | 21 | 6727.17 | 38.1 |
| | GGAUAAUAUUCUGUUGUCCUCUG | SEQ ID NO:270 | 23 | 7263.35 | 39.1 |
| LP3689 | GGUGGCCUGACCAGGAACUAC | SEQ ID NO:271 | 21 | 6750.15 | 61.9 |
| | GUAGUUCCUGGUCAGGCCACCAU | SEQ ID NO:272 | 23 | 7299.42 | 56.5 |
| LP3690 | GUGGCCUGACCAGGAACUACU | SEQ ID NO:273 | 21 | 6711.11 | 57.1 |
| | AGUAGUUCCUGGUCAGGCCACCA | SEQ ID NO:274 | 23 | 7322.46 | 56.5 |
| LP3692 | GGCCUGACCAGGAACUACUGC | SEQ ID NO:275 | 21 | 6710.12 | 61.9 |
| | GCAGUAGUUCCUGGUCAGGCCAC | SEQ ID NO:276 | 23 | 7338.46 | 60.9 |
| LP3695 | CUGACCAGGAACUACUGCAGG | SEQ ID NO:277 | 21 | 6734.15 | 57.1 |
| | CCUGCAGUAGUUCCUGGUCAGGC | SEQ ID NO:278 | 23 | 7315.42 | 60.9 |
| LP3699 | CCAGGAACUACUGCAGGAAUC | SEQ ID NO:279 | 21 | 6718.15 | 52.4 |
| | GAUUCCUGCAGUAGUUCCUGGUC | SEQ ID NO:280 | 23 | 7277.37 | 52.2 |
| LP3709 | CUGCAGGAAUCCAGAUGCUGA | SEQ ID NO:281 | 21 | 6735.14 | 52.4 |
| | UCAGCAUCUGGAUUCCUGCAGUA | SEQ ID NO:282 | 23 | 7284.41 | 47.8 |
| LP3776 | UACUGCAACCUGACACAAUGU | SEQ ID NO:283 | 21 | 6640.07 | 42.9 |
| | ACAUUGUGUCAGGUUGCAGUACU | SEQ ID NO:284 | 23 | 7325.43 | 43.5 |
| LP3781 | CAACCUGACACAAUGUCCAGU | SEQ ID NO:285 | 21 | 6639.08 | 47.6 |
| | ACUGGACAUUGUGUCAGGUUGCA | SEQ ID NO:286 | 23 | 7364.47 | 47.8 |
| LP3785 | CUGACACAAUGUCCAGUGACA | SEQ ID NO:287 | 21 | 6679.11 | 47.6 |
| | UGUCACUGGACAUUGUGUCAGGU | SEQ ID NO:288 | 23 | 7341.43 | 47.8 |
| LP3787 | GACACAAUGUCCAGUGACAGA | SEQ ID NO:289 | 21 | 6742.18 | 47.6 |
| | UCUGUCACUGGACAUUGUGUCAG | SEQ ID NO:290 | 23 | 7301.4 | 47.8 |
| LP3789 | CACAAUGUCCAGUGACAGAAU | SEQ ID NO:291 | 21 | 6703.14 | 42.9 |
| | AUUCUGUCACUGGACAUUGUGUC | SEQ ID NO:292 | 23 | 7262.36 | 43.5 |
| LP3794 | UGUCCAGUGACAGAAUCAAGU | SEQ ID NO:293 | 21 | 6720.13 | 42.9 |
| | ACUUGAUUCUGUCACUGGACAUU | SEQ ID NO:294 | 23 | 7246.36 | 39.1 |
| LP3798 | CAGUGACAGAAUCAAGUGUCC | SEQ ID NO:295 | 21 | 6719.14 | 47.6 |
| | GGACACUUGAUUCUGUCACUGGA | SEQ ID NO:296 | 23 | 7324.44 | 47.8 |
| LP3799 | AGUGACAGAAUCAAGUGUCCU | SEQ ID NO:297 | 21 | 6720.13 | 42.9 |
| | AGGACACUUGAUUCUGUCACUGG | SEQ ID NO:298 | 23 | 7324.44 | 47.8 |
| LP3875 | CAGGACUGCUACCAUGGUGAU | SEQ ID NO:299 | 21 | 6712.1 | 52.4 |
| | AUCACCAUGGUAGCAGUCCUGGA | SEQ ID NO:300 | 23 | 7346.49 | 52.2 |
| LP3880 | CUGCUACCAUGGUGAUGGACA | SEQ ID NO:301 | 21 | 6712.1 | 52.4 |
| | UGUCCAUCACCAUGGUAGCAGUC | SEQ ID NO:302 | 23 | 7283.42 | 52.2 |
| LP3886 | CCAUGGUGAUGGACAGAGUUA | SEQ ID NO:303 | 21 | 6776.16 | 47.6 |
| | UAACUCUGUCCAUCACCAUGGUA | SEQ ID NO:304 | 23 | 7228.38 | 43.5 |
| LP3935 | GGAAGGACAUGUCAGUCUUGG | SEQ ID NO:305 | 21 | 6792.16 | 52.4 |
| | CCAAGACUGACAUGUCCUUCCUG | SEQ ID NO:306 | 23 | 7243.39 | 52.2 |
| LP3947 | CAGUCUUGGUCCUCUAUGACA | SEQ ID NO:307 | 21 | 6609.99 | 47.6 |
| | UGUCAUAGAGGACCAAGACUGAC | SEQ ID NO:308 | 23 | 7393.56 | 47.8 |
| LP3954 | GGUCCUCUAUGACACCACACU | SEQ ID NO:309 | 21 | 6592.01 | 52.4 |
| | AGUGUGGUGUCAUAGAGGACCAA | SEQ ID NO:310 | 23 | 7450.58 | 47.8 |
| LP4027 | CUGCAGGAAUCCAGAUGCUGA | SEQ ID NO:311 | 21 | 6735.14 | 52.4 |
| | UCAGCAUCUGGAUUCCUGCAGUA | SEQ ID NO:312 | 23 | 7284.41 | 47.8 |
| LP4032 | GGAAUCCAGAUGCUGAGAUUC | SEQ ID NO:313 | 21 | 6736.13 | 47.6 |
| | GAAUCUCAGCAUCUGGAUUCCUG | SEQ ID NO:314 | 23 | 7284.41 | 47.8 |
| LP4033 | GAAUCCAGAUGCUGAGAUUCG | SEQ ID NO:315 | 21 | 6736.13 | 47.6 |
| | CGAAUCUCAGCAUCUGGAUUCCU | SEQ ID NO:316 | 23 | 7244.38 | 47.8 |
| LP4040 | GAUGCUGAGAUUCGCCCUUGG | SEQ ID NO:317 | 21 | 6705.06 | 57.1 |
| | CCAAGGGCGAAUCUCAGCAUCUG | SEQ ID NO:318 | 23 | 7345.5 | 56.5 |
| LP4046 | GAGAUUCGCCCUUGGUGUUAU | SEQ ID NO:319 | 21 | 6667.01 | 47.6 |
| | AUAACACCAAGGGCGAAUCUCAG | SEQ ID NO:320 | 23 | 7376.57 | 47.8 |
| LP4048 | GAUUCGCCCUUGGUGUUAUAC | SEQ ID NO:321 | 21 | 6626.98 | 47.6 |
| | GUAUAACACCAAGGGCGAAUCUC | SEQ ID NO:322 | 23 | 7353.53 | 47.8 |
| LP4051 | UCGCCCUUGGUGUUAUACCAU | SEQ ID NO:323 | 21 | 6586.95 | 47.6 |
| | AUGGUAUAACACCAAGGGCGAAU | SEQ ID NO:324 | 23 | 7417.59 | 43.5 |
| LP4052 | CGCCCUUGGUGUUAUACCAUG | SEQ ID NO:325 | 21 | 6625.99 | 52.4 |
| | CAUGGUAUAACACCAAGGGCGAA | SEQ ID NO:326 | 23 | 7416.6 | 47.8 |
| LP4055 | CCUUGGUGUUAUACCAUGGAU | SEQ ID NO:327 | 21 | 6651.01 | 42.9 |
| | AUCCAUGGUAUAACACCAAGGGC | SEQ ID NO:328 | 23 | 7353.53 | 47.8 |
| LP4056 | CUUGGUGUUAUACCAUGGAUC | SEQ ID NO:329 | 21 | 6651.01 | 42.9 |
| | GAUCCAUGGUAUAACACCAAGGG | SEQ ID NO:330 | 23 | 7393.56 | 47.8 |
| LP4059 | GGUGUUAUACCAUGGAUCCCA | SEQ ID NO:331 | 21 | 6673.06 | 47.6 |
| | UGGGAUCCAUGGUAUAACACCAA | SEQ ID NO:332 | 23 | 7354.52 | 43.5 |
| LP4382 | GAUGCUGAGAUUCGCCCUUGG | SEQ ID NO:333 | 21 | 6705.06 | 57.1 |
| | CCAAGGGCGAAUCUCAGCAUCUG | SEQ ID NO:334 | 23 | 7345.5 | 56.5 |
| LP4389 | AGAUUCGCCCUUGGUGUUACA | SEQ ID NO:335 | 21 | 6650.02 | 47.6 |
| | UGUAACACCAAGGGCGAAUCUCA | SEQ ID NO:336 | 23 | 7353.53 | 47.8 |
| LP4393 | UCGCCCUUGGUGUUACACCAU | SEQ ID NO:337 | 21 | 6585.96 | 52.4 |
| | AUGGUGUAACACCAAGGGCGAAU | SEQ ID NO:338 | 23 | 7433.59 | 47.8 |
| LP4394 | CGCCCUUGGUGUUACACCAUG | SEQ ID NO:339 | 21 | 6625 | 57.1 |
| | CAUGGUGUAACACCAAGGGCGAA | SEQ ID NO:340 | 23 | 7432.6 | 52.2 |
| LP4397 | CCUUGGUGUUACACCAUGGAU | SEQ ID NO:341 | 21 | 6650.02 | 47.6 |
| | AUCCAUGGUGUAACACCAAGGGC | SEQ ID NO:342 | 23 | 7369.53 | 52.2 |
| LP4398 | CUUGGUGUUACACCAUGGAUC | SEQ ID NO:343 | 21 | 6650.02 | 47.6 |
| | GAUCCAUGGUGUAACACCAAGGG | SEQ ID NO:344 | 23 | 7409.56 | 52.2 |
| LP4403 | UGUUACACCAUGGAUCCCAGU | SEQ ID NO:345 | 21 | 6633.03 | 47.6 |
| | ACUGGGAUCCAUGGUGUAACACC | SEQ ID NO:346 | 23 | 7346.49 | 52.2 |
| LP4407 | ACACCAUGGAUCCCAGUGUCA | SEQ ID NO:347 | 21 | 6655.08 | 52.4 |
| | UGACACUGGGAUCCAUGGUGUAA | SEQ ID NO:348 | 23 | 7387.51 | 47.8 |
| LP4410 | CCAUGGAUCCCAGUGUCAGGU | SEQ ID NO:349 | 21 | 6688.07 | 57.1 |
| | ACCUGACACUGGGAUCCAUGGUG | SEQ ID NO:350 | 23 | 7362.49 | 56.5 |
| LP4418 | CCCAGUGUCAGGUGGGAGUAC | SEQ ID NO:351 | 21 | 6767.14 | 61.9 |
| | GUACUCCCACCUGACACUGGGAU | SEQ ID NO:352 | 23 | 7282.43 | 56.5 |
| LP4428 | GGUGGGAGUACUGCAACCUGA | SEQ ID NO:353 | 21 | 6791.17 | 57.1 |
| | UCAGGUUGCAGUACUCCCACCUG | SEQ ID NO:354 | 23 | 7259.39 | 56.5 |
| LP4503 | UUCCAAGCACAGAGGCUCCUU | SEQ ID NO:355 | 21 | 6632.04 | 52.4 |
| | AAGGAGCCUCUGUGCUUGGAACC | SEQ ID NO:356 | 23 | 7362.49 | 56.5 |
| LP4508 | AGCACAGAGGCUCCUUCUGAA | SEQ ID NO:357 | 21 | 6695.11 | 52.4 |
| | UUCAGAAGGAGCCUCUGUGCUUG | SEQ ID NO:358 | 23 | 7340.44 | 52.2 |
| LP4510 | CACAGAGGCUCCUUCUGAACA | SEQ ID NO:359 | 21 | 6655.08 | 52.4 |
| | UGUUCAGAAGGAGCCUCUGUGCU | SEQ ID NO:360 | 23 | 7340.44 | 52.2 |
| LP4514 | GAGGCUCCUUCUGAACAAGCA | SEQ ID NO:361 | 21 | 6695.11 | 52.4 |
| | UGCUUGUUCAGAAGGAGCCUCUG | SEQ ID NO:362 | 23 | 7340.44 | 52.2 |
| LP4516 | GGCUCCUUCUGAACAAGCACC | SEQ ID NO:363 | 21 | 6631.05 | 57.1 |
| | GGUGCUUGUUCAGAAGGAGCCUC | SEQ ID NO:364 | 23 | 7379.48 | 56.5 |
| LP4520 | CCUUCUGAACAAGCACCACCU | SEQ ID NO:365 | 21 | 6575.02 | 52.4 |
| | AGGUGGUGCUUGUUCAGAAGGAG | SEQ ID NO:366 | 23 | 7483.57 | 52.2 |
| LP4525 | UGAACAAGCACCACCUGAGAA | SEQ ID NO:367 | 21 | 6725.19 | 47.6 |
| | UUCUCAGGUGGUGCUUGUUCAGA | SEQ ID NO:368 | 23 | 7318.39 | 47.8 |
| LP4532 | GCACCACCUGAGAAAAGCCCU | SEQ ID NO:369 | 21 | 6677.13 | 57.1 |
| | AGGGCUUUUCUCAGGUGGUGCUU | SEQ ID NO:370 | 23 | 7334.39 | 52.2 |
| LP4536 | CACCUGAGAAAAGCCCUGUGG | SEQ ID NO:371 | 21 | 6734.15 | 57.1 |
| | CCACAGGGCUUUUCUCAGGUGGU | SEQ ID NO:372 | 23 | 7316.41 | 56.5 |
| LP4537 | ACCUGAGAAAAGCCCUGUGGU | SEQ ID NO:373 | 21 | 6735.14 | 52.4 |
| | ACCACAGGGCUUUUCUCAGGUGG | SEQ ID NO:374 | 23 | 7339.45 | 56.5 |
| LP4544 | AAAAGCCCUGUGGUCCAGGAU | SEQ ID NO:375 | 21 | 6735.14 | 52.4 |
| | AUCCUGGACCACAGGGCUUUUCU | SEQ ID NO:376 | 23 | 7260.38 | 52.2 |
| LP4549 | CCCUGUGGUCCAGGAUUGCUA | SEQ ID NO:377 | 21 | 6665.03 | 57.1 |
| | UAGCAAUCCUGGACCACAGGGCU | SEQ ID NO:378 | 23 | 7345.5 | 56.5 |
| LP4551 | CUGUGGUCCAGGAUUGCUACC | SEQ ID NO:379 | 21 | 6665.03 | 57.1 |
| | GGUAGCAAUCCUGGACCACAGGG | SEQ ID NO:380 | 23 | 7424.57 | 60.9 |
| LP4553 | GUGGUCCAGGAUUGCUACCAU | SEQ ID NO:381 | 21 | 6689.06 | 52.4 |
| | AUGGUAGCAAUCCUGGACCACAG | SEQ ID NO:382 | 23 | 7369.53 | 52.2 |
| LP4555 | GGUCCAGGAUUGCUACCAUGG | SEQ ID NO:383 | 21 | 6728.1 | 57.1 |
| | CCAUGGUAGCAAUCCUGGACCAC | SEQ ID NO:384 | 23 | 7305.47 | 56.5 |
| LP4559 | CAGGAUUGCUACCAUGGUGAU | SEQ ID NO:385 | 21 | 6713.09 | 47.6 |
| | AUCACCAUGGUAGCAAUCCUGGA | SEQ ID NO:386 | 23 | 7330.49 | 47.8 |
| LP4638 | GGUCAUCUAUGAUACCACACU | SEQ ID NO:387 | 21 | 6617.03 | 42.9 |
| | AGUGUGGUAUCAUAGAUGACCAA | SEQ ID NO:388 | 23 | 7395.54 | 39.1 |
| LP4643 | UCUAUGAUACCACACUGGCAU | SEQ ID NO:389 | 21 | 6617.03 | 42.9 |
| | AUGCCAGUGUGGUAUCAUAGAUG | SEQ ID NO:390 | 23 | 7388.5 | 43.5 |
| LP4650 | UACCACACUGGCAUCAGAGGA | SEQ ID NO:391 | 21 | 6718.15 | 52.4 |
| | UCCUCUGAUGCCAGUGUGGUAUC | SEQ ID NO:392 | 23 | 7277.37 | 52.2 |
| LP4658 | UGGCAUCAGAGGACCCCAGAA | SEQ ID NO:393 | 21 | 6757.19 | 57.1 |
| | UUCUGGGGUCCUCUGAUGCCAGU | SEQ ID NO:394 | 23 | 7293.37 | 56.5 |
| LP4661 | CAUCAGAGGACCCCAGAAAAC | SEQ ID NO:395 | 21 | 6724.2 | 52.4 |
| | GUUUUCUGGGGUCCUCUGAUGCC | SEQ ID NO:396 | 23 | 7270.33 | 56.5 |
| LP4662 | AUCAGAGGACCCCAGAAAACU | SEQ ID NO:397 | 21 | 6725.19 | 47.6 |
| | AGUUUUCUGGGGUCCUCUGAUGC | SEQ ID NO:398 | 23 | 7294.36 | 52.2 |
| LP4669 | GACCCCAGAAAACUACCCAAA | SEQ ID NO:399 | 21 | 6668.17 | 47.6 |
| | UUUGGGUAGUUUUCUGGGGUCCU | SEQ ID NO:400 | 23 | 7312.34 | 47.8 |
| LP4670 | ACCCCAGAAAACUACCCAAAU | SEQ ID NO:401 | 21 | 6629.13 | 42.9 |
| | AUUUGGGUAGUUUUCUGGGGUCC | SEQ ID NO:402 | 23 | 7335.38 | 47.8 |
| LP4671 | CCCCAGAAAACUACCCAAAUG | SEQ ID NO:403 | 21 | 6645.13 | 47.6 |
| | CAUUUGGGUAGUUUUCUGGGGUC | SEQ ID NO:404 | 23 | 7335.38 | 47.8 |
| LP4678 | AAACUACCCAAAUGCUGGCCU | SEQ ID NO:405 | 21 | 6639.08 | 47.6 |
| | AGGCCAGCAUUUGGGUAGUUUUC | SEQ ID NO:406 | 23 | 7341.43 | 47.8 |
| LP4707 | ACUACUGCAGGAAUCCAGAUU | SEQ ID NO:407 | 21 | 6680.1 | 42.9 |
| | AAUCUGGAUUCCUGCAGUAGUUC | SEQ ID NO:408 | 23 | 7285.4 | 43.5 |
| LP4709 | UACUGCAGGAAUCCAGAUUCU | SEQ ID NO:409 | 21 | 6657.06 | 42.9 |
| | AGAAUCUGGAUUCCUGCAGUAGU | SEQ ID NO:410 | 23 | 7348.47 | 43.5 |
| LP4711 | CUGCAGGAAUCCAGAUUCUGG | SEQ ID NO:411 | 21 | 6712.1 | 52.4 |
| | CCAGAAUCUGGAUUCCUGCAGUA | SEQ ID NO:412 | 23 | 7307.45 | 47.8 |
| LP4714 | CAGGAAUCCAGAUUCUGGGAA | SEQ ID NO:413 | 21 | 6759.17 | 47.6 |
| | UUCCCAGAAUCUGGAUUCCUGCA | SEQ ID NO:414 | 23 | 7244.38 | 47.8 |
| LP4716 | GGAAUCCAGAUUCUGGGAAAC | SEQ ID NO:415 | 21 | 6759.17 | 47.6 |
| | GUUUCCCAGAAUCUGGAUUCCUG | SEQ ID NO:416 | 23 | 7261.37 | 47.8 |
| LP4717 | GAAUCCAGAUUCUGGGAAACA | SEQ ID NO:417 | 21 | 6743.17 | 42.9 |
| | UGUUUCCCAGAAUCUGGAUUCCU | SEQ ID NO:418 | 23 | 7222.33 | 43.5 |
| LP4722 | CAGAUUCUGGGAAACAACCCU | SEQ ID NO:419 | 21 | 6679.11 | 47.6 |
| | AGGGUUGUUUCCCAGAAUCUGGA | SEQ ID NO:420 | 23 | 7364.47 | 47.8 |
| LP4724 | GAUUCUGGGAAACAACCCUGG | SEQ ID NO:421 | 21 | 6735.14 | 52.4 |
| | CCAGGGUUGUUUCCCAGAAUCUG | SEQ ID NO:422 | 23 | 7300.41 | 52.2 |
| LP4727 | UCUGGGAAACAACCCUGGUGU | SEQ ID NO:423 | 21 | 6712.1 | 52.4 |
| | ACACCAGGGUUGUUUCCCAGAAU | SEQ ID NO:424 | 23 | 7307.45 | 47.8 |
| LP4790 | ACACAAUGCUCAGAAACAGAA | SEQ ID NO:425 | 21 | 6733.22 | 38.1 |
| | UUCUGUUUCUGAGCAUUGUGUCA | SEQ ID NO:426 | 23 | 7240.31 | 39.1 |
| LP4796 | UGCUCAGAAACAGAAUCAGGU | SEQ ID NO:427 | 21 | 6743.17 | 42.9 |
| | ACCUGAUUCUGUUUCUGAGCAUU | SEQ ID NO:428 | 23 | 7223.32 | 39.1 |
| LP4798 | CUCAGAAACAGAAUCAGGUGU | SEQ ID NO:429 | 21 | 6743.17 | 42.9 |
| | ACACCUGAUUCUGUUUCUGAGCA | SEQ ID NO:430 | 23 | 7245.37 | 43.5 |
| LP4800 | CAGAAACAGAAUCAGGUGUCC | SEQ ID NO:431 | 21 | 6742.18 | 47.6 |
| | GGACACCUGAUUCUGUUUCUGAG | SEQ ID NO:432 | 23 | 7301.4 | 47.8 |
| LP4801 | AGAAACAGAAUCAGGUGUCCU | SEQ ID NO:433 | 21 | 6743.17 | 42.9 |
| | AGGACACCUGAUUCUGUUUCUGA | SEQ ID NO:434 | 23 | 7285.4 | 43.5 |
| LP4808 | GAAUCAGGUGUCCUAGAGACU | SEQ ID NO:435 | 21 | 6736.13 | 47.6 |
| | AGUCUCUAGGACACCUGAUUCUG | SEQ ID NO:436 | 23 | 7284.41 | 47.8 |
| LP4814 | GGUGUCCUAGAGACUCCCACU | SEQ ID NO:437 | 21 | 6648.04 | 57.1 |
| | AGUGGGAGUCUCUAGGACACCUG | SEQ ID NO:438 | 23 | 7402.52 | 56.5 |
| LP4819 | CCUAGAGACUCCCACUGUUGU | SEQ ID NO:439 | 21 | 6609 | 52.4 |
| | ACAACAGUGGGAGUCUCUAGGAC | SEQ ID NO:440 | 23 | 7409.56 | 52.2 |
| LP4927a | GAGUUAUCGAGGCACAUUCUC | SEQ ID NO:441 | 21 | 6673.06 | 47.6 |
| | GAGAAUGUGCCUCGAUAACUCUG | SEQ ID NO:442 | 23 | 7347.48 | 47.8 |
| LP4934 | CGAGGCACAUUCUCCACCACU | SEQ ID NO:443 | 21 | 6591.02 | 57.1 |
| | AGUGGUGGAGAAUGUGCCUCGAU | SEQ ID NO:444 | 23 | 7443.54 | 52.2 |
| LP4944 | UCUCCACCACUGUCACAGGAA | SEQ ID NO:445 | 21 | 6615.05 | 52.4 |
| | UUCCUGUGACAGUGGUGGAGAAU | SEQ ID NO:446 | 23 | 7404.5 | 47.8 |
| LP4945 | CUCCACCACUGUCACAGGAAG | SEQ ID NO:447 | 21 | 6654.09 | 57.1 |
| | CUUCCUGUGACAGUGGUGGAGAA | SEQ ID NO:448 | 23 | 7403.51 | 52.2 |
| LP4950 | CCACUGUCACAGGAAGGACAU | SEQ ID NO:449 | 21 | 6718.15 | 52.4 |
| | AUGUCCUUCCUGUGACAGUGGUG | SEQ ID NO:450 | 23 | 7317.4 | 52.2 |
| LP4951 | CACUGUCACAGGAAGGACAUG | SEQ ID NO:451 | 21 | 6758.18 | 52.4 |
| | CAUGUCCUUCCUGUGACAGUGGU | SEQ ID NO:452 | 23 | 7277.37 | 52.2 |
| LP4961 | GGAAGGACAUGUCAAUCUUGG | SEQ ID NO:453 | 21 | 6776.16 | 47.6 |
| | CCAAGAUUGACAUGUCCUUCCUG | SEQ ID NO:454 | 23 | 7244.38 | 47.8 |
| LP4962 | GAAGGACAUGUCAAUCUUGGU | SEQ ID NO:455 | 21 | 6737.12 | 42.9 |
| | ACCAAGAUUGACAUGUCCUUCCU | SEQ ID NO:456 | 23 | 7228.38 | 43.5 |
| LP4966 | GACAUGUCAAUCUUGGUCAUC | SEQ ID NO:457 | 21 | 6634.02 | 42.9 |
| | GAUGACCAAGAUUGACAUGUCCU | SEQ ID NO:458 | 23 | 7331.48 | 43.5 |
| LP4969 | AUGUCAAUCUUGGUCAUCCAU | SEQ ID NO:459 | 21 | 6594.98 | 38.1 |
| | AUGGAUGACCAAGAUUGACAUGU | SEQ ID NO:460 | 23 | 7395.54 | 39.1 |
| LP4973 | CAAUCUUGGUCAUCCAUGACA | SEQ ID NO:461 | 21 | 6617.03 | 42.9 |
| | UGUCAUGGAUGACCAAGAUUGAC | SEQ ID NO:462 | 23 | 7371.51 | 43.5 |
| LP4978 | UUGGUCAUCCAUGACACCACA | SEQ ID NO:463 | 21 | 6616.04 | 47.6 |
| | UGUGGUGUCAUGGAUGACCAAGA | SEQ ID NO:464 | 23 | 7427.54 | 47.8 |
| LP4980 | GGUCAUCCAUGACACCACACC | SEQ ID NO:465 | 21 | 6614.06 | 57.1 |
| | GGUGUGGUGUCAUGGAUGACCAA | SEQ ID NO:466 | 23 | 7443.54 | 52.2 |
| LP5118 | AGUACUGCAACCUGACGCGAU | SEQ ID NO:467 | 21 | 6695.11 | 52.4 |
| | AUCGCGUCAGGUUGCAGUACUCC | SEQ ID NO:468 | 23 | 7299.42 | 56.5 |
| LP5121 | ACUGCAACCUGACGCGAUGCU | SEQ ID NO:469 | 21 | 6671.08 | 57.1 |
| | AGCAUCGCGUCAGGUUGCAGUAC | SEQ ID NO:470 | 23 | 7362.49 | 56.5 |
| LP5129 | CUGACGCGAUGCUCAGACACA | SEQ ID NO:471 | 21 | 6694.12 | 57.1 |
| | UGUGUCUGAGCAUCGCGUCAGGU | SEQ ID NO:472 | 23 | 7356.44 | 56.5 |
| LP5132 | ACGCGAUGCUCAGACACAGAA | SEQ ID NO:473 | 21 | 6741.19 | 52.4 |
| | UUCUGUGUCUGAGCAUCGCGUCA | SEQ ID NO:474 | 23 | 7277.37 | 52.2 |
| LP5134 | GCGAUGCUCAGACACAGAAGG | SEQ ID NO:475 | 21 | 6797.22 | 57.1 |
| | CCUUCUGUGUCUGAGCAUCGCGU | SEQ ID NO:476 | 23 | 7253.34 | 56.5 |
| LP5138 | UGCUCAGACACAGAAGGGACU | SEQ ID NO:477 | 21 | 6758.18 | 52.4 |
| | AGUCCCUUCUGUGUCUGAGCAUC | SEQ ID NO:478 | 23 | 7237.34 | 52.2 |
| LP5140 | CUCAGACACAGAAGGGACUGU | SEQ ID NO:479 | 21 | 6758.18 | 52.4 |
| | ACAGUCCCUUCUGUGUCUGAGCA | SEQ ID NO:480 | 23 | 7260.38 | 52.2 |
| LP5142 | CAGACACAGAAGGGACUGUGG | SEQ ID NO:481 | 21 | 6837.25 | 57.1 |
| | CCACAGUCCCUUCUGUGUCUGAG | SEQ ID NO:482 | 23 | 7236.35 | 56.5 |
| LP5143 | AGACACAGAAGGGACUGUGGU | SEQ ID NO:483 | 21 | 6838.24 | 52.4 |
| | ACCACAGUCCCUUCUGUGUCUGA | SEQ ID NO:484 | 23 | 7220.35 | 52.2 |
| LP164 | AGAGUUAUCGAGGCACGUACU | SEQ ID NO:485 | 21 | 6736.13 | 47.6 |
| | AGUACGUGCCUCGAUAACUCUGU | SEQ ID NO:486 | 23 | 7284.41 | 47.8 |
| LP165 | GAGUUAUCGAGGCACGUACUC | SEQ ID NO:487 | 21 | 6712.1 | 52.4 |
| | GAGUACGUGCCUCGAUAACUCUG | SEQ ID NO:488 | 23 | 7323.45 | 52.2 |
| LP166 | AGUUAUCGAGGCACGUACUCC | SEQ ID NO:489 | 21 | 6672.07 | 52.4 |
| | GGAGUACGUGCCUCGAUAACUCU | SEQ ID NO:490 | 23 | 7323.45 | 52.2 |
| LP173 | GAGGCACGUACUCCACCACUG | SEQ ID NO:491 | 21 | 6670.09 | 61.9 |
| | CAGUGGUGGAGUACGUGCCUCGA | SEQ ID NO:492 | 23 | 7418.52 | 60.9 |
| LP228 | GACACCACAUCAACAUAAUAG | SEQ ID NO:493 | 21 | 6670.15 | 38.1 |
| | CUAUUAUGUUGAUGUGGUGUCAU | SEQ ID NO:494 | 23 | 7304.37 | 34.8 |
| LP229 | ACACCACAUCAACAUAAUAGG | SEQ ID NO:495 | 21 | 6670.15 | 38.1 |
| | CCUAUUAUGUUGAUGUGGUGUCA | SEQ ID NO:496 | 23 | 7303.38 | 39.1 |
| LP231 | ACCACAUCAACAUAAUAGGAC | SEQ ID NO:497 | 21 | 6670.15 | 38.1 |
| | GUCCUAUUAUGUUGAUGUGGUGU | SEQ ID NO:498 | 23 | 7320.37 | 39.1 |
| LP307 | GCUGUGGCAGCUCCUUAUUGU | SEQ ID NO:499 | 21 | 6642.98 | 52.4 |
| | ACAAUAAGGAGCUGCCACAGCAU | SEQ ID NO:500 | 23 | 7376.57 | 47.8 |
| LP314 | CAGCUCCUUAUUGUUAUACGA | SEQ ID NO:501 | 21 | 6594.98 | 38.1 |
| | UCGUAUAACAAUAAGGAGCUGCC | SEQ ID NO:502 | 23 | 7354.52 | 43.5 |
| LP315 | AGCUCCUUAUUGUUAUACGAG | SEQ ID NO:503 | 21 | 6635.01 | 38.1 |
| | CUCGUAUAACAAUAAGGAGCUGC | SEQ ID NO:504 | 23 | 7354.52 | 43.5 |
| LP321 | UUAUUGUUAUACGAGGGAUCC | SEQ ID NO:505 | 21 | 6675.04 | 38.1 |
| | GGAUCCCUCGUAUAACAAUAAGG | SEQ ID NO:506 | 23 | 7354.52 | 43.5 |
| LP323 | AUUGUUAUACGAGGGAUCCCG | SEQ ID NO:507 | 21 | 6713.09 | 47.6 |
| | CGGGAUCCCUCGUAUAACAAUAA | SEQ ID NO:508 | 23 | 7314.49 | 43.5 |
| LP324 | UUGUUAUACGAGGGAUCCCGG | SEQ ID NO:509 | 21 | 6729.09 | 52.4 |
| | CCGGGAUCCCUCGUAUAACAAUA | SEQ ID NO:510 | 23 | 7290.46 | 47.8 |
| LP326 | GUUAUACGAGGGAUCCCGGUG | SEQ ID NO:511 | 21 | 6768.13 | 57.1 |
| | CACCGGGAUCCCUCGUAUAACAA | SEQ ID NO:512 | 23 | 7289.47 | 52.2 |
| LP333 | GAGGGAUCCCGGUGUCAGGUG | SEQ ID NO:513 | 21 | 6823.17 | 66.7 |
| | CACCUGACACCGGGAUCCCUCGU | SEQ ID NO:514 | 23 | 7257.41 | 65.2 |
| LP360 | CUGCAACCUGACGCAAUGCUC | SEQ ID NO:515 | 21 | 6631.05 | 57.1 |
| | GAGCAUUGCGUCAGGUUGCAGUA | SEQ ID NO:516 | 23 | 7403.51 | 52.2 |
| LP364 | AACCUGACGCAAUGCUCAGAC | SEQ ID NO:517 | 21 | 6678.12 | 52.4 |
| | GUCUGAGCAUUGCGUCAGGUUGC | SEQ ID NO:518 | 23 | 7356.44 | 56.5 |
| LP388 | GAAGGGACUGCCGUCGCGCCU | SEQ ID NO:519 | 21 | 6742.12 | 71.4 |
| | AGGCGCGACGGCAGUCCCUUCUG | SEQ ID NO:520 | 23 | 7353.47 | 69.6 |
| LP399 | CGUCGCGCCUCCGACUGUUAC | SEQ ID NO:521 | 21 | 6599.98 | 66.7 |
| | GUAACAGUCGGAGGCGCGACGGC | SEQ ID NO:522 | 23 | 7479.61 | 69.6 |
| LP400 | GUCGCGCCUCCGACUGUUACC | SEQ ID NO:523 | 21 | 6599.98 | 66.7 |
| | GGUAACAGUCGGAGGCGCGACGG | SEQ ID NO:524 | 23 | 7519.64 | 69.6 |
| LP401 | UCGCGCCUCCGACUGUUACCC | SEQ ID NO:525 | 21 | 6559.95 | 66.7 |
| | GGGUAACAGUCGGAGGCGCGACG | SEQ ID NO:526 | 23 | 7519.64 | 69.6 |
| LP402 | CGCGCCUCCGACUGUUACCCC | SEQ ID NO:527 | 21 | 6558.96 | 71.4 |
| | GGGGUAACAGUCGGAGGCGCGAC | SEQ ID NO:528 | 23 | 7519.64 | 69.6 |
| LP442 | CCUUCCGAACAAGCACCGACU | SEQ ID NO:529 | 21 | 6614.06 | 57.1 |
| | AGUCGGUGCUUGUUCGGAAGGAG | SEQ ID NO:530 | 23 | 7459.54 | 56.5 |
| LP508 | AGUUAUCGAGGCACAUACUCC | SEQ ID NO:531 | 21 | 6656.07 | 47.6 |
| | GGAGUAUGUGCCUCGAUAACUCU | SEQ ID NO:532 | 23 | 7324.44 | 47.8 |
| LP571 | ACACCACACUCGCAUAGUCGG | SEQ ID NO:533 | 21 | 6654.09 | 57.1 |
| | CCGACUAUGCGAGUGUGGUGUCA | SEQ ID NO:534 | 23 | 7379.48 | 56.5 |
| LP572 | CACCACACUCGCAUAGUCGGA | SEQ ID NO:535 | 21 | 6654.09 | 57.1 |
| | UCCGACUAUGCGAGUGUGGUGUC | SEQ ID NO:536 | 23 | 7356.44 | 56.5 |
| LP573 | ACCACACUCGCAUAGUCGGAC | SEQ ID NO:537 | 21 | 6654.09 | 57.1 |
| | GUCCGACUAUGCGAGUGUGGUGU | SEQ ID NO:538 | 23 | 7396.47 | 56.5 |
| LP574 | CCACACUCGCAUAGUCGGACC | SEQ ID NO:539 | 21 | 6630.06 | 61.9 |
| | GGUCCGACUAUGCGAGUGUGGUG | SEQ ID NO:540 | 23 | 7435.51 | 60.9 |
| LP575 | CACACUCGCAUAGUCGGACCC | SEQ ID NO:541 | 21 | 6630.06 | 61.9 |
| | GGGUCCGACUAUGCGAGUGUGGU | SEQ ID NO:542 | 23 | 7435.51 | 60.9 |
| LP576 | ACACUCGCAUAGUCGGACCCC | SEQ ID NO:543 | 21 | 6630.06 | 61.9 |
| | GGGGUCCGACUAUGCGAGUGUGG | SEQ ID NO:544 | 23 | 7474.55 | 65.2 |
| LP577 | CACUCGCAUAGUCGGACCCCA | SEQ ID NO:545 | 21 | 6630.06 | 61.9 |
| | UGGGGUCCGACUAUGCGAGUGUG | SEQ ID NO:546 | 23 | 7435.51 | 60.9 |
| LP578 | ACUCGCAUAGUCGGACCCCAG | SEQ ID NO:547 | 21 | 6670.09 | 61.9 |
| | CUGGGGUCCGACUAUGCGAGUGU | SEQ ID NO:548 | 23 | 7395.48 | 60.9 |
| LP579 | CUCGCAUAGUCGGACCCCAGA | SEQ ID NO:549 | 21 | 6670.09 | 61.9 |
| | UCUGGGGUCCGACUAUGCGAGUG | SEQ ID NO:550 | 23 | 7395.48 | 60.9 |
| LP580 | UCGCAUAGUCGGACCCCAGAA | SEQ ID NO:551 | 21 | 6694.12 | 57.1 |
| | UUCUGGGGUCCGACUAUGCGAGU | SEQ ID NO:552 | 23 | 7356.44 | 56.5 |
| LP583 | CAUAGUCGGACCCCAGAAUAC | SEQ ID NO:553 | 21 | 6678.12 | 52.4 |
| | GUAUUCUGGGGUCCGACUAUGCG | SEQ ID NO:554 | 23 | 7356.44 | 56.5 |
| LP2709 | AGCCCCUUAUUGUUAUACGAG | SEQ ID NO:555 | 21 | 6634.02 | 42.9 |
| | CUCGUAUAACAAUAAGGGGCUGC | SEQ ID NO:556 | 23 | 7370.52 | 47.8 |
| LP2717 | AUUGUUAUACGAGGGAUCCCA | SEQ ID NO:557 | 21 | 6697.09 | 42.9 |
| | UGGGAUCCCUCGUAUAACAAUAA | SEQ ID NO:558 | 23 | 7315.48 | 39.1 |
| LP2788 | ACUGCCGUCGCGCCUCCAACU | SEQ ID NO:559 | 21 | 6582.99 | 66.7 |
| | AGUUGGAGGCGCGACGGCAGUCC | SEQ ID NO:560 | 23 | 7456.57 | 69.6 |
| LP2789 | CUGCCGUCGCGCCUCCAACUA | SEQ ID NO:561 | 21 | 6582.99 | 66.7 |
| | UAGUUGGAGGCGCGACGGCAGUC | SEQ ID NO:562 | 23 | 7457.56 | 65.2 |
| LP2790 | UGCCGUCGCGCCUCCAACUAU | SEQ ID NO:563 | 21 | 6583.98 | 61.9 |
| | AUAGUUGGAGGCGCGACGGCAGU | SEQ ID NO:564 | 23 | 7481.59 | 60.9 |
| LP2794 | GUCGCGCCUCCAACUAUUACC | SEQ ID NO:565 | 21 | 6567.98 | 57.1 |
| | GGUAAUAGUUGGAGGCGCGACGG | SEQ ID NO:566 | 23 | 7521.62 | 60.9 |
| LP2799 | GCCUCCAACUAUUACCCCGAU | SEQ ID NO:567 | 21 | 6551.98 | 52.4 |
| | AUCGGGGUAAUAGUUGGAGGCGC | SEQ ID NO:568 | 23 | 7482.58 | 56.5 |
| LP2877 | GGAGUGCUACCACGGAAAUGG | SEQ ID NO:569 | 21 | 6814.21 | 57.1 |
| | CCAUUUCCGUGGUAGCACUCCUG | SEQ ID NO:570 | 23 | 7236.35 | 56.5 |
| LP2878 | GAGUGCUACCACGGAAAUGGA | SEQ ID NO:571 | 21 | 6798.21 | 52.4 |
| | UCCAUUUCCGUGGUAGCACUCCU | SEQ ID NO:572 | 23 | 7197.31 | 52.2 |
| LP2879 | AGUGCUACCACGGAAAUGGAC | SEQ ID NO:573 | 21 | 6758.18 | 52.4 |
| | GUCCAUUUCCGUGGUAGCACUCC | SEQ ID NO:574 | 23 | 7236.35 | 56.5 |
| LP2907 | UCAAGGCACAUACUUCAUUAC | SEQ ID NO:575 | 21 | 6601.03 | 38.1 |
| | GUAAUGAAGUAUGUGCCUUGAUA | SEQ ID NO:576 | 23 | 7373.49 | 34.8 |
| LP2911 | GGCACAUACUUCAUUACUGUC | SEQ ID NO:577 | 21 | 6593.99 | 42.9 |
| | GACAGUAAUGAAGUAUGUGCCUU | SEQ ID NO:578 | 23 | 7372.5 | 39.1 |
| LP2922 | CAUUACUGUCACAGGAAGAAC | SEQ ID NO:579 | 21 | 6703.14 | 42.9 |
| | GUUCUUCCUGUGACAGUAAUGAA | SEQ ID NO:580 | 23 | 7309.43 | 39.1 |
| LP3056 | CUUGGUGUUAUACAACAGAUC | SEQ ID NO:581 | 21 | 6658.05 | 38.1 |
| | GAUCUGUUGUAUAACACCAAGGG | SEQ ID NO:582 | 23 | 7371.51 | 43.5 |
| LP3069 | AACAGAUCCCAGUGUCAGGUG | SEQ ID NO:583 | 21 | 6735.14 | 52.4 |
| | CACCUGACACUGGGAUCUGUUGU | SEQ ID NO:584 | 23 | 7300.41 | 52.2 |
| LP3101 | ACCUGACACGAUGCUCAGAUG | SEQ ID NO:585 | 21 | 6695.11 | 52.4 |
| | CAUCUGAGCAUCGUGUCAGGUUG | SEQ ID NO:586 | 23 | 7340.44 | 52.2 |
| LP3121 | GCAGAAUGGACUGCCUUCGUC | SEQ ID NO:587 | 21 | 6688.07 | 57.1 |
| | GACGAAGGCAGUCCAUUCUGCAU | SEQ ID NO:588 | 23 | 7346.49 | 52.2 |
| LP3137 | UCGUCCCUCCGAAUGUUAUUC | SEQ ID NO:589 | 21 | 6546.92 | 47.6 |
| | GAAUAACAUUCGGAGGGACGAAG | SEQ ID NO:590 | 23 | 7496.66 | 47.8 |
| LP3138 | CGUCCCUCCGAAUGUUAUUCU | SEQ ID NO:591 | 21 | 6546.92 | 47.6 |
| | AGAAUAACAUUCGGAGGGACGAA | SEQ ID NO:592 | 23 | 7480.66 | 43.5 |
| LP3139 | GUCCCUCCGAAUGUUAUUCUG | SEQ ID NO:593 | 21 | 6586.95 | 47.6 |
| | CAGAAUAACAUUCGGAGGGACGA | SEQ ID NO:594 | 23 | 7456.63 | 47.8 |
| LP3142 | CCUCCGAAUGUUAUUCUGGCU | SEQ ID NO:595 | 21 | 6586.95 | 47.6 |
| | AGCCAGAAUAACAUUCGGAGGGA | SEQ ID NO:596 | 23 | 7456.63 | 47.8 |
| LP3153 | UAUUCUGGCUCCAAGCCUAGA | SEQ ID NO:597 | 21 | 6633.03 | 47.6 |
| | UCUAGGCUUGGAGCCAGAAUAAC | SEQ ID NO:598 | 23 | 7370.52 | 47.8 |
| LP3229 | UACCAUUAUGGACAGAGUUAC | SEQ ID NO:599 | 21 | 6681.09 | 38.1 |
| | GUAACUCUGUCCAUAAUGGUAGU | SEQ ID NO:600 | 23 | 7309.43 | 39.1 |
| LP3230 | ACCAUUAUGGACAGAGUUACC | SEQ ID NO:601 | 21 | 6680.1 | 42.9 |
| | GGUAACUCUGUCCAUAAUGGUAG | SEQ ID NO:602 | 23 | 7348.47 | 43.5 |
| LP3314 | ACCAGCAUAGUCGGACCCCAG | SEQ ID NO:603 | 21 | 6693.13 | 61.9 |
| | CUGGGGUCCGACUAUGCUGGUGU | SEQ ID NO:604 | 23 | 7372.44 | 60.9 |
| LP3315 | CCAGCAUAGUCGGACCCCAGA | SEQ ID NO:605 | 21 | 6693.13 | 61.9 |
| | UCUGGGGUCCGACUAUGCUGGUG | SEQ ID NO:606 | 23 | 7372.44 | 60.9 |
| LP3475 | GUCCUUGCAACUCUCACGGUG | SEQ ID NO:607 | 21 | 6625 | 57.1 |
| | CACCGUGAGAGUUGCAAGGACAC | SEQ ID NO:608 | 23 | 7408.57 | 56.5 |
| LP3585 | GAGUUAUCGAGGCUCAUUCUC | SEQ ID NO:609 | 21 | 6650.02 | 47.6 |
| | GAGAAUGAGCCUCGAUAACUCUG | SEQ ID NO:610 | 23 | 7370.52 | 47.8 |
| LP3586 | AGUUAUCGAGGCUCAUUCUCU | SEQ ID NO:611 | 21 | 6610.98 | 42.9 |
| | AGAGAAUGAGCCUCGAUAACUCU | SEQ ID NO:612 | 23 | 7354.52 | 43.5 |
| LP3665 | AGAGGACAACAGAAUAUUAUC | SEQ ID NO:613 | 21 | 6751.2 | 33.3 |
| | GAUAAUAUUCUGUUGUCCUCUGA | SEQ ID NO:614 | 23 | 7247.35 | 34.8 |
| LP3725 | AUGCUGAGAUUAGUCCUUGGU | SEQ ID NO:615 | 21 | 6691.04 | 42.9 |
| | ACCAAGGACUAAUCUCAGCAUCU | SEQ ID NO:616 | 23 | 7274.46 | 43.5 |
| LP3731 | AGAUUAGUCCUUGGUGUUAUA | SEQ ID NO:617 | 21 | 6676.03 | 33.3 |
| | UAUAACACCAAGGACUAAUCUCA | SEQ ID NO:618 | 23 | 7282.49 | 34.8 |
| LP3733 | AUUAGUCCUUGGUGUUAUACC | SEQ ID NO:619 | 21 | 6611.97 | 38.1 |
| | GGUAUAACACCAAGGACUAAUCU | SEQ ID NO:620 | 23 | 7338.52 | 39.1 |
| LP3761 | CCAAUGUCAGAUGGGAGUACU | SEQ ID NO:621 | 21 | 6736.13 | 47.6 |
| | AGUACUCCCAUCUGACAUUGGGA | SEQ ID NO:622 | 23 | 7307.45 | 47.8 |
| LP3810 | AUCAAGUGUCCUUGCGACGUC | SEQ ID NO:623 | 21 | 6649.03 | 52.4 |
| | GACGUCGCAAGGACACUUGAUUC | SEQ ID NO:624 | 23 | 7346.49 | 52.2 |
| LP3812 | CAAGUGUCCUUGCGACGUCCA | SEQ ID NO:625 | 21 | 6648.04 | 57.1 |
| | UGGACGUCGCAAGGACACUUGAU | SEQ ID NO:626 | 23 | 7386.52 | 52.2 |
| LP3816 | UGUCCUUGCGACGUCCACGGC | SEQ ID NO:627 | 21 | 6640.01 | 66.7 |
| | GCCGUGGACGUCGCAAGGACACU | SEQ ID NO:628 | 23 | 7400.54 | 65.2 |
| LP3817 | GUCCUUGCGACGUCCACGGCU | SEQ ID NO:629 | 21 | 6640.01 | 66.7 |
| | AGCCGUGGACGUCGCAAGGACAC | SEQ ID NO:630 | 23 | 7423.58 | 65.2 |
| LP3822 | UGCGACGUCCACGGCUGUUUC | SEQ ID NO:631 | 21 | 6641 | 61.9 |
| | GAAACAGCCGUGGACGUCGCAAG | SEQ ID NO:632 | 23 | 7447.61 | 60.9 |
| LP3836 | CUGUUUCUGAACAAGCACCAA | SEQ ID NO:633 | 21 | 6640.07 | 42.9 |
| | UUGGUGCUUGUUCAGAAACAGCC | SEQ ID NO:634 | 23 | 7324.44 | 47.8 |
| LP3870 | CACAGUCCAGGACUGCUACCA | SEQ ID NO:635 | 21 | 6654.09 | 57.1 |
| | UGGUAGCAGUCCUGGACUGUGGG | SEQ ID NO:636 | 23 | 7435.51 | 60.9 |
| LP3904 | AGUUAUCGAGGCUCAUUCUCC | SEQ ID NO:637 | 21 | 6609.99 | 47.6 |
| | GGAGAAUGAGCCUCGAUAACUCU | SEQ ID NO:638 | 23 | 7370.52 | 47.8 |
| LP4049 | AGAUUCGCCCUUGGUGUUAUA | SEQ ID NO:639 | 21 | 6651.01 | 42.9 |
| | UAUAACACCAAGGGCGAAUCUCA | SEQ ID NO:640 | 23 | 7337.53 | 43.5 |
| LP4126 | GAAUCAACUCUCCUCACAACU | SEQ ID NO:641 | 21 | 6560.01 | 42.9 |
| | AGUUGUGAGGAGAGUUGAUUCCA | SEQ ID NO:642 | 23 | 7428.53 | 43.5 |
| LP4134 | UCUCCUCACAACUCCCACGGU | SEQ ID NO:643 | 21 | 6527.95 | 57.1 |
| | ACCGUGGGAGUUGUGAGGAGAGU | SEQ ID NO:644 | 23 | 7522.61 | 56.5 |
| LP4138 | CUCACAACUCCCACGGUGGUC | SEQ ID NO:645 | 21 | 6607.02 | 61.9 |
| | GACCACCGUGGGAGUUGUGAGGA | SEQ ID NO:646 | 23 | 7481.59 | 60.9 |
| LP4145 | CUCCCACGGUGGUCCCAGUUC | SEQ ID NO:647 | 21 | 6599.98 | 66.7 |
| | GAACUGGGACCACCGUGGGAGUU | SEQ ID NO:648 | 23 | 7441.56 | 60.9 |
| LP4211 | CUGGGGUCCAGGACUGCUACC | SEQ ID NO:649 | 21 | 6703.08 | 66.7 |
| | GGUAGCAGUCCUGGACCCCAGUG | SEQ ID NO:650 | 23 | 7377.5 | 65.2 |
| LP4230 | CCGAGGUGAUGGACAGAGUUA | SEQ ID NO:651 | 21 | 6815.2 | 52.4 |
| | UAACUCUGUCCAUCACCUCGGUA | SEQ ID NO:652 | 23 | 7204.35 | 47.8 |
| LP4242 | ACAGAGUUAUCGAGGCACACU | SEQ ID NO:653 | 21 | 6719.14 | 47.6 |
| | AGUGUGCCUCGAUAACUCUGUCC | SEQ ID NO:654 | 23 | 7260.38 | 52.2 |
| LP4243 | CAGAGUUAUCGAGGCACACUC | SEQ ID NO:655 | 21 | 6695.11 | 52.4 |
| | GAGUGUGCCUCGAUAACUCUGUC | SEQ ID NO:656 | 23 | 7300.41 | 52.2 |
| LP4291 | CAGUCUUGGUCGUCUAUGACA | SEQ ID NO:657 | 21 | 6650.02 | 47.6 |
| | UGUCAUAGACGACCAAGACUGAC | SEQ ID NO:658 | 23 | 7353.53 | 47.8 |
| LP4301 | CGUCUAUGACACCACAUUGGC | SEQ ID NO:659 | 21 | 6632.04 | 52.4 |
| | GCCAAUGUGGUGUCAUAGACGAC | SEQ ID NO:660 | 23 | 7386.52 | 52.2 |
| LP4306 | AUGACACCACAUUGGCAUCGG | SEQ ID NO:661 | 21 | 6695.11 | 52.4 |
| | CCGAUGCCAAUGUGGUGUCAUAG | SEQ ID NO:662 | 23 | 7363.48 | 52.2 |
| LP4312 | CCACAUUGGCAUCGGAGGAUC | SEQ ID NO:663 | 21 | 6711.11 | 57.1 |
| | GAUCCUCCGAUGCCAAUGUGGUG | SEQ ID NO:664 | 23 | 7339.45 | 56.5 |
| LP4321 | CAUCGGAGGAUCCCAUUAUAC | SEQ ID NO:665 | 21 | 6656.07 | 47.6 |
| | GUAUAAUGGGAUCCUCCGAUGCC | SEQ ID NO:666 | 23 | 7323.45 | 52.2 |
| LP4322 | AUCGGAGGAUCCCAUUAUACU | SEQ ID NO:667 | 21 | 6657.06 | 42.9 |
| | AGUAUAAUGGGAUCCUCCGAUGC | SEQ ID NO:668 | 23 | 7347.48 | 47.8 |
| LP4325 | GGAGGAUCCCAUUAUACUAUC | SEQ ID NO:669 | 21 | 6657.06 | 42.9 |
| | GAUAGUAUAAUGGGAUCCUCCGA | SEQ ID NO:670 | 23 | 7371.51 | 43.5 |
| LP4326 | GAGGAUCCCAUUAUACUAUCC | SEQ ID NO:671 | 21 | 6617.03 | 42.9 |
| | GGAUAGUAUAAUGGGAUCCUCCG | SEQ ID NO:672 | 23 | 7387.51 | 47.8 |
| LP4438 | UACUGCAACCUGACACGAUGU | SEQ ID NO:673 | 21 | 6656.07 | 47.6 |
| | ACAUCGUGUCAGGUUGCAGUACU | SEQ ID NO:674 | 23 | 7324.44 | 47.8 |
| LP4439 | ACUGCAACCUGACACGAUGUC | SEQ ID NO:675 | 21 | 6655.08 | 52.4 |
| | GACAUCGUGUCAGGUUGCAGUAC | SEQ ID NO:676 | 23 | 7363.48 | 52.2 |
| LP4460 | CAGUGACAGAAUCGAGUGUCC | SEQ ID NO:677 | 21 | 6735.14 | 52.4 |
| | GGACACUCGAUUCUGUCACUGGA | SEQ ID NO:678 | 23 | 7323.45 | 52.2 |
| LP4464 | GACAGAAUCGAGUGUCCUCAC | SEQ ID NO:679 | 21 | 6695.11 | 52.4 |
| | GUGAGGACACUCGAUUCUGUCAC | SEQ ID NO:680 | 23 | 7323.45 | 52.2 |
| LP4584 | ACGGAGUUAUCGAGGCAUAUC | SEQ ID NO:681 | 21 | 6736.13 | 47.6 |
| | GAUAUGCCUCGAUAACUCCGUCC | SEQ ID NO:682 | 23 | 7243.39 | 52.2 |
| LP4585 | CGGAGUUAUCGAGGCAUAUCC | SEQ ID NO:683 | 21 | 6712.1 | 52.4 |
| | GGAUAUGCCUCGAUAACUCCGUC | SEQ ID NO:684 | 23 | 7283.42 | 52.2 |
| LP4586 | GGAGUUAUCGAGGCAUAUCCU | SEQ ID NO:685 | 21 | 6713.09 | 47.6 |
| | AGGAUAUGCCUCGAUAACUCCGU | SEQ ID NO:686 | 23 | 7307.45 | 47.8 |
| LP4587 | GAGUUAUCGAGGCAUAUCCUC | SEQ ID NO:687 | 21 | 6673.06 | 47.6 |
| | GAGGAUAUGCCUCGAUAACUCCG | SEQ ID NO:688 | 23 | 7346.49 | 52.2 |
| LP4599 | CAUAUCCUCCACCACUGUCAC | SEQ ID NO:689 | 21 | 6511.95 | 52.4 |
| | GUGACAGUGGUGGAGGAUAUGCC | SEQ ID NO:690 | 23 | 7482.58 | 56.5 |
| LP4634 | AAUCUUGGUCAUCUAUGAUAC | SEQ ID NO:691 | 21 | 6619.01 | 33.3 |
| | GUAUCAUAGAUGACCAAGAUUGA | SEQ ID NO:692 | 23 | 7379.54 | 34.8 |
| LP4703 | CCGAGAACUACUGCAGGAAUC | SEQ ID NO:693 | 21 | 6718.15 | 52.4 |
| | GAUUCCUGCAGUAGUUCUCGGUC | SEQ ID NO:694 | 23 | 7277.37 | 52.2 |
| LP4741 | CCCUGGUGUUACACAACCGAU | SEQ ID NO:695 | 21 | 6632.04 | 52.4 |
| | AUCGGUUGUGUAACACCAGGGUU | SEQ ID NO:696 | 23 | 7364.47 | 47.8 |
| LP4742 | CCUGGUGUUACACAACCGAUC | SEQ ID NO:697 | 21 | 6632.04 | 52.4 |
| | GAUCGGUUGUGUAACACCAGGGU | SEQ ID NO:698 | 23 | 7403.51 | 52.2 |
| LP4743 | CUGGUGUUACACAACCGAUCC | SEQ ID NO:699 | 21 | 6632.04 | 52.4 |
| | GGAUCGGUUGUGUAACACCAGGG | SEQ ID NO:700 | 23 | 7442.55 | 56.5 |
| LP4762 | CCGUGUGUGAGGUGGGAGUAC | SEQ ID NO:701 | 21 | 6824.16 | 61.9 |
| | GUACUCCCACCUCACACACGGAU | SEQ ID NO:702 | 23 | 7225.41 | 56.5 |
| LP4906 | CAGUGCUACCAUGGUAAUGGC | SEQ ID NO:703 | 21 | 6712.1 | 52.4 |
| | GCCAUUACCAUGGUAGCACUGCC | SEQ ID NO:704 | 23 | 7282.43 | 56.5 |
| LP4907 | AGUGCUACCAUGGUAAUGGCC | SEQ ID NO:705 | 21 | 6712.1 | 52.4 |
| | GGCCAUUACCAUGGUAGCACUGC | SEQ ID NO:706 | 23 | 7322.46 | 56.5 |
| LP4926 | CCAGAGUUAUCGAGGCACAUU | SEQ ID NO:707 | 21 | 6696.1 | 47.6 |
| | AAUGUGCCUCGAUAACUCUGGCC | SEQ ID NO:708 | 23 | 7283.42 | 52.2 |
| LP5067 | AGAUGCCGAUACAGGCCCUUG | SEQ ID NO:711 | 21 | 6711.11 | 57.1 |
| | CAAGGGCCUGUAUCGGCAUCUGG | SEQ ID NO:712 | 23 | 7378.49 | 60.9 |
| LP5107 | AGCAUCAGGUGGGAGUACUGC | SEQ ID NO:713 | 21 | 6791.17 | 57.1 |
| | GCAGUACUCCCACCUGAUGCUGG | SEQ ID NO:714 | 23 | 7298.43 | 60.9 |
| LP5129 | ACCUGACGCGAUGCUCAGACA | SEQ ID NO:715 | 21 | 6694.12 | 57.1 |
| | UGUCUGAGCAUCGCGUCAGGUUG | SEQ ID NO:716 | 23 | 7356.44 | 56.5 |
| LP5160 | UGUGGUCGCUCCUCCGACUGU | SEQ ID NO:717 | 21 | 6617.96 | 61.9 |
| | ACAGUCGGAGGAGCGACCACAGU | SEQ ID NO:718 | 23 | 7447.61 | 60.9 |
| LP5313 | UAGACACAGCACGUUCAUUCC | SEQ ID NO:719 | 21 | 6616.04 | 47.6 |
| | GGAAUGAACGUGCUGUGUCUAUG | SEQ ID NO:720 | 23 | 7404.5 | 47.8 |
| LP5314 | AGACACAGCACGUUCAUUCCA | SEQ ID NO:721 | 21 | 6639.08 | 47.6 |
| | UGGAAUGAACGUGCUGUGUCUAU | SEQ ID NO:722 | 23 | 7365.46 | 43.5 |
| LP5431 | CUUUUUGACUACUGUGAUAUC | SEQ ID NO:723 | 21 | 6572.93 | 33.3 |
| | GAUAUCACAGUAGUCAAAAAGUU | SEQ ID NO:724 | 23 | 7363.54 | 30.4 |
| LP5432 | UUUUUGACUACUGUGAUAUCC | SEQ ID NO:725 | 21 | 6572.93 | 33.3 |
| | GGAUAUCACAGUAGUCAAAAAGU | SEQ ID NO:726 | 23 | 7402.58 | 34.8 |
| LP5433 | UUUUGACUACUGUGAUAUCCC | SEQ ID NO:727 | 21 | 6571.94 | 38.1 |
| | GGGAUAUCACAGUAGUCAAAAAG | SEQ ID NO:728 | 23 | 7441.62 | 39.1 |
| LP5598 | CUGUGGAGGCACCUUAAUAUC | SEQ ID NO:729 | 21 | 6673.06 | 47.6 |
| | GAUAUUAAGGUGCCUCCACAGAA | SEQ ID NO:730 | 23 | 7354.52 | 43.5 |
| LP5599 | UGUGGAGGCACCUUAAUAUCC | SEQ ID NO:731 | 21 | 6673.06 | 47.6 |
| | GGAUAUUAAGGUGCCUCCACAGA | SEQ ID NO:732 | 23 | 7370.52 | 47.8 |
| LP5705 | AAGUGAACCUCGAAUCUCAUG | SEQ ID NO:733 | 21 | 6680.1 | 42.9 |
| | CAUGAGAUUCGAGGUUCACUUCU | SEQ ID NO:734 | 23 | 7285.4 | 43.5 |
| LP5706 | AGUGAACCUCGAAUCUCAUGU | SEQ ID NO:735 | 21 | 6657.06 | 42.9 |
| | ACAUGAGAUUCGAGGUUCACUUC | SEQ ID NO:736 | 23 | 7308.44 | 43.5 |
| LP5761 | CCCACACAAGCAGAUAUUGCC | SEQ ID NO:737 | 21 | 6638.09 | 52.4 |
| | GGCAAUAUCUGCUUGUGUGGGCU | SEQ ID NO:738 | 23 | 7357.43 | 52.2 |
| LP5807 | UCAUCACUGACAAAGUAAUGC | SEQ ID NO:739 | 21 | 6664.1 | 38.1 |
| | GCAUUACUUUGUCAGUGAUGACG | SEQ ID NO:740 | 23 | 7325.43 | 43.5 |
| LP5808 | CAUCACUGACAAAGUAAUGCC | SEQ ID NO:741 | 21 | 6663.11 | 42.9 |
| | GGCAUUACUUUGUCAGUGAUGAC | SEQ ID NO:742 | 23 | 7325.43 | 43.5 |
| LP5933 | AAGCCCAGCUCCUUGUUAUUG | SEQ ID NO:743 | 21 | 6609.99 | 47.6 |
| | CAAUAACAAGGAGCUGGGCUUCC | SEQ ID NO:744 | 23 | 7369.53 | 52.2 |
| LP5936 | CCCAGCUCCUUGUUAUUGAGA | SEQ ID NO:745 | 21 | 6609.99 | 47.6 |
| | UCUCAAUAACAAGGAGCUGGGCU | SEQ ID NO:746 | 23 | 7370.52 | 47.8 |
| LP5951 | UUGAGAAUGAAGUGUGCAAUC | SEQ ID NO:747 | 21 | 6761.15 | 38.1 |
| | GAUUGCACACUUCAUUCUCAAUA | SEQ ID NO:748 | 23 | 7213.37 | 34.8 |
| LP5959 | GAAGUGUGCAAUCACUAUAAG | SEQ ID NO:749 | 21 | 6744.16 | 38.1 |
| | CUUAUAGUGAUUGCACACUUCAU | SEQ ID NO:750 | 23 | 7230.36 | 34.8 |
| LP5960 | AAGUGUGCAAUCACUAUAAGU | SEQ ID NO:751 | 21 | 6705.12 | 33.3 |
| | ACUUAUAGUGAUUGCACACUUCA | SEQ ID NO:752 | 23 | 7253.4 | 34.8 |
| LP5971 | CACUAUAAGUAUAUUUGUGCU | SEQ ID NO:753 | 21 | 6620 | 28.6 |
| | AGCACAAAUAUACUUAUAGUGAU | SEQ ID NO:754 | 23 | 7324.5 | 26.1 |
| LP5978 | AGUAUAUUUGUGCUGAGCAUU | SEQ ID NO:755 | 21 | 6676.03 | 33.3 |
| | AAUGCUCAGCACAAAUAUACUUA | SEQ ID NO:756 | 23 | 7283.48 | 30.4 |
| LP6127 | GUCUAUGCUCGUGUUUCAAGG | SEQ ID NO:757 | 21 | 6667.01 | 47.6 |
| | CCUUGAAACACGAGCAUAGACAC | SEQ ID NO:758 | 23 | 7336.54 | 47.8 |
| LP6154 | ACUUGGAUUGAGGGAAUGAUG | SEQ ID NO:759 | 21 | 6817.18 | 42.9 |
| | CAUCAUUCCCUCAAUCCAAGUAA | SEQ ID NO:760 | 23 | 7195.39 | 39.1 |
| LP6209 | CAUCAACCUACUUAGAAGCUG | SEQ ID NO:761 | 21 | 6640.07 | 42.9 |
| | CAGCUUCUAAGUAGGUUGAUGCU | SEQ ID NO:762 | 23 | 7325.43 | 43.5 |
| LP6215 | CCUACUUAGAAGCUGAAACGU | SEQ ID NO:763 | 21 | 6680.1 | 42.9 |
| | ACGUUUCAGCUUCUAAGUAGGUU | SEQ ID NO:764 | 23 | 7286.39 | 39.1 |
| LP6259 | AAUAAUAGACAGCAAUCAAAC | SEQ ID NO:765 | 21 | 6718.21 | 28.6 |
| | GUUUGAUUGCUGUCUAUUAUUUC | SEQ ID NO:766 | 23 | 7202.26 | 30.4 |
| LP6264 | UAGACAGCAAUCAAACGAAGA | SEQ ID NO:767 | 21 | 6773.25 | 38.1 |
| | UCUUCGUUUGAUUGCUGUCUAUU | SEQ ID NO:768 | 23 | 7178.23 | 34.8 |
| LP6332 | UUUUGUGUAUAAGCUUUUAAG | SEQ ID NO:769 | 21 | 6637.98 | 23.8 |
| | CUUAAAAGCUUAUACACAAAAAU | SEQ ID NO:770 | 23 | 7291.51 | 21.7 |
| LP6334 | UUGUGUAUAAGCUUUUAAGGU | SEQ ID NO:771 | 21 | 6677.02 | 28.6 |
| | ACCUUAAAAGCUUAUACACAAAA | SEQ ID NO:772 | 23 | 7290.52 | 26.1 |
| LP6335 | UGUGUAUAAGCUUUUAAGGUC | SEQ ID NO:773 | 21 | 6676.03 | 33.3 |
| | GACCUUAAAAGCUUAUACACAAA | SEQ ID NO:774 | 23 | 7306.52 | 30.4 |
| LP6349 | UAAGGUCUGACUGACAAAUUC | SEQ ID NO:775 | 21 | 6681.09 | 38.1 |
| | GAAUUUGUCAGUCAGACCUUAAA | SEQ ID NO:776 | 23 | 7316.47 | 34.8 |
| LP6359 | CUGACAAAUUCUGUAUUAAGG | SEQ ID NO:777 | 21 | 6682.08 | 33.3 |
| | CCUUAAUACAGAAUUUGUCAGUC | SEQ ID NO:778 | 23 | 7253.4 | 34.8 |
| LP6360 | UGACAAAUUCUGUAUUAAGGU | SEQ ID NO:779 | 21 | 6683.07 | 28.6 |
| | ACCUUAAUACAGAAUUUGUCAGU | SEQ ID NO:780 | 23 | 7277.43 | 30.4 |
| LP6389 | UAUGACAUUUGUUAAAAAUAA | SEQ ID NO:781 | 21 | 6675.1 | 14.3 |
| | UUAUUUUUAACAAAUGUCAUAGC | SEQ ID NO:782 | 23 | 7239.38 | 21.7 |
| LP6406 | AUAAACUCUGCACUUAUUUUG | SEQ ID NO:783 | 21 | 6579.97 | 28.6 |
| | CAAAAUAAGUGCAGAGUUUAUUU | SEQ ID NO:784 | 23 | 7341.49 | 26.1 |
| OLP2706 | CAGCCCCUUAUUGUUAUACG | SEQ ID NO:785 | 20 | 6,264.80 | 45.0 |
| | UCGUAUAACAAUAAGGGGCU | SEQ ID NO:786 | 20 | 6,414.90 | 40.0 |
| SLN2545 | CGGUAAUGGACAGAGUUAU | SEQ ID NO:787 | 19 | 6125.7 | 42.1 |
| | AUAACUCUGUCCAUUACCG | SEQ ID NO:788 | 19 | 5,942.60 | 42.1 |

### II. siRNA synthesis (natural RNA/2'-methoxy or 2'-fluoro-modified RNA/GalNAc-RNA)

In this application, dsRNAs which comprise ribonucleotides only or which comprise 2'-methoxy or 2'-fluoro modified oligonucleotides were synthesized according to a theoretical yield of 1 µmol. All oligonucleotides (including the unmodified dsRNAs listed in Table 2 and the modified dsRNAs listed in Table 8) were prepared on an LK-192X synthesizer using a 1 µmol universal Frit carrier (1000Å = 100nm, Biocomma) or a CPG carrier with a GalNAc derivative L96 having a protective group (WuXi AppTec/Glen Research, loading capacity 30 µmol/g). All phosphoramidite monomers (Hongene biotech/Hitgene) were diluted 1:20 (g/mL) in anhydrous acetonitrile solvent. Coupling was performed for 3 minutes twice. Deprotection was performed using 3% TCA, activation was performed using 0.3M benzylthiotetrazolyl acetonitrile solution, and capping and oxidation were performed using CAPA/CAPB and 50 mM I₂ solution respectively. After trityl-off synthesis, the solid support was transferred to a 2 mL centrifuge tube, and 1.2 mL of ammonia was added and the tube was heated in a 65°C oven for 3 hours to remove the protecting groups. The product was then cooled to room temperature and concentrated under vacuum for 30 minutes. The solution was then filtered through a 0.22 µm filter into a vial and single-stranded product was purified using a semi-preparative reverse-phase purification system with an elution gradient of 7% to 30% (ACN:100 mM TEAA) over 10 minutes at a flow rate of 5 mL/min. After preparation and purification, the sample was concentrated under vacuum and spin-dried at room temperature. Finally, the sample was dissolved with water, and each solution was desalted on a GE Hi-Trap desalting column to elute the final oligonucleotide product. All properties and purity were confirmed using ESI-MS and IEX HPLC, respectively. Concentration was determined using a microplate reader under UV light. Equimolar amounts of sense and antisense strands were mixed and transferred to a new 2 mL EP tube. The product was heated at 95 °C for 5 minutes, slowly annealed to room temperature, and then spin-dried using a vacuum concentrator at room temperature to obtain the final product.

### III. Detection of the inhibitory activity of LPA-siRNA in thepsicheck-2 system in vitro

### 1. Construction of detection plasmid

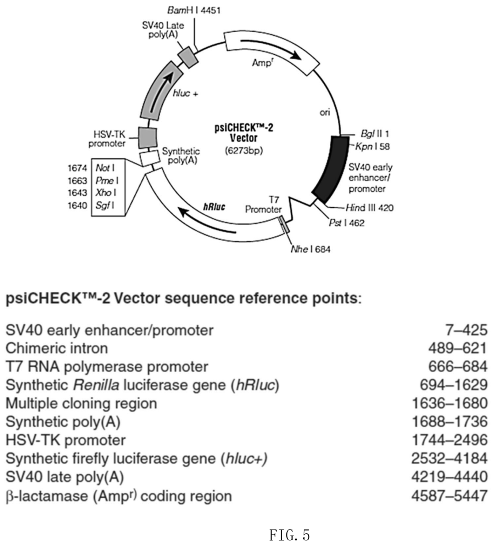

The LPA recombinant plasmid was constructed using psicheck-2 plasmid (GenScript Biotech Co., Ltd.). The psicheck-2 plasmid was purchased from Promega, and the plasmid map thereof is shown in Figure 5. It contains the target sequences (i.e., the target sequence, which is complementary to the antisense strand of any of the siRNAs to be tested) of all LPA siRNAs to be tested, and the cloning sites are the 5'XhoI and 3'NotI sites of the psicheck-2 plasmid.

### 2. Co-transfection of LPA siRNA and the recombinant plasmid

293T cells and transfection reagents were purchased from commercial sources. The cells were cultured in a DMEM medium containing 10% fetal bovine serum in a 5% CO₂, 37°C constant temperature incubator, and were plated for transfection when they were in the logarithmic growth phase and in good condition (70% confluence). The cell density was adjusted, and 5×10⁴ cells per well were plated in a 96-well plate. The transfection complex was prepared as follows: 5µL Opti-MEM, 8ng recombinant plasmid and 1µL of 10uM siRNA were mixed, and 5µL Opti-MEM and 1µL Lipofectamine2000 transfection reagent were mixed, and the mixtures were allowed to stand for 5min. Then, the two mixtures were mixed and allowed to stand for 5min. The above transfection complex was added to a 96-well plate and incubated in a 5% CO₂, 37°C constant temperature incubator for 24h.

In addition to the experimental group, the following control groups were set up for each cell transfection: NC was a negative control (irrelevant siRNA), Lipo group was a transfection reagent control group, and Blank group was an untreated control group (no siRNA added). Both the experimental group and the control group were repeated 3 times.

### 3. DLR detection and analysis

The Dual-Luciferase Reporter Assay System kit (Promega) was used for detection. The cells were lysed and collected according to the instructions of the kit. The fluorescence intensity of Photinus pyralis luciferase and Renilla reniformis luciferase was detected in turn using the InfiniteEplex microplate reader (TECAN). The ratio of the fluorescence intensity of Renilla reniformis luciferase to that of Photinus pyralis luciferase was calculated, and the NC group was used as the control for normalization. Tables 4 and 5 show the results of three replicates of DLR detection, showing the mean value of the dual luciferase reporter gene expression level of the LPA siRNA experimental group relative to the NC group.

**Table 4 results of DLR detection**

| | 100 nM | | | |
|---|---|---|---|---|
| Primer Name | DLR result 1 | DLR result 2 | DLR result 3 | Mean value of DLR |
| LP1787 | 0.179 | 0.161 | 0.44 | 0.26 |
| LP1790 | 0.175 | 0.228 | 0.365 | 0.256 |
| LP1795 | 0.072 | 0.083 | 0.13 | 0.095 |
| LP1798 | 0.063 | 0.119 | 0.121 | 0.101 |
| LP1799 | 0.098 | 0.144 | 0.129 | 0.124 |
| LP1802 | 0.113 | 0.176 | 0.146 | 0.145 |
| LP1806 | 0.158 | 0.264 | 0.351 | 0.258 |
| LP1810 | 0.234 | 0.241 | 0.398 | 0.291 |
| LP1813 | 0.077 | 0.088 | 0.221 | 0.128 |
| LP1816 | 0.145 | 0.124 | 0.28 | 0.183 |
| LP1817 | 0.277 | 0.198 | 0.262 | 0.246 |
| LP1820 | 0.184 | 0.156 | 0.222 | 0.187 |
| LP1825 | 0.363 | 0.344 | 0.475 | 0.394 |
| LP1833 | 0.307 | 0.271 | 0.431 | 0.336 |
| LP1836 | 0.276 | 0.284 | 0.405 | 0.322 |
| LP1839 | 0.216 | 0.212 | 0.311 | 0.246 |
| LP 1840 | 0.147 | 0.15 | 0.374 | 0.223 |
| LP2249 | 0.17 | 0.342 | 0.309 | 0.274 |
| LP2250 | 0.141 | 0.242 | 0.233 | 0.206 |
| LP2253 | 0.129 | 0.213 | 0.189 | 0.177 |
| LP2257 | 0.108 | 0.154 | 0.149 | 0.137 |
| LP2261 | 0.105 | 0.176 | 0.19 | 0.157 |
| LP2264 | 0.052 | 0.088 | 0.097 | 0.079 |
| LP2482 | 0.216 | 0.278 | 0.269 | 0.254 |
| LP2523 | 0.307 | 0.118 | 0.745 | 0.39 |
| LP2526 | 0.184 | 0.153 | 0.27 | 0.203 |
| LP2528 | 0.181 | 0.14 | 0.318 | 0.213 |
| LP2531 | 0.155 | 0.329 | 0.352 | 0.278 |
| LP2540 | 0.285 | 0.193 | 0.569 | 0.349 |
| LP2542 | 0.218 | 0.192 | 0.291 | 0.234 |
| LP2545 | 0.213 | 0.226 | 0.323 | 0.254 |
| LP2546 | 0.139 | 0.346 | 0.215 | 0.233 |
| LP2552 | 0.09 | 0.164 | 0.197 | 0.151 |
| LP2553 | 0.149 | 0.1 | 0.217 | 0.156 |
| LP2556 | 0.159 | 0.101 | 0.278 | 0.18 |
| LP2681 | 0.317 | 0.081 | 0.385 | 0.261 |
| LP2683 | 0.15 | 0.123 | 0.178 | 0.15 |
| LP2687 | 0.174 | 0.057 | 0.247 | 0.159 |
| LP2689 | 0.232 | 0.083 | 0.266 | 0.194 |
| LP2693 | 0.572 | 0.028 | 0.604 | 0.401 |
| LP2696 | 0.146 | 0.16 | 0.288 | 0.198 |
| LP2702 | 0.148 | 0.14 | 0.275 | 0.187 |
| LP2704 | 0.148 | 0.218 | 0.308 | 0.225 |
| LP2737 | 0.188 | 0.221 | 0.398 | 0.269 |
| LP2739 | 0.168 | 0.168 | 0.34 | 0.225 |
| LP2745 | 0.22 | 0.236 | 0.363 | 0.273 |
| LP2748 | 0.31 | 0.22 | 0.446 | 0.326 |
| LP2752 | 0.189 | 0.14 | 0.153 | 0.161 |
| LP2756 | 0.147 | 0.1 | 0.216 | 0.154 |
| LP2759 | 0.132 | 0.075 | 0.183 | 0.13 |
| LP2765 | 0.293 | 0.214 | 0.333 | 0.28 |
| LP2767 | 0.129 | 0.143 | 0.247 | 0.173 |
| LP2770 | 0.175 | 0.181 | 0.241 | 0.199 |
| LP2933 | 0.103 | 0.139 | 0.143 | 0.128 |
| LP2941 | 0.089 | 0.113 | 0.134 | 0.112 |
| LP2945 | 0.11 | 0.126 | 0.146 | 0.127 |
| LP2952 | 0.056 | 0.034 | 0.095 | 0.062 |
| LP3020 | 0.16 | 0.145 | 0.25 | 0.185 |
| LP3022 | 0.187 | 0.15 | 0.283 | 0.207 |
| LP3023 | 0.286 | 0.218 | 0.384 | 0.296 |
| LP3025 | 0.276 | 0.213 | 0.327 | 0.272 |
| LP3029 | 0.485 | 0.421 | 0.59 | 0.499 |
| LP3039 | 0.342 | 0.44 | 0.701 | 0.494 |
| LP3042 | 0.37 | 0.351 | 0.494 | 0.405 |
| LP3045 | 0.345 | 0.659 | 0.45 | 0.484 |
| LP3046 | 0.379 | 0.362 | 0.495 | 0.412 |
| LP3047 | 0.291 | 0.245 | 0.389 | 0.308 |
| LP3073 | 0.158 | 0.194 | 0.285 | 0.212 |
| LP3074 | 0.21 | 0.167 | 0.266 | 0.214 |
| LP3075 | 0.273 | 0.271 | 0.348 | 0.298 |
| LP3079 | 0.224 | 0.235 | 0.245 | 0.235 |
| LP3084 | 0.128 | 0.134 | 0.248 | 0.17 |
| LP3090 | 0.079 | 0.103 | 0.254 | 0.145 |
| LP3097 | 0.22 | 0.18 | 0.386 | 0.262 |
| LP3098 | 0.186 | 0.205 | 0.416 | 0.269 |
| LP3100 | 0.316 | 0.278 | 0.525 | 0.373 |
| LP3104 | 0.214 | 0.206 | 0.298 | 0.239 |
| LP3275 | 0.114 | 0.113 | 0.147 | 0.125 |
| LP3276 | 0.135 | 0.106 | 0.151 | 0.13 |
| LP3279 | 0.099 | 0.116 | 0.151 | 0.122 |
| LP3283 | 0.112 | 0.125 | 0.209 | 0.148 |
| LP3287 | 0.109 | 0.116 | 0.191 | 0.139 |
| LP3294 | 0.067 | 0.063 | 0.108 | 0.08 |
| LP3299 | 0.317 | 0.307 | 0.447 | 0.357 |
| LP3302 | 0.273 | 0.217 | 0.305 | 0.265 |
| LP3306 | 0.143 | 0.14 | 0.233 | 0.172 |
| LP3311 | 0.154 | 0.152 | 0.247 | 0.184 |
| LP3343 | 0.284 | 0.436 | 0.806 | 0.509 |
| LP3347 | 0.117 | 0.11 | 0.29 | 0.172 |
| LP3348 | 0.121 | 0.104 | 0.251 | 0.159 |
| LP3350 | 0.152 | 0.129 | 0.245 | 0.175 |
| LP3353 | 0.269 | 0.211 | 0.371 | 0.284 |
| LP3355 | 0.276 | 0.231 | 0.365 | 0.29 |
| LP3357 | 0.374 | 0.274 | 0.497 | 0.382 |
| LP3362 | 0.193 | 0.153 | 0.278 | 0.208 |
| LP3365 | 0.391 | 0.302 | 0.332 | 0.342 |
| LP3367 | 0.097 | 0.092 | 0.182 | 0.124 |
| LP3370 | 0.153 | 0.152 | 0.307 | 0.204 |
| LP3372 | 0.163 | 0.204 | 0.227 | 0.198 |
| LP3373 | 0.178 | 0.215 | 0.258 | 0.217 |
| LP3378 | 0.117 | 0.137 | 0.147 | 0.133 |
| LP3380 | 0.113 | 0.145 | 0.194 | 0.151 |
| LP3385 | 0.133 | 0.341 | 0.268 | 0.247 |
| LP3414 | 0.344 | 0.753 | 0.915 | 0.67 |
| LP3416 | 0.184 | 0.169 | 0.268 | 0.207 |
| LP3426 | 0.122 | 0.131 | 0.188 | 0.147 |
| LP3568 | 0.825 | 0.774 | 0.952 | 0.85 |
| LP3572 | 0.902 | 0.697 | 1.006 | 0.868 |
| LP3576 | 0.308 | 0.326 | 0.335 | 0.323 |
| LP3578 | 0.387 | 0.484 | 0.693 | 0.521 |
| LP3579 | 0.492 | 0.453 | 0.87 | 0.605 |
| LP3582 | 0.814 | 1.154 | 0.904 | 0.957 |
| LP3607 | 0.337 | 0.417 | 0.657 | 0.47 |
| LP3608 | 0.584 | 0.589 | 0.846 | 0.673 |
| LP3612 | 0.959 | 1.161 | 1.045 | 1.055 |
| LP3617 | 0.967 | 1.099 | 1.027 | 1.031 |
| LP3618 | 0.947 | 1.171 | 1.092 | 1.07 |
| LP3623 | 0.932 | 1.174 | 1.016 | 1.041 |
| LP3627 | 0.968 | 1.189 | 1.058 | 1.072 |
| LP3629 | 0.299 | 0.471 | 0.727 | 0.499 |
| LP3632 | 0.438 | 0.214 | 0.767 | 0.473 |
| LP3636 | 0.926 | 0.858 | 0.92 | 0.901 |
| LP3641 | 0.342 | 0.369 | 0.581 | 0.431 |
| LP3645 | 0.93 | 1.058 | 1.054 | 1.014 |
| LP3651 | 0.907 | 1.183 | 0.621 | 0.904 |
| LP3657 | 0.907 | 1.046 | 1.05 | 1.001 |
| LP3659 | 0.729 | 1.157 | 1.091 | 0.992 |
| LP3662 | 0.93 | 1.18 | 1.062 | 1.057 |
| LP3662 | 0.973 | 1.041 | 1.024 | 1.013 |
| LP3664 | 0.968 | 1.059 | 1.012 | 1.013 |
| LP3689 | 0.005 | 0.185 | 0.234 | 0.141 |
| LP3690 | 0.185 | 0.166 | 0.204 | 0.185 |
| LP3692 | 0.2 | 0.165 | 0.205 | 0.19 |
| LP3695 | 0.256 | 0.261 | 0.306 | 0.274 |
| LP3699 | 0.326 | 0.305 | 0.37 | 0.333 |
| LP3709 | 0.122 | 0.115 | 0.152 | 0.129 |
| LP3776 | 0.237 | 0.169 | 0.232 | 0.212 |
| LP3781 | 0.968 | 0.449 | 0.738 | 0.719 |
| LP3785 | 0.741 | 0.565 | 0.777 | 0.694 |
| LP3787 | 0.964 | 1.005 | 0.983 | 0.984 |
| LP3789 | 0.935 | 0.744 | 1.03 | 0.903 |
| LP3794 | 0.974 | 1.133 | 0.976 | 1.028 |
| LP3798 | 0.935 | 1.088 | 1.144 | 1.055 |
| LP3799 | 0.949 | 0.72 | 0.847 | 0.839 |
| LP3875 | 0.886 | 0.935 | 1.032 | 0.951 |
| LP3880 | 0.958 | 0.826 | 1.1 | 0.961 |
| LP3886 | 0.935 | 0.909 | 1.125 | 0.99 |
| LP3935 | 0.931 | 1.101 | 0.816 | 0.949 |
| LP3947 | 0.39 | 0.305 | 0.449 | 0.382 |
| LP3954 | 0.219 | 0.193 | 0.21 | 0.207 |
| LP4027 | 0.099 | 0.122 | 0.144 | 0.122 |
| LP4032 | 0.173 | 0.236 | 0.256 | 0.222 |
| LP4033 | 0.902 | 0.812 | 1.023 | 0.912 |
| LP4040 | 0.916 | 1.028 | 1.003 | 0.982 |
| LP4046 | 0.857 | 1.035 | 1.029 | 0.973 |
| LP4048 | 0.895 | 0.791 | 0.924 | 0.87 |
| LP4051 | 0.832 | 0.895 | 0.886 | 0.871 |
| LP4052 | 0.92 | 0.8 | 0.84 | 0.853 |
| LP4055 | 0.842 | 0.958 | 1.05 | 0.95 |
| LP4056 | 0.575 | 0.906 | 1.027 | 0.836 |
| LP4059 | 0.162 | 0.369 | 0.203 | 0.245 |
| LP4382 | 0.323 | 0.475 | 0.35 | 0.383 |
| LP4389 | 0.208 | 0.442 | 0.22 | 0.29 |
| LP4393 | 0.342 | 0.613 | 0.354 | 0.437 |
| LP4394 | 0.292 | 0.485 | 0.252 | 0.343 |
| LP4397 | 0.331 | 0.538 | 0.463 | 0.444 |
| LP4398 | 0.237 | 0.536 | 0.376 | 0.383 |
| LP4403 | 0.274 | 0.709 | 0.548 | 0.51 |
| LP4407 | 0.38 | 0.439 | 0.397 | 0.405 |
| LP4410 | 0.53 | 0.369 | 0.482 | 0.46 |
| LP4418 | 0.25 | 0.212 | 0.25 | 0.238 |
| LP4428 | 0.183 | 0.136 | 0.224 | 0.181 |
| LP4503 | 0.742 | 0.721 | 0.771 | 0.745 |
| LP4508 | 0.323 | 0.294 | 0.346 | 0.321 |
| LP4510 | 0.271 | 0.289 | 0.342 | 0.301 |
| LP4514 | 0.211 | 0.305 | 0.501 | 0.339 |
| LP4516 | 0.274 | 0.274 | 0.379 | 0.309 |
| LP4520 | 0.501 | 0.443 | 0.438 | 0.461 |
| LP4525 | 0.31 | 0.322 | 0.402 | 0.345 |
| LP4532 | 0.485 | 0.579 | 0.718 | 0.594 |
| LP4536 | 0.454 | 0.487 | 0.529 | 0.49 |
| LP4537 | 0.528 | 0.605 | 0.853 | 0.662 |
| LP4544 | 0.415 | 0.437 | 0.735 | 0.529 |
| LP4549 | 0.222 | 0.264 | 0.519 | 0.335 |
| LP4551 | 0.287 | 0.285 | 0.283 | 0.285 |
| LP4553 | 0.153 | 0.121 | 0.153 | 0.143 |
| LP4555 | 0.209 | 0.257 | 0.263 | 0.243 |
| LP4559 | 0.312 | 0.288 | 0.242 | 0.28 |
| LP4638 | 0.2 | 0.245 | 0.209 | 0.218 |
| LP4643 | 0.348 | 0.335 | 0.371 | 0.351 |
| LP4650 | 0.362 | 0.402 | 0.409 | 0.391 |
| LP4658 | 0.171 | 0.196 | 0.34 | 0.236 |
| LP4661 | 0.291 | 0.25 | 0.24 | 0.26 |
| LP4662 | 0.444 | 0.419 | 0.276 | 0.379 |
| LP4669 | 0.212 | 0.127 | 0.22 | 0.186 |
| LP4670 | 0.203 | 0.133 | 0.183 | 0.173 |
| LP4671 | 0.201 | 0.142 | 0.132 | 0.158 |
| LP4678 | 0.755 | 0.361 | 0.513 | 0.543 |
| LP4707 | 0.174 | 0.124 | 0.181 | 0.16 |
| LP4709 | 0.374 | 0.255 | 0.444 | 0.358 |
| LP4711 | 0.269 | 0.272 | 0.199 | 0.247 |
| LP4714 | 0.316 | 0.485 | 0.311 | 0.371 |
| LP4716 | 0.573 | 0.852 | 0.4 | 0.608 |
| LP4717 | 0.539 | 0.866 | 0.524 | 0.643 |
| LP4722 | 0.483 | 0.706 | 0.42 | 0.536 |
| LP4724 | 0.559 | 0.668 | 0.497 | 0.575 |
| LP4727 | 0.442 | 0.561 | 0.409 | 0.471 |
| LP4790 | 0.411 | 0.527 | 0.384 | 0.441 |
| LP4796 | 0.307 | 0.323 | 0.237 | 0.289 |
| LP4798 | 0.473 | 0.54 | 0.463 | 0.492 |
| LP4800 | 0.47 | 0.561 | 0.313 | 0.448 |
| LP4801 | 0.409 | 0.531 | 0.359 | 0.433 |
| LP4808 | 0.559 | 0.65 | 0.392 | 0.533 |
| LP4814 | 0.549 | 0.587 | 0.428 | 0.521 |
| LP4819 | 0.347 | 0.406 | 0.295 | 0.349 |
| LP4934 | 0.267 | 0.167 | 0.19 | 0.208 |
| LP4944 | 0.293 | 0.231 | 0.246 | 0.257 |
| LP4945 | 0.265 | 0.221 | 0.177 | 0.221 |
| LP4950 | 0.482 | 0.428 | 0.353 | 0.421 |
| LP4951 | 0.354 | 0.266 | 0.207 | 0.275 |
| LP4961 | 0.356 | 0.21 | 0.185 | 0.25 |
| LP4962 | 0.396 | 0.237 | 0.217 | 0.283 |
| LP4966 | 0.515 | 0.355 | 0.446 | 0.439 |
| LP4969 | 0.455 | 0.353 | 0.289 | 0.365 |
| LP4973 | 0.55 | 0.447 | 0.412 | 0.47 |
| LP4978 | 0.431 | 0.474 | 0.446 | 0.45 |
| LP4980 | 0.428 | 0.568 | 0.505 | 0.5 |
| LP5118 | 0.203 | 0.207 | 0.176 | 0.195 |
| LP5121 | 0.248 | 0.194 | 0.22 | 0.221 |
| LP5129 | 0.245 | 0.188 | 0.223 | 0.218 |
| LP5132 | 0.24 | 0.204 | 0.279 | 0.241 |
| LP5134 | 0.213 | 0.124 | 0.178 | 0.172 |
| LP5138 | 0.93 | 0.207 | 0.302 | 0.48 |
| LP5140 | 0.972 | 0.33 | 0.449 | 0.584 |
| LP5142 | 0.6 | 0.679 | 0.464 | 0.581 |
| LP5143 | 1.012 | 0.927 | 0.655 | 0.865 |
| OLP2706 | 0.153 | 0.12 | 0.171 | 0.148 |
| SLN2545 | 0.37 | 0.389 | 0.275 | 0.344 |

**Table 5 results of DLR detection**

| | 33nM | | | |
|---|---|---|---|---|
| Primer Name | DLR result 1 | DLR result 2 | DLR result 3 | Mean value of DLR |
| LP164 | 0.04 | 0.05 | 0.05 | 0.04 |
| LP165 | 0.04 | 0.05 | 0.04 | 0.04 |
| LP166 | 0.03 | 0.04 | 0.04 | 0.04 |
| LP173 | 0.06 | 0.06 | 0.06 | 0.06 |
| LP228 | 0.13 | 0.16 | 0.15 | 0.15 |
| LP229 | 0.09 | 0.10 | 0.11 | 0.10 |
| LP231 | 0.19 | 0.20 | 0.22 | 0.20 |
| LP307 | 0.07 | 0.09 | 0.10 | 0.09 |
| LP314 | 0.10 | 0.09 | 0.09 | 0.09 |
| LP315 | 0.07 | 0.09 | 0.07 | 0.08 |
| LP321 | 0.12 | 0.12 | 0.12 | 0.12 |
| LP323 | 0.25 | 0.26 | 0.26 | 0.26 |
| LP324 | 0.20 | 0.24 | 0.25 | 0.23 |
| LP326 | 0.08 | 0.09 | 0.11 | 0.09 |
| LP333 | 0.27 | 0.27 | 0.36 | 0.30 |
| LP360 | 0.10 | 0.11 | 0.13 | 0.12 |
| LP364 | 0.18 | 0.16 | 0.18 | 0.18 |
| LP388 | 0.26 | 0.37 | 0.36 | 0.33 |
| LP399 | 0.13 | 0.15 | 0.15 | 0.14 |
| LP400 | 0.11 | 0.15 | 0.18 | 0.15 |
| LP401 | 0.15 | 0.23 | 0.28 | 0.22 |
| LP402 | 0.14 | 0.13 | 0.22 | 0.16 |
| LP442 | 0.07 | 0.09 | 0.12 | 0.09 |
| LP508 | 0.04 | 0.04 | 0.04 | 0.04 |
| LP571 | 0.10 | 0.14 | 0.13 | 0.12 |
| LP572 | 0.12 | 0.14 | 0.11 | 0.12 |
| LP573 | 0.10 | 0.13 | 0.09 | 0.11 |
| LP574 | 0.11 | 0.13 | 0.10 | 0.11 |
| LP575 | 0.11 | 0.10 | 0.13 | 0.11 |
| LP576 | 0.20 | 0.21 | 0.28 | 0.23 |
| LP577 | 0.07 | 0.05 | 0.09 | 0.07 |
| LP578 | 0.09 | 0.10 | 0.11 | 0.10 |
| LP579 | 0.10 | 0.10 | 0.10 | 0.10 |
| LP580 | 0.11 | 0.16 | 0.14 | 0.14 |
| LP583 | 0.11 | 0.09 | 0.10 | 0.10 |
| LP2709 | 0.08 | 0.07 | 0.09 | 0.08 |
| LP2717 | 0.07 | 0.05 | 0.07 | 0.06 |
| LP2788 | 0.09 | 0.07 | 0.16 | 0.11 |
| LP2789 | 0.09 | 0.06 | 0.12 | 0.09 |
| LP2790 | 0.08 | 0.06 | 0.08 | 0.07 |
| LP2794 | 0.07 | 0.09 | 0.10 | 0.09 |
| LP2799 | 0.08 | 0.10 | 0.11 | 0.10 |
| LP2877 | 0.07 | 0.09 | 0.10 | 0.09 |
| LP2878 | 0.07 | 0.06 | 0.09 | 0.08 |
| LP2879 | 0.07 | 0.08 | 0.13 | 0.10 |
| LP2907 | 0.07 | 0.08 | 0.11 | 0.09 |
| LP2911 | 0.07 | 0.05 | 0.09 | 0.07 |
| LP2922 | 0.11 | 0.11 | 0.11 | 0.11 |
| LP3056 | 0.06 | 0.07 | 0.08 | 0.07 |
| LP3069 | 0.16 | 0.17 | 0.17 | 0.17 |
| LP3101 | 0.09 | 0.15 | 0.13 | 0.12 |
| LP3121 | 0.06 | 0.08 | 0.09 | 0.08 |
| LP3137 | 0.11 | 0.12 | 0.15 | 0.13 |
| LP3138 | 0.10 | 0.10 | 0.17 | 0.13 |
| LP3139 | 0.09 | 0.10 | 0.12 | 0.10 |
| LP3142 | 0.07 | 0.09 | 0.09 | 0.08 |
| LP3153 | 0.11 | 0.13 | 0.18 | 0.14 |
| LP3229 | 0.12 | 0.13 | 0.17 | 0.14 |
| LP3230 | 0.08 | 0.12 | 0.13 | 0.11 |
| LP3314 | 0.10 | 0.10 | 0.13 | 0.11 |
| LP3315 | 0.07 | 0.06 | 0.12 | 0.08 |
| LP3475 | 0.13 | 0.14 | 0.18 | 0.15 |
| LP3585 | 0.05 | 0.06 | 0.08 | 0.06 |
| LP3586 | 0.07 | 0.07 | 0.12 | 0.09 |
| LP3665 | 0.09 | 0.11 | 0.15 | 0.12 |
| LP3725 | 0.15 | 0.16 | 0.19 | 0.16 |
| LP3731 | 0.11 | 0.10 | 0.12 | 0.11 |
| LP3733 | 0.19 | 0.16 | 0.17 | 0.17 |
| LP3761 | 0.08 | 0.09 | 0.08 | 0.09 |
| LP3810 | 0.08 | 0.07 | 0.07 | 0.07 |
| LP3812 | 0.07 | 0.08 | 0.09 | 0.08 |
| LP3816 | 0.24 | 0.24 | 0.27 | 0.25 |
| LP3817 | 0.24 | 0.21 | 0.23 | 0.23 |
| LP3822 | 0.24 | 0.16 | 0.10 | 0.17 |
| LP3836 | 0.04 | 0.04 | 0.04 | 0.04 |
| LP3870 | 0.08 | 0.08 | 0.08 | 0.08 |
| LP3904 | 0.10 | 0.09 | 0.08 | 0.09 |
| LP4049 | 0.10 | 0.09 | 0.10 | 0.10 |
| LP4126 | 0.09 | 0.07 | 0.08 | 0.08 |
| LP4134 | 0.23 | 0.19 | 0.20 | 0.21 |
| LP4138 | 0.18 | 0.16 | 0.15 | 0.16 |
| LP4145 | 0.21 | 0.20 | 0.19 | 0.20 |
| LP4211 | 0.13 | 0.08 | 0.12 | 0.11 |
| LP4230 | 0.06 | 0.05 | 0.07 | 0.06 |
| LP4242 | 0.08 | 0.07 | 0.07 | 0.07 |
| LP4243 | 0.09 | 0.08 | 0.08 | 0.08 |
| LP4291 | 0.07 | 0.07 | 0.07 | 0.07 |
| LP4301 | 0.18 | 0.14 | 0.19 | 0.17 |
| LP4306 | 0.26 | 0.25 | 0.24 | 0.25 |
| LP4312 | 0.10 | 0.09 | 0.09 | 0.09 |
| LP4321 | 0.13 | 0.11 | 0.12 | 0.12 |
| LP4322 | 0.11 | 0.09 | 0.09 | 0.10 |
| LP4325 | 0.13 | 0.10 | 0.10 | 0.11 |
| LP4326 | 0.11 | 0.10 | 0.09 | 0.10 |
| LP4438 | 0.09 | 0.08 | 0.08 | 0.08 |
| LP4439 | 0.14 | 0.10 | 0.11 | 0.12 |
| LP4460 | 0.07 | 0.06 | 0.07 | 0.07 |
| LP4464 | 0.10 | 0.13 | 0.11 | 0.11 |
| LP4584 | 0.10 | 0.10 | 0.09 | 0.10 |
| LP4585 | 0.11 | 0.10 | 0.11 | 0.11 |
| LP4586 | 0.13 | 0.11 | 0.10 | 0.11 |
| LP4587 | 0.14 | 0.13 | 0.11 | 0.13 |
| LP4599 | 0.12 | 0.10 | 0.11 | 0.11 |
| LP4634 | 0.14 | 0.12 | 0.12 | 0.13 |
| LP4703 | 0.06 | 0.05 | 0.06 | 0.06 |
| LP4741 | 0.06 | 0.06 | 0.05 | 0.06 |
| LP4742 | 0.06 | 0.05 | 0.06 | 0.06 |
| LP4762 | 0.08 | 0.07 | 0.07 | 0.07 |
| LP4906 | 0.10 | 0.09 | 0.08 | 0.09 |
| LP4907 | 0.12 | 0.10 | 0.09 | 0.10 |
| LP4926 | 0.19 | 0.17 | 0.16 | 0.18 |
| LP4927 | 0.08 | 0.07 | 0.08 | 0.08 |
| LP4743 | 0.06 | 0.06 | 0.06 | 0.06 |
| LP5067 | 0.24 | 0.20 | 0.26 | 0.23 |
| LP5107 | 0.20 | 0.19 | 0.20 | 0.20 |
| LP5129 | 0.10 | 0.08 | 0.09 | 0.09 |
| LP5160 | 0.07 | 0.06 | 0.06 | 0.07 |
| LP5313 | 0.11 | 0.08 | 0.09 | 0.09 |
| LP5314 | 0.12 | 0.11 | 0.10 | 0.11 |
| LP5431 | 0.13 | 0.11 | 0.15 | 0.13 |
| LP5432 | 0.14 | 0.14 | 0.15 | 0.14 |
| LP5433 | 0.15 | 0.13 | 0.13 | 0.14 |
| LP5598 | 0.15 | 0.13 | 0.12 | 0.13 |
| LP5599 | 0.17 | 0.12 | 0.14 | 0.14 |
| LP5705 | 0.06 | 0.07 | 0.08 | 0.07 |
| LP5706 | 0.08 | 0.07 | 0.08 | 0.08 |
| LP5761 | 0.10 | 0.09 | 0.11 | 0.10 |
| LP5807 | 0.15 | 0.13 | 0.14 | 0.14 |
| LP5808 | 0.16 | 0.14 | 0.14 | 0.15 |
| LP5933 | 0.24 | 0.17 | 0.20 | 0.20 |
| LP5936 | 0.17 | 0.14 | 0.14 | 0.15 |
| LP5951 | 0.11 | 0.08 | 0.09 | 0.09 |
| LP5959 | 0.08 | 0.08 | 0.08 | 0.08 |
| LP5960 | 0.08 | 0.08 | 0.08 | 0.08 |
| LP5971 | 0.04 | 0.04 | 0.04 | 0.04 |
| LP5978 | 0.07 | 0.07 | 0.08 | 0.07 |
| LP6127 | 0.07 | 0.07 | 0.07 | 0.07 |
| LP6154 | 0.09 | 0.08 | 0.09 | 0.09 |
| LP6209 | 0.14 | 0.12 | 0.12 | 0.13 |
| LP6215 | 0.06 | 0.06 | 0.06 | 0.06 |
| LP6259 | 0.05 | 0.05 | 0.05 | 0.05 |
| LP6264 | 0.11 | 0.10 | 0.11 | 0.11 |
| LP6332 | 0.23 | 0.20 | 0.20 | 0.21 |
| LP6334 | 0.10 | 0.10 | 0.09 | 0.10 |
| LP6335 | 0.04 | 0.03 | 0.03 | 0.03 |
| LP6349 | 0.07 | 0.06 | 0.06 | 0.07 |
| LP6359 | 0.06 | 0.05 | 0.05 | 0.05 |
| LP6360 | 0.06 | 0.04 | 0.06 | 0.05 |
| LP6389 | 0.03 | 0.03 | 0.03 | 0.03 |
| LP6406 | 0.09 | 0.08 | 0.07 | 0.08 |
| OLP2706 | 0.07 | 0.06 | 0.14 | 0.09 |

As shown in Tables 4 and 5, by LPA siRNA DLR screening of a total of 387 pairs of siRNAs in 293T cells, the top 50 siRNA molecules with higher activity were found (siRNA names are shown in Table 7), which were then subsequently screened in vitro using RT4 cells.

### IV. Screening by LPA siRNA qPCR

### 1. LPA siRNA transfection into RT4 cells

RT4 cells were cultured in McCoy's medium containing 10% fetal bovine serum in a 5% CO₂, 37°C constant temperature incubator. When the cells were in the logarithmic growth phase and in good condition (70% confluence), they were plated for transfection. The cell density was adjusted, and 1.5×10⁵ cells per well were plated in a 24-well plate. The transfection complex was prepared as follows: 50µL Opti-MEM and 1µL 10nM siRNA were mixed, and 50µL Opti-MEM and 1.5µL RNAi Max transfection reagent were mixed, and then the two mixtures were mixed and allowed to stand for 15min. The above transfection complex was added to a 24-well plate and incubated in a 5% CO₂, 37°C constant temperature incubator for 48h. In addition to the experimental group, the following control groups were set up for each cell transfection: NC group was a negative control (irrelevant siRNA), RNAi Max group was a transfection reagent control, and Blank group was an untreated control (no siRNA added).

### 2. Real-time fluorescence quantitative PCR analysis:

After 48 hours of transfection, cells were lysed and total RNA was extracted using a column extraction kit (Novagen). GAPDH gene was used as the internal control gene, and real-time fluorescence quantitative PCR reaction was performed using the Sybrgreen method and a CFX96 fluorescence quantitative PCR instrument (Bio-Rad). The primers used were:

**Table 6 information of primer sequences**

| Primer name | Sequence (5' to 3') |
|---|---|
| H-LPA-F | TCCGAACAAGCACCGACTG (SEQ ID NO:789) |
| H-LPA-R | GGTCCGACTATGCGAGTGT (SEQ ID NO:790) |
| H-GAPDH-F | GGAGCGAGATCCCTCCAAAAT (SEQ ID NO:791) |
| H-GAPDH-R | GGCTGTTGTCATACTTCTCATGG (SEQ ID NO:792) |

### 3. Data Analysis

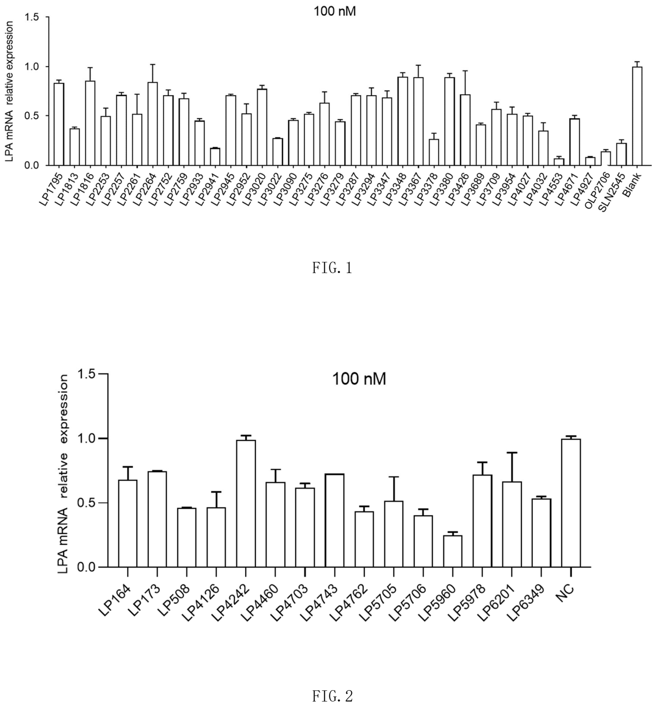

After the PCR reaction, the 2-ΔΔCt (Livak) method was used to perform relative quantitative analysis using the control gene as the standard. The results in Table 7 and Figures 1 and 2 show that 100 nM LP4553 and LP4927 have high inhibitory activities in RT4 cells, which are 6.9% and 5.6% higher than that of the positive control OLP2706 respectively; and 15.5% and 14.2% higher than that of the positive control SLN2545 respectively. It should be understood that all dsRNAs used in this example, including the positive control, are naked nucleic acid molecules without chemical modification.

**Table 7 Inhibitory activities of the top 50 siRNA sequences**

| Relative expression of LPA mRNA | |
|---|---|
| Blank | 100.0% |
| NC | 100.0% |
| LP164 | 68.3% |
| LP173 | 74.7% |
| LP508 | 45.8% |
| LP4126 | 46.4% |
| LP4242 | 99.1% |
| LP4460 | 66.3% |
| LP4703 | 61.7% |
| LP4743 | 72.7% |
| LP4762 | 43.4% |
| LP5705 | 51.7% |
| LP5706 | 40.3% |
| LP5960 | 24.9% |
| LP5978 | 72.1% |
| LP6201 | 66.8% |
| LP6349 | 53.6% |
| LP1795 | 83.7% |
| LP 1813 | 37.2% |
| LP1816 | 85.9% |
| LP2253 | 49.7% |
| LP2257 | 71.3% |
| LP2261 | 51.7% |
| LP2264 | 84.5% |
| LP2752 | 70.9% |
| LP2759 | 67.9% |
| LP2933 | 45.0% |
| LP2941 | 17.6% |
| LP2945 | 71.2% |
| LP2952 | 52.3% |
| LP3020 | 77.6% |
| LP3022 | 27.5% |
| LP3090 | 45.8% |
| LP3275 | 52.0% |
| LP3276 | 63.5% |
| LP3279 | 44.3% |
| LP3287 | 71.0% |
| LP3294 | 71.0% |
| LP3347 | 68.8% |
| LP3348 | 89.7% |
| LP3367 | 89.5% |
| LP3378 | 26.6% |
| LP3380 | 89.4% |
| LP3426 | 72.0% |
| LP3689 | 41.3% |
| LP3709 | 56.7% |
| LP3954 | 51.8% |
| LP4027 | 50.3% |
| LP4032 | 35.2% |
| LP4553 | 7.1% |
| LP4671 | 47.3% |
| LP4927 | 8.4% |
| OLP2706 | 14.0% |
| SLN2545 | 22.6% |

### Example 2. Optimization of LPA-siRNA

### I. transfection of Hep3B cells with LPA plasmid

In order to increase the expression of LPA, LPA plasmid was transfected into Hep3B cells. The full-length sequence of LPA mRNA (Gene ID: 4018) was inserted into the pcDNATM 3.1 plasmid to recombinantly construct a plasmid that can be transcribed to form LPA mRNA (GenScript Biotech Co., Ltd.). After the Hep3B cells were passaged, this transfection experiment was performed after growing in the incubator for 24 hours. In a 10 cm culture dish, 5 µL of lipo2000 was added to 500 µL of opti-MEM, and 10 µg of plasmid was added to 500 µL of opti-MEM. The plasmid mixture was allowed to stand for 5 minutes, and added with lipo2000 mixture. After gently blowing three times, it was allowed to stand for 10 minutes. The mixture was added into a 10 cm culture dish and could be used for transfection after 24 hours. The top 5 highly active siRNA molecules were further confirmed, and their sequences were modified and optimized (Table 8), and compared with the positive control sequence (Table 9) (Table 10).

**Table 8 Top 5 highly active siRNA modified sequences**

| name | Sequence (5'->3') | length | Molecular weight (g/mole) | GC content |
|---|---|---|---|---|
| LP 164-E05 | AmsGmsAmGmUmUmAfUmCfGfAfGmGmCmA mCmGmUmAmCmUm(SEQ ID NO:1)-L96 | 21 | 8,803.58 | 47.6% |
| | AmsGfsUmAmCmGmUmGmCmCmUmCmGmAf UmAfAmCmUmCmUmsGmsUm (SEQ ID NO:2) | 23 | 7,647.20 | 47.8% |
| LP173-E05 | GmsAmsGmGmCmAmCfGmUfAfCfUmCmCmAm CmCmAmCmUmGm(SEQ ID NO:3)-L96 | 21 | 8,737.58 | 61.9% |
| | CmsAfsGmUmGmGmUmGmGmAmGmUmAmCf GmUfGmCmCmUmCmsGmsAm(SEQ ID NO:4) | 23 | 7,769.20 | 60.9% |
| LP508-E05 | AmsGmsUmUmAmUmCfGmAfGfGfCmAmCmA mUmAmCmUmCmCm(SEQ ID NO:5)-L96 | 21 | 8,723.58 | 47.6% |
| | GmsGfsAmGmUmAmUmGmUmGmCmCmUmCf GmAfUmAmAmCmUmsCmsUm(SEQ ID NO:6) | 23 | 7675.2 | 47.8% |
| LP2941 -E05 | CmsUmsGmCmCmAmAfGmCfUfUfGmGmUmCm AmUmCmUmAmUm (SEQ ID NO:7)-L96 | 21 | 8,677.48 | 47.6% |
| | AmsUfsAmGmAmUmGmAmCmCmAmAmGmCf UmUfGmGmCmAmGmsGmsUm(SEQ ID NO:8) | 23 | 7,761.30 | 47.8% |
| LP4927 -E05 | CmsAmsGmAmGmUmUfAmUfCfGfAmGmGmC mAmCmAmUmUmCm(SEQ ID NO:9)-L96 | 21 | 8,736.58 | 47.6% |
| | GmsAfsAmUmGmUmGmCmCmUmCmGmAmUf AmAfCmUmCmUmGmsGmsCm(SEQ ID NO:10) | 23 | 7,674.20 | 52.2% |

In the above table, Am, Um, Cm and Gm represent 2'-O-methyl modified ribonucleotides A, U, C and G respectively; Af, Uf, Cf and Gf represent 2'-fluoro modified ribonucleotides A, U, C and G respectively; s indicates that the preceding and the posterior nucleotides are ligated by a phosphorothioate backbone.

**Table 9 Positive control sequence (5'->3')**

| | |
|---|---|
| OLP2706-modified | Sense strand CmAmGmCmCmCmCfUmUfAfUfUfGmUmUmAmUmAmCmGms (SEQ ID NO:793)-L96 |
| | Antisense strand UmsCfsGmUfAmUfAmAfCfAmAmUmAmAfGmGfGmGmCmUm (SEQ ID NO:794) |
| SLN2545-modified | CmGmGmUmAmAmUfGfGfAmCmAmGmAmGmUmUmsAmsUm (SEQ ID NO:795)-L96 |
| | AmsUfsAmAfCmUfCmUfGmUfCmCfAmUfUmAfCmsCfsGm (SEQ ID NO:796) |

In the above table, Am, Um, Cm and Gm represent 2'-O-methyl modified ribonucleotides A, U, C and G respectively; Af, Uf, Cf and Gf represent 2'-fluoro modified ribonucleotides A, U, C and G respectively; s indicates that the preceding and the posterior nucleotides are ligated by a phosphorothioate backbone.

**Table 10 Inhibitory activities of the top five modified siRNA sequences**

| LPA mRNA inhibition efficiency | |
|---|---|
| LP164-E05 | 85.7% |
| LP173-E05 | 84.0% |
| LP508-E05 | 90.3% |
| LP2941-E05 | 83.2% |
| LP4927-E05 | 90.6% |
| OLP2706 -modified | 81.7% |
| SLN2545 -modified | 84.9% |

### II. IC50 value in transfected Hep3B cells

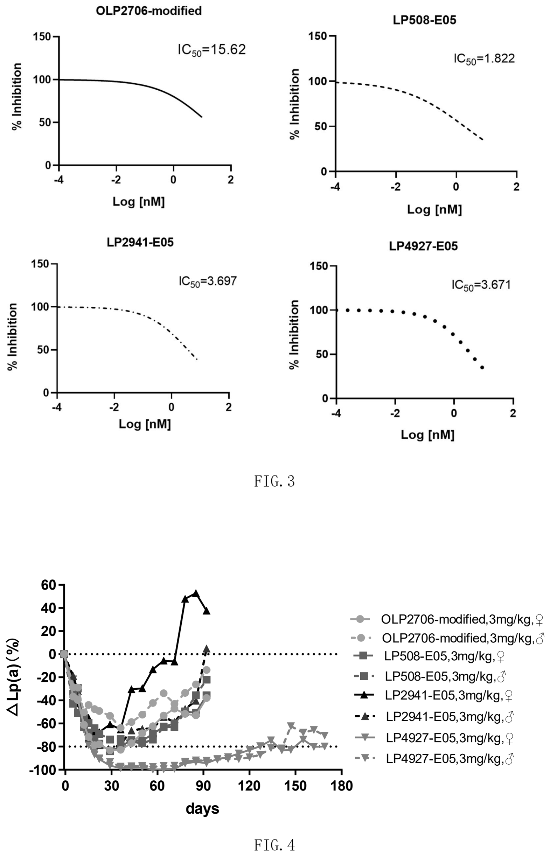

LP508, LP2941, LP4927 and the positive control OLP2706-modified were diluted to different concentrations with Nuclease-Free Water (Invitrogen), and the highest final concentration was set to 10 nM. 10-fold gradient dilutions were made to a total of 6 concentrations, and transfected into Hep3B cells. The steps of LPA plasmid transfection, siRNA transfection and quantitative PCR detection and analysis were set forth in Example 1, and the IC₅₀ values were analyzed and calculated using Graphpad Prism software. As shown in Figure 3, the IC₅₀ values of LP508-E05, LP2941-E05, LP4927-E05 and the positive control OLP2706-modified in Hep3B cells were 1.822nM, 3.697nM, 3.671nM and 15.62nM, respectively. The inhibition rates of LP508-E05, LP2941-E05 and LP4927-E05 on LPA mRNA were better than that of the positive control OLP2706-modified.

### Example 3: In vivo effectiveness test in rhesus monkeys

Eight rhesus monkeys (Beijing JOINN Laboratories) were randomly assigned to a positive control group or an experimental test group (four test compounds OLP2706-modified, LP508-E05, LP2941-E05, and LP4927-E05) respectively based on body weights and lipoprotein (Lp(a)) levels. Each group consisted of two animals, one male and one female. Prior to administration, serum was collected from each animal and baseline Lp(a) levels were measured. The test drug was administered once (dosage of 3 mg/kg, subcutaneous injection), and about 3 mL of blood was collected from the subcutaneous vein of the forelimb or hindlimb on days -1 (1 day before administration), 5, 8, 12, 15, 19, 22, 29, 36, 43, 50, 57, 64, 71, 78, 85, 92, 99, 106, 113, 120, 127, 134, 141, 148, 155, 162, and 169 (fasting overnight before blood collection, the day of injection was day 1), and the serum was separated for Lp(a) detection.

### I. Lp(a) protein detection

The level of Lp(a) protein in serum was detected using Human Lipoprotein A ELISA Kit (abcam, ab212165), and all values were normalized based on the baseline value of each animal collected before administration and presented as a percentage of the initial level (FIG.4, Table 11).

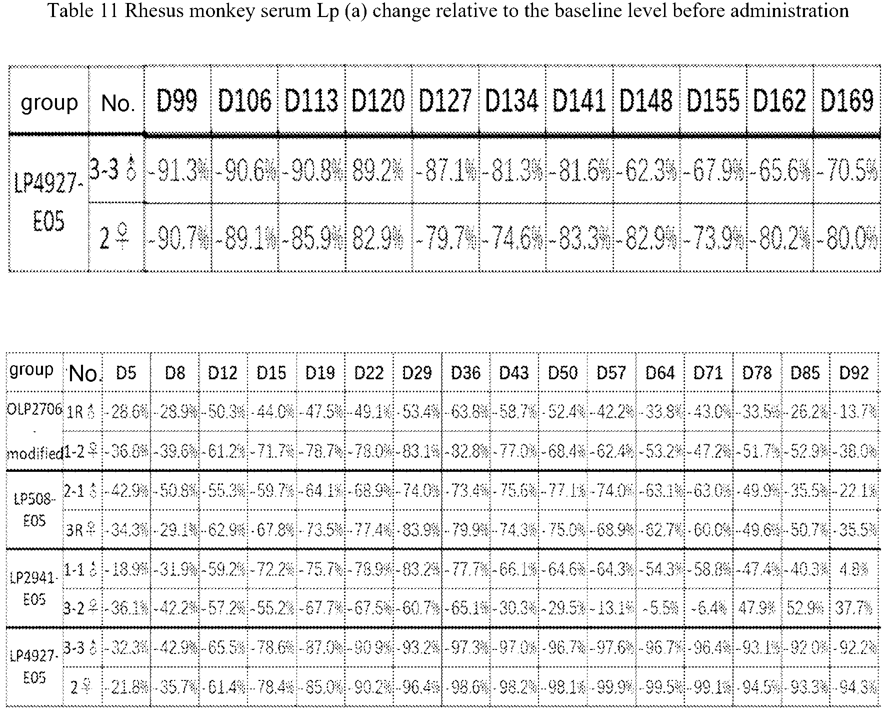

The results (Figure 4, Table 11) show that compared with before administration, the serum Lp(a) protein levels of the four groups of animals were significantly reduced after administration; wherein the OLP2706-modified (positive control), LP508-E05, and LP2941-E05 administration groups decreased by 64% to 84% from the 19th day to the 36th day; the LP4927-E05 administration group decreased by 97% to 99% on the 36th day. Then, OLP2706-modified (positive control), LP508-E05, and LP2941-E05 rebounded. The experiment was continued to be observed. On the 71st day, LP4927-E05 still maintained a stable decline, inhibiting the amount of Lp(a) protein in serum by 96% to 99%. By the 92nd day, the decrease in the OLP2706-modified (positive control) and LP508-E05 administration groups was only 17% to 38%, the LP2941-E05 administration group returned to the pre-administration level, and the LP4927-E05 group continued to decrease by 92% to 94%. When the LP4927-E05 sampling time was extended to the 169th day, the serum Lp(a) protein level still decreased by 70% to 80%. It can be seen that the long-term effect of LP4927-E05 in reducing the level of Lp(a) protein in serum is significantly better than the positive control and other dsRNAs targeting LPA mRNA.

The above description is only directed to preferred embodiments, which are only used as an example and does not limit the combination of the necessary features for the implementation of the present application. The title provided is not intended to limit the various embodiments of the present application. Terms such as "comprising", "comprises" and "including" are not intended to be limiting. In addition, unless otherwise specified, plural forms are included when there is no numeral modification, and "or" means "and/or". Unless otherwise defined herein, the meaning of all technical and scientific terms used herein is the same as that generally understood by those skilled in the art. All disclosures and patents mentioned in this application are incorporated herein by reference. Without departing from the scope and spirit of the present application, various modifications and variations of the described methods and compositions of the present application are obvious to those skilled in the art. Although the present application is described by specific preferred embodiments, it should be understood that the present application claimed for protection should not be unduly limited to these specific embodiments. In fact, those various variations of the described modes for implementing the present application that are obvious to those skilled in the relevant art are intended to be included in the scope of the attached items.

## Claims

1. A dsRNA molecule for inhibiting Lp(a) gene expression, comprising a sense strand and an antisense strand that are complementary to each other to form a double-stranded region, wherein the sense strand and the antisense strand each comprise or consist of 15-25 nucleotides, and the antisense strand is complementary to at least 15, 16, 17, 18, 19, 20, or 21 consecutive nucleotides of a sense strand sequence shown in Table 2, and the double-stranded region is 15-25bp in length; optionally, wherein at least one nucleotide in the dsRNA molecule is modified.

2. The dsRNA molecule according to claim 1, **characterized in that** the nucleotide sequence of the sense strand and the nucleotide sequence of the antisense strand are selected from the sense strand and antisense strand sequences shown in Table 2.

3. The dsRNA molecule according to claim 1 or 2, **characterized in that** the modification is selected from any one or more of the following: locked nucleic acid modification, open ring or non-locked nucleic acid modification, 2'-methoxyethyl modification, 2'-O-methyl modification, 2'-O-allyl modification, 2'-C-allyl modification, 2'-fluoro modification, 2'-deoxy modification, 2'-hydroxyl modification, phosphorothioate backbone modification, DNA modification, fluorescent probe modification, and ligand modification.

4. The dsRNA molecule according to claim 3, **characterized in that** the modification patterns of the dsRNA molecule include: (1) sense strand: 17-21nt, preferably 21nt in length; composed of alternating 2'-O-methyl modified regions and 2'-fluoro modified regions, each modified region being 1 to 10 nucleotides in length; the modification patterns of the first modified region from the 5' end and that from the 3' end being the same; and the consecutive nucleotide regions from the 1st to the 3rd position from the 5' end being ligated by phosphorothioate backbones; (2)antisense strand: 19-23nt, preferably 23nt in length; composed of alternating 2'-O-methyl modified regions, 2'-fluoro modified regions, unmodified regions or DNA regions, each modified region being 1-11 nucleotides in length; and the consecutive nucleotide regions from the 1st to the 3rd position from the 5' end and the consecutive nucleotide regions from the 1st to the 3rd position from the 3 ' end all being ligated by phosphorothioate backbones.

5. The dsRNA molecule according to claim 1, **characterized in that** the base sequences of the sense strand and the antisense strand of the dsRNA molecule are or comprise sequences selected from any one of the following groups:
1) sense strand: CAGAGUUAUCGAGGCACAUUC (SEQ ID NO:709),
antisense strand: GAAUGUGCCUCGAUAACUCUGGC (SEQ ID NO:710);
2) sense strand: AGAGUUAUCGAGGCACGUACU (SEQ ID NO:485),
antisense strand: AGUACGUGCCUCGAUAACUCUGU (SEQ ID NO:486);
3) sense strand: GAGGCACGUACUCCACCACUG (SEQ ID NO:491),
antisense strand: CAGUGGUGGAGUACGUGCCUCGA (SEQ ID NO:492);
4) sense strand: AGUUAUCGAGGCACAUACUCC(SEQ ID NO:531),
antisense strand: GGAGUAUGUGCCUCGAUAACUCU(SEQ ID NO:532); and
5) sense strand: CUGCCAAGCUUGGUCAUCUAU (SEQ ID NO:119),
antisense strand: AUAGAUGACCAAGCUUGGCAGGU (SEQ ID NO:120).

6. The dsRNA molecule according to claim 1, wherein the sequences of the sense strand and the antisense strand of the dsRNA comprise or are selected from any one of the following groups:
1) sense strand:
CmsAmsGmAmGmUmUfAmUfCfGfAmGmGmCmAmCmAmUmUmCm (SEQ ID NO:9),
antisense strand:
GmsAfsAmUmGmUmGmCmCmUmCmGmAmUfAmAfCmUmCmUmGmsGmsCm (SEQ ID NO:10);
2) sense strand:
AmsGmsAmGmUmUmAfUmCfGfAfGmGmCmAmCmGmUmAmCmUm (SEQ ID NO:1) antisense strand:
AmsGfsUmAmCmGmUmGmCmCmUmCmGmAfUmAfAmCmUmCmUmsGmsUm (SEQ ID NO:2)
3) sense strand:
GmsAmsGmGmCmAmCfGmUfAfCfUmCmCmAmCmCmAmCmUmGm (SEQ ID NO:3) antisense strand:
CmsAfsGmUmGmGmUmGmGmAmGmUmAmCfGmUfGmCmCmUmCmsGmsAm (SEQ ID NO:4)
4) sense strand:
AmsGmsUmUmAmUmCfGmAfGfGfCmAmCmAmUmAmCmUmCmCm (SEQ ID NO:5) antisense strand:
GmsGfsAmGmUmAmUmGmUmGmCmCmUmCfGmAfUmAmAmCmUmsCmsUm (SEQ ID NO:6); and
5) sense strand:
CmsUmsGmCmCmAmAfGmCfUfUfGmGmUmCmAmUmCmUmAmUm (SEQ ID NO:7) antisense strand:
AmsUfsAmGmAmUmGmAmCmCmAmAmGmCfUmUfGmGmCmAmGmsGmsUm (SEQ ID NO:8); wherein Am, Um, Cm and Gm represent 2'-O-methyl modified ribonucleotides A, U, C and G respectively; Af, Uf, Cf and Gf represent 2'-fluoro-modified ribonucleotides A, U, C and G respectively; s indicates that the preceding and the posterior nucleotides are ligated by a phosphorothioate backbone.

7. The dsRNA molecule according to claim 6, further comprising at least one asialoglycoprotein receptor (ASGPR) ligand.

8. The dsRNA molecule according to claim 7, wherein the ligand is linked to a 5'-terminal or 3'-terminal nucleotide of the nucleotide sequence of the sense strand or antisense strand of the dsRNA through a phosphodiester bond or a phosphorothioate bond.

9. The dsRNA molecule according to any one of claims 1 to 8, wherein the ASGPR ligand is one or more GalNAc derivatives linked by a divalent or trivalent branched structure.

10. The dsRNA molecule according to claim 9, wherein the GalNAc derivative is inked by a trivalent branched structure comprising the following structure:

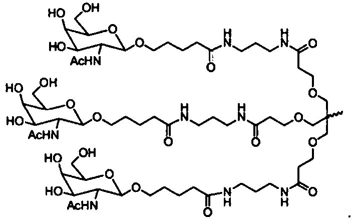

11. The dsRNA molecule according to claim 10, wherein the GalNac derivative is L96, and the structure of L96 is as follows:

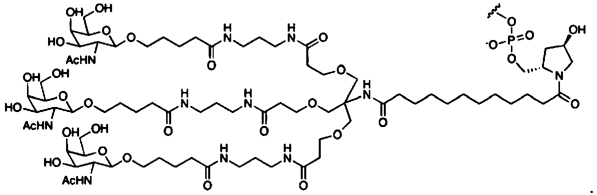

12. The dsRNA molecule according to claim 11, wherein the L96 is linked to a 3' terminal nucleotide of the nucleotide sequence of the sense strand of the dsRNA through a phosphodiester bond or a phosphorothioate bond; and the structure of the L96 and its linkage mode to the nucleotide sequence of the sense strand are as follows:

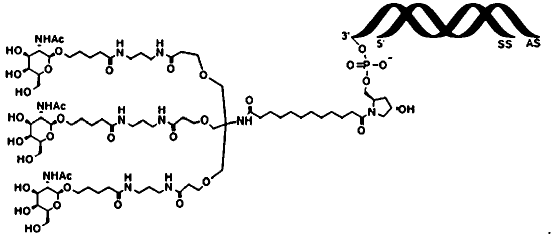

13. A use of a dsRNA, selected from any one of the following groups:
(I) Use of the dsRNA molecule according to any one of claims 1 to 12 in inhibiting Lp(a) gene expression or in preparing a product for inhibiting Lp(a) gene expression;
(II) Use of the dsRNA molecule of any one of claims 1 to 12 in a product for reducing the level of Lp(a) particles;
(III) Use of the dsRNA molecule of any one of claims 1 to 12 for preventing and/or treating a condition, pathology or syndrome associated with an elevated level of Lp(a) particles, or for the preparation of a product for preventing and/or treating a condition, pathology or syndrome associated with an elevated level of Lp(a) particles;
preferably, the disease associated with an elevated level of Lp(a) particles is selected from any one or more of the following: stroke, atherosclerosis, thrombosis, cardiovascular disease, and aortic valve stenosis.
